# EUROPEAN PATENT SPECIFICATION

(11) **EP 2 018 435 B1**
(45) Date of publication and mention of the grant of the patent: **11.07.2012**
(21) Application number: 07783871.2
(22) Date of filing: 17.05.2007
(51) Int. Cl.: C12N 15/82, A01H 5/00

(54) **Artificial plant minichromosomes**
Künstliche Pflanzenminichromosome
Minichromosomes de plantes artificielles

(30) Priority: 17.05.2006 US 801004 P
(43) Date of publication of application: 28.01.2009
(60) Divisional application: 10014358.5
(73) Proprietor: Pioneer Hi-Bred International Inc., Johnston, IA 50131-1014 (US)
(72) Inventor: ANANIEV, Evgueni, Johnston, Iowa 50131 (US); CHAMBERLIN, Mark A., Windsor Heights, Iowa 50311 (US); GORDON-KAMM, William J., Urbandale, Iowa 50322 (US); SVITASHEV, Sergei, Johnston, Iowa 50131 (US); WU, Chengcang, Verona WI 53593 (US)
(74) Representative: Bentham, Andrew
(86) International application number: PCT/US2007/069139
(87) International publication number: WO 2007/137114

(56) References cited:
- WO-A-2005/010142
- HAN FANGPU ET AL: "High frequency of centromere inactivation resulting in stable dicentric chromosomes of maize" PROCEEDINGS OF THE NATIONAL ACADEMY OF SCIENCES OF THE UNITED STATES OF AMERICA, vol. 103, no. 9, February 2006 (2006-02), pages 3238-3243, XP002462810 ISSN: 0027-8424
- NAGAKI KIYOTAKA ET AL: "Molecular and cytological analyses of large tracks of centromeric DNA reveal the structure and evolutionary dynamics of maize centromeres." GENETICS, vol. 163, no. 2, February 2003 (2003-02), pages 759-770, XP002462760 ISSN: 0016-6731
- DATABASE EMBL [Online] 15 April 2006 (2006-04-15), "Zea mays chromosome 4 clone CH201-478E6; ZMMBBc0478E06, *** SEQUENCING IN PROGRESS ***, 12 unordered pieces." XP002462994 retrieved from EBI accession no. EMBL:AC185251 Database accession no. AC185251
- DAWE R KELLY ET AL: "A maize homolog of mammalian CENPC is a constitutive component of the inner kinetochore" PLANT CELL, vol. 11, no. 7, July 1999 (1999-07), pages 1227-1238, XP002462808 ISSN: 1040-4651
- ANANIEV E V ET AL: "Chromosome-specific molecular organization of maize (Zea mays L.) centromeric regions" PROCEEDINGS OF THE NATIONAL ACADEMY OF SCIENCES OF THE UNITED STATES OF AMERICA, vol. 95, no. 22, 27 October 1998 (1998-10-27), pages 13073-13078, XP002462809 ISSN: 0027-8424
- KASZAS ETIENNE ET AL: "Meiotic transmission rates correlate with physical features of rearranged centromeres in maize" GENETICS, vol. 150, no. 4, December 1998 (1998-12), pages 1683-1692, XP002462811 ISSN: 0016-6731
- BROWN W R A ET AL: "Artificial chromosomes: ideal vectors?" TRENDS IN BIOTECHNOLOGY, ELSEVIER, AMSTERDAM, NL, vol. 18, no. 5, May 2000 (2000-05), pages 218-223, XP004196003 ISSN: 0167-7799
- YU WEICHANG ET AL: "Construction and behavior of engineered minichromosomes in maize" PROCEEDINGS OF THE NATIONAL ACADEMY OF SCIENCES OF THE UNITED STATES OF AMERICA, vol. 104, no. 21, May 2007 (2007-05), pages 8924-8929, XP002462806 ISSN: 0027-8424

### SEQUENCE LISTING

<110> PIONEER HI-BRED INTERNATIONAL, INC.
<120> Artificial Plant Minichromosomes
<130> 2083-PCT
<150> 60/801,004
   <151> 2006-05-17
<160> 193
<170> FastSEQ for Windows Version 4.0
<210> 1
   <211> 4635
   <212> DNA
   <213> Zea mays
<220>
   <221> source
   <222> (1)...(4635)
   <223> CentA
<400> 1
   tgatgagaac ataacccgca cagatatgac catgttaatg gctcctgcta caaagacatt 60
   gaggaacaaa gaagttgatt ggggaccaag taatgatatt tccaacattt ccaacaaagc 120
   aagcacatca tcaaatttaa agatatactt gggtgaggag catacactag agtcgaggac 180
   gactctatta caagaagggg aggatgatga ggacatcact gccatcaata caccacacca 240
   gcgacctcct tcaccattta ataatggacc agtaaacgag tccgtgcacg taaattttat 300
   tatcaggtga actcgttcct tattgttgaa gctaatcatt ccttaaatga ggtactaata 360
   ccttgtgatt actttattaa tctaaggtgt ttgggaggtg aaccatctag aatttgagaa 420
   ggcaacaagg caataaaagc tgctccactt gaggggattt cgaaactaca acaagtgcaa 480
   gtttaagagg gcatatcttt cagctcctaa ggttgtttaa tgcaaataag cacttgttgg 540
   aaaggtctct ttgtctactt tctagtggat caagaatcaa cgagagatca gacactaagt 600
   gtccagaaac tgccgagtga actcctgctc tacccaagtc aatttcgtaa ctgcagcatg 660
   caccaaatta aatggagcat aactttccac tcccaaggtt gtttagtgca aataactact 720
   tgttggaaag ctctcttcgt ctactttcat gtgcatcaat aatcaatgac agaaaccaaa 780
   cgaggcgtcc agaaactgcc gagagagttt cgttctccat tagaactcct ttctattcct 840
   ctatttaagc aactagcagc caccaaagaa cttgggtttt tgtttgatgt aagtttagcc 900
   tttgctactt ccttgtaaac gcatgtgtcg gctagaccac ccggatactt gaaacagaac 960
   cccaactcta tcagatccgt gagtgtctgc tttttatctt gttcttgctt gttctcgatt 1020
   gcttgcaggt tcaaggctgt tcttggcacg gcaagggcag caacaacagg agccgatgta 1080
   actatcgcta aggcgcagca cccttgtggt tgttgtagtc ggatagcaca acgtcgacct 1140
   ccaccccaaa tcgtagttat caggagacgg tgtacctgtc gctcaaggcg ccacaccatc 1200
   ttggttgtgg tagtcgggca gccaacgtcg ttctccaaca agtttccacc tccatcatct 1260
   ctcatcgaaa gatcgggcac ccttctaccc gttgggttta tcaagtggta tcaaatttca 1320
   ggttgctcgg tgagagatct caatcttcct tgttttgttt acctacagtc cacttttgcc 1380
   caaagatata tttagagcag aaattcacct aaaaacagtt tgagcctttg ctttactact 1440
   tagttttcga cttgttgaat tccggtagct gcatttgggt cgagttgctg gtctaaagtt 1500
   ttcttaccgc tagagtttcg agttcgcgcc accttgtttc aatcaccagt ttagacctct 1560
   tgctgcaatt caaccaaaaa gaagagaaag caaaaggcga gtgcacaaaa aaagccgcac 1620
   taatcagcaa aacaaaaaaa gacacgtgca aaacaaaaga gagagaaaaa aaccagttct 1680
   gaattttggt agataaaatt tgtaagtgca acaaaacaaa aggcagtttg tgtgccttct 1740
   ttttatagtt tcagaaatca gattgttgtt ctgagctttt ggtgatacta tttgtgtaac 1800
   ggctcgcgtc tctattacgg tttggactag gaccagcaca acaccttgtg gaacgtttat 1860
   tcaacttgtt gtggctaacg tggtactagc tattccttgg aactattgtt taaacagcca 1920
   cctataaatc cacaaaattt tctacaacac caccaggttg tgctagcagc cactgttgtt 1980
   gttgttcgtg ctgtttgcca gcgcctcctg ctttgcgtgg tgagaacttg taagaacttg 2040
   tttaaccagt ttgagagtga gagattacaa caatgattcc tagtagttta tagaatcaaa 2100
   gatatttttt attgtttctt gtctttacta aacatggcag gtgatatgga catttttgac 2160
   ccaaccgaac gttatattgg aggcatcatt caacacttgc ctttatatgc cggtaaattc 2220
   gatcctcatg catacattga ttgggagcta aagctagata aggaatttga taagcatgat 2280
   ctatctcaaa aacaaaagat ttatattgcc tctaatttgt taactgagca cgcattgatg 2340
   gaatggaaat acatttgtag gcacaacaaa gttccacaat cttgggaaga cttcaaactt 2400
   cattttagag atgcattcat tcctgcatac tatgctgatc atttgctttc taaattagac 2460
   accttaaagc agggtgctag gactgtgaaa gattattatt atgattttaa aatttttacc 2520
   atgtttgctc gtttagatga atgcatggaa gatgtcatga ctaggttcat gaaaggactc 2580
   aattctgaaa ttcagactat agtcatgcat gaagcataca aacacatttc tcacttgttt 2640
   ttgcttgcat gtaaagctga aaatgagatt ctattataca attatacaag cactgaacat 2700
   gtgagccata attcctcttt tgcatcttct ctacatgctg atcaagaaca caaaataatg 2760
   aaaccagctg ttgtttttcc atcatcacaa gaagaattga ttgctgacac ttgtgatagt 2820
   gaagatttgt gggataatga ttcacatgta ctaagacaac aactagtaaa tgaacatgtt 2880
   acatctatta ttgaaccaaa cattttggct aaaaaggaac atgtaatttg tattgcaaac 2940
   gaaactgaag aaataaattt gctctcttct ttaaatactt ggggctatat tgaatttgat 3000
   gatctttttg agctcggtaa tttggaaaat attttatttg ctagattcaa ctataccatg 3060
   tccttctcat gatatatttt atattgctgg caagtacaac aacataggac aatttcttgt 3120
   gcatagaatt tctatttcat ctagatatgt tgtttcttca ctttgtgcaa ataagatatt 3180
   ggtatgttct caagaagaaa agaatctctt gtttccatgt actttagttg aagtttcagg 3240
   tttatatttg aaagacatta ataaaagctt agtcatcaac atcaatcatg atgcaaaacc 3300
   gaggacggtt tgctatcaag aaggggagaa tgatgagaac ataacccgca cagatatgac 3360
   catgttaatg gctcctgcta caaagacatt aaggaacaaa gaagttgatt ggggaccaag 3420
   taatgatatt ttcaacattt ccaacaaagc aagcacatca tcaaatttaa agatatactt 3480
   gggtgaggag catacactag agtcgaggac gactctatta caagaagggg aggacgatga 3540
   ggacatcact gccatcaata caccacacca gcgacctcct tcaccattta ataatggacc 3600
   agtaaacgag tccgtgcacg taaatttaat tatcaggtga actcgttcct tgttgttgaa 3660
   gctaatcatt ccttaaatga ggtactaata ccttgtgatt actttattat tctaaggtgt 3720
   ttgggaggtg aaccatctag aatttgagaa ggcaacaagg caataaaagt tgctccactt 3780
   gaggggattt cgaaactaca acaagtgcaa gtttaagagg gcatatcttt cagctcctaa 3840
   ggttgtttaa tgcaaataag cacttgttgg aaaggtctct ttgtctactt tctagtggat 3900
   caagaatcaa cgagagatca gacactaagt gtccagaaac tgccgagtga actcctgctc 3960
   tacccaagtc aatttcgtaa ctgcagcatg caccaaatta aatggagcat aactttccac 4020
   tcccaaggtt gtttagtgca aataactact tgttggaaag ctctcttcgt ctactttcat 4080
   gtgcatcaat aatcaatgac agaaaccaaa cgaggcgtcc agaaactgcc gagagagttt 4140
   cgttctccat tagaactcct ttctattcct ctatttaagc aactagcagc caccaaagaa 4200
   cttgggtttt tgtttgatgt aagtttagcc tttgctactt ccttgtaaac tcatgtgtcg 4260
   gctagaccac ccggatactt gaaacaaaac cccaactcta tcagatccgt gagtgtctgc 4320
   tttttatctt gttcttgctt gttctcgatt gcttgcaggt tcaaggctgt tcttggcacg 4380
   gcaagagcag caacaacagg agccggtgta actatcgcta aggcgcagca cccttgtggt 4440
   tgttgtagtc ggatagcaca acgtcgacct ccaccccaaa tcgtagttat caggagacgg 4500
   tgtacctgtc gctcaaggca ccacaccatc ttggttgtgg tagtcgggca gccaacgtcg 4560
   ttctccaaca agttttccac ctccatcatc tctcatcgaa agatcgggca cccttctacc 4620
   cgttgcgttt atcaa                                                  4635
<210> 2
   <211> 156
   <212> DNA
   <213> Zea mays
<220>
   <221> source
   <222> (1)...(156)
   <223> CentC
<400> 2
   ggttccggtg gcaaaaactc gtgctttgta tgcaccccga cacccgtttt cggaatgggt 60
   gacgtgcgac aacgaaattg cgcgaaacca ccccaaacat gagttttgga cctaaagtag 120
   tggattgggc atgttcgttg cgaaaaacga agaaat                          156
<210> 3
   <211> 6915
   <212> DNA
   <213> Zea mays
<220>
   <221> source
   <222> (1)...(6915)
   <223> CRM1
<400> 3
   cttggtcttg gacagtacct cactgatgaa gtagacctga tgaagctgag gtgcccgatc 60
   tttcggcgag tagagataat tccgatttgg cggaagatga cccttgcgat ccgactacga 120
   cgagcaagcc cgaggcgcca atgcaatcgc tgaaccaact ccctgtggtt accgaccttg 180
   ctgatgcgag atcggcctga tcacgaagat cgtttcctgt gcgcaatcga agaacgaaca 240
   agaataagat gcgagcaatc taatctatta ctcgagggtg gagttctgaa tacacgaaga 300
   cagcgcagat tagcgcgtgt tcgagagtag ctaaggctaa cgtaaaacaa aactcaggaa 360
   ataaaggagg cgcagctcct gaataaatag agagggggcg cagcccctag gggcggccaa 420
   ccctaggtcg tccattatgg gccgcaattg ggctggtcgt ctattcttcc gggccttcgt 480
   tctttaacaa catgatgtag ttcaattctc ttgcacgggc ccgagtcact ggcccaggtg 540
   gaaggggtgg cgcctgggct ggagaaggtg ctgctggtgt agatgtgctc gtgttggtgt 600
   tgatgtccgc atcatcctcc ccttcttgaa ctgaagtcgt cctcgacggc aactcctcat 660
   cttctcccgc atacggtttc aaatctgcaa cattaaaact agtggaaaca ccaaactccg 720
   caggtaggtc gagggtataa gcattatcat taatcttggt tagtatctta aaaggaccag 780
   cagcacgagg catcaattta gaacggcgca aagtaggaaa acgatccttt ctcaaatgca 840
   accaaaccat atcaccaggt tcaaaagtaa catgttttct tcctttacta ccagcaacct 900
   gattttttgt attagtagca gcaatgttct gtttcgtttg ttcatgtatg gtaatcattt 960
   gttcaacatg tgcagaagca tctacatgtg gggcgttcgc agcattaagt gaaatcaaat 1020
   caataggtgc cctaggaatg taaccataaa caatctggaa agggcacatc tttgtagaag 1080
   aatgcgtggc atgattgtaa gcaaattcaa catgaggcaa gcaatcctcc caacgtcgca 1140
   aattcttgtc taaaacagcc ctaagcatgg tagataaagt tctatttact acctcagttt 1200
   gtccatcagt ttgagggtga caagtagtgc taaacaacaa tttagttccc aatttattcc 1260
   aaagagatct ccaaaaatgg cttagaaact tagcatcgcg atcagagact attgtttttg 1320
   gaataccatg taaacgaata atttctctaa agaacaattc agcaacaatg ctagcatcat 1380
   cagttttatg acaaggtata aagtgagcca ttttagagaa tctatcaaca accacaaaaa 1440
   tactatccct ccccttctta gttctaggca agcccaaaac aaagtccata gagatatcaa 1500
   gccaaggaga agaagggaca ggcaaaggca tatacaaacc atggttgttc aaccgtgact 1560
   tagctttctg acaagtagtg cagcgtgcaa caaggcgctc cacatcagcg cgcatccgag 1620
   gccaaaagaa gtgggcagcc aacacctcat gtgtcttgta gacgccaaaa tgccccatga 1680
   gaccgcctcc atgcgcttcc tgtaacaaca aaagacgaac cgagctagct ggaacacaca 1740
   gcttgttagc gcgaaacagg aacccatcct gtatgtgaaa tttgccccat ggttttccat 1800
   taatacaatg gccgaaagca tctttaaaat cagcatcgtc aacatattga tccttcacag 1860
   tgtccaaacc aaagatttta aaatctaact gtgacagcat ggtatagcga cgagacaaag 1920
   catcagcaat aacattgtcc ttcccgttct tgtgtttaat aatgtaagga aaggactcaa 1980
   tgaattctac ccatttagca tgacgacggt tcagatttgt ttgggtacga atatgtttta 2040
   aagcctcatg atcagaatgg attatgaact cacgatgcca aagatagtgc tgccatgtat 2100
   gcaaagtgcg cactaaagcg taaagctcct tatcataagt agaatatttc agactagcac 2160
   cgcttaattt ttcactaaaa taagcaactg gttttccttc ttgtaacaaa acagcaccca 2220
   gcccaatacc actagcatcg cattcaagct cgaaaacttt attaaaatca ggcaattgca 2280
   agaggggagc ttgggttaac ttatctttca aagtgctgaa cgctacctcc tgcgaatcac 2340
   tccaggcaaa cggcacatct ttctttgtaa gctcatgtag aggcgctgca atggagctaa 2400
   aatcacgaac aaatctgcgg tagaaaccgg caagtccaag aaagctccga atttgtgtga 2460
   ccgtcgtcgg tgtaggccac tcccgaatgg cagcaatctt gctgctatcc acctcaatgc 2520
   cctgcggagt aaccacataa ccaagaaacg agacacgttg cgtgcaaaat gtgcactttt 2580
   ccatgttacc aaacaagcga gcagcacgca aagcgtcaaa aacagcacgc aaatgttcta 2640
   aatgctcctc tatagacttg ctgtaaataa ggatatcatc gaaataaaca acaacaaaca 2700
   aacctatgaa ggcccgtaga acttcgttca ttaaacgcat aaaggtgctg ggagcattag 2760
   tcaatccaaa tggcataacc aaccattcat ataaaccaaa tttcgtttta aaagccgttt 2820
   tccattcatc accgagtttc attctaatct gatggtaacc gctacgcaaa tcaaccttag 2880
   agaaaataac ggcaccacta agctcatcta gcatatcatc aaggcgtgga ataggatatc 2940
   gataacgaac tgtgatgtta ttaatagcac gacaatctac gcacatacgc catgacccat 3000
   ctttctttgg aacgagtaaa acgggaaccg agcaagggct aagagactca cgaatgtaac 3060
   ccttatcaag cagcgtctgc acctggcgct ggatctcctt cgtctcatct ggatttgtac 3120
   ggtacggagc gcggttcgga agagaagtgc cggggatgag atcgatctga tgctcaatgc 3180
   cacggagggg aggaagaccc ggtggtaagt ccgtaggata aacatcagca tactcctgca 3240
   aaaggttaac aacagcaggg ggtatatcca aagacggtgc atcatcaagc ggaacaagca 3300
   ttcgcgagca tacaagtgca taacagggca tatgagcttc atggagatca tcaaaatcag 3360
   cacgtgtagc aagtaaaaca ggagagtgca acttgatttc agatttaata ggtgcggctg 3420
   atgtcgattt gacttgttgt gcagttttag cagccctagt aagatcatct ttcaaaattt 3480
   ggtcaggggt cattggatgt ataattattt tctggccttt aaacatgaaa gaataatgat 3540
   ttgaacgacc atgatgtaaa ctatcagtat catattgcca aggtcgaccc aataacaaag 3600
   agcatgcttc cataggaata acatcgcaat caacataatc agaataagaa cccagcgaaa 3660
   aggggacacg taccgaacgt gttaccttta ttttaccacc atcattaagc cattgaatgt 3720
   gatacggatg tggatgtgtg cgagtgggca aggataattt ctctaccaac gctgtacttg 3780
   ccaaattgtt gcagctgcca ctatcgatga tgatgcgaat cgaccgttcg tgcacgacgc 3840
   ccttggtatg gaatagagtg tgtcgctgat ttttttcggc ctgggcaacc tgtgtgctga 3900
   gaacacgctg cacaacaaga ctctcatacc tatcagcgtc gatgggatca acgtggactt 3960
   cctcattttc tgcatggtta gtggcaatca tagcatgact agtttcctca gaatcactgg 4020
   ctgaagagta ctcaccattg tcacgtataa gcaaggtacg cttgtttggg cagtcccgaa 4080
   tcatgtgccc aaaccctctg caacgatggc actgaatatc ccgtgtacgt cctgtggaag 4140
   aagcggcgcc tttggcaggg ggcgccactg gcttggccgg ccctgtgcgc gatgtagtgc 4200
   taggcgtagg aggtgcaggg ctggaaggag ttgagctgtg tgttggaccc cggcctgcaa 4260
   aagagttagt atatgtcttt gatcgtcgtc cctgcacttc acgttcagct ttgcaagcat 4320
   attcaaacaa tgtggttata tcaaaataat ccttataatc aagtatatcc tgaatttccc 4380
   tgttcaaacc accacgaaaa cgcgccatag cagcgtcatc tgactcaacc aaaccacaac 4440
   gaagcatacc cttttgcaac tcctggtaat attcctcaac agattgtgaa ccttgttgaa 4500
   aacgctgcat tttgttaagc aaatcacgag cataatagga aggaacaaat ctgtggcgca 4560
   tggcagtttt taattgggtc caagtaatga cactgttaat gggaagtttt tgtttatact 4620
   cacgccacca aattaaagca aaatcagtaa attcactaat ggcagccttc acttggctat 4680
   tagcaggaat atcatggcat gaaaatttct gttctacctc taattcccaa tcaagatatg 4740
   cagcaggatc atatttacca ttaaaagatg gaattttaaa tttaatctta gaaaataagt 4800
   cattaggggg atgacgaacc acacgacgtg cacgaccacg gcgatctcca tcgtcctgct 4860
   cagtgtcacc gccgtattcc tgctccatct ttgtggtcaa tgcatcaagg cgtgccagga 4920
   tggtgtcgag tgtggtgcga gtcgccgttt gagcaaggtc aagttggttg aaacgctcgg 4980
   tcgtcgaagt gatcgttgaa tcaagccgtt catgcatcgt cctaatgtca gcagcaagtc 5040
   catcaacttg gccccttact tcctgcaact gggcatccac catgtcgtgt gctcctgcca 5100
   tagttagcgc aaacaccaaa aggagaaaaa ccaacgacaa aaacaggggt gtactgctca 5160
   caaggcgctc acactagtgc tgttatcaag ttcttatccg ttcttaccaa gccacagtgg 5220
   tgaactgcaa ccaacaggtg gaaccggtga aagattggat gagcgattgc ttggagaaac 5280
   agaaacctgc tcgtcgtaga aatatgtgga gttgtgggta ggctgcactc aagtcaagga 5340
   ttagcacgat caaacaataa tgcaaagttg aattatagtg caaaacacga aactatattg 5400
   ctggccacag gtgcaaagga tggatggatg gaaatagcag aatggcagta acgtaaatat 5460
   tgtactagtg atgccaaaaa ggcactagta caaatcacag gtgattttgt ttttcttttt 5520
   tgtatgattt ttttgatatt tttctcagca caagaagcaa caagatagga gctacacgaa 5580
   gtttcaccta aaacagatat cagatgtggt ctacagaaaa tcaggaagtt ctctgaaaag 5640
   cgtgcgagaa ctttgacgga tttttttctt tattttcctg aatttttttg acaattttgt 5700
   cgaaccccaa acagaccgta ggtgagtttg gccgggctca gaatggtgtc aactagctcc 5760
   tgtaaaaatt tcagattttt tggacacccg agcgaaaagt tatgcccggt ttaaggaagg 5820
   tacctcaaat tatgttttca aacgaccgga atgaaacaac cgtatccttt ctccttcgtt 5880
   gttttttgtt tctgtttttt tttttacgta accgaaggag aaaaacaagg aaacgatgtt 5940
   gactcggttt gttttttttt ctgttttttt ttctgttttt tttcgtaacc gaaggagaaa 6000
   atcaaggaaa acagccgttg actcggtttg ttttttctgt ttttttttga cgtaaccgaa 6060
   ggagaaaaac aaggaaacaa tgttgactcg gtttgtggtg tgatcaaacg agagatggtg 6120
   gcggcgctag ggtttgaatg gtggaagaac acaatgcaac cagcaacaaa tgacgcgaaa 6180
   gcacacaaat tcaacaatgc agattattga aagaaagtgc gaggctcaaa agggtgctgg 6240
   gataagatct aacctgaatt tttatgtggt tttgtggact gtaggaaaaa aaaacgctcg 6300
   ataaactcac cgatcaacct agaaatctga taccaattga tgaagctgag gtgcccgatc 6360
   tttcggcgag tagagataat tccgatttgg cggaagatga cccttgcgat ccgactacga 6420
   cgagcaagcc cgaggcgcca atgcaatcgc tgaaccaact ccctgtggtt accgaccttg 6480
   ctgatgcgag atcggcctga tcacgaagat cgtttcctgt gcgcaatcga agaacgaaca 6540
   agaataagat gcgagcaatc taatctatta ctcgagggtg gagttctgaa tacacgaaga 6600
   cagcgcagat tagcgcgtgt tcgagagtag ctaaggctaa cgtaaaacaa aactcaggaa 6660
   ataaaggagg cgcagctcct gaataaatag agagggggcg cagcccctag gggcggccaa 6720
   ccctaggtcg tccattatgg gccgcaattg ggctggtcgt ctattcttcc gggccttcgt 6780
   tctttaacaa catgatgtag ttcaattctc ttgcacgggc ccgagtcact ggcccaggtg 6840
   gaaggggtgg cgcctgggct ggagaaggtg ctgctggtgt agatgtgctc gtgttggtgt 6900
   tgatgtccgc atcaa                                                  6915
<210> 4
   <211> 7572
   <212> DNA
   <213> Zea mays
<220>
   <221> source
   <222> (1)...(7572)
   <223> CRM2
<400> 4
   tgatgaagac atccacacta ctgatgcatc tataccaata caagtaccaa tttctggtcc 60
   cattactcgc gctcgtgctc gtcaactcaa ccatcaggtg attacactct tgagttcatg 120
   tccatcatat ttagaccatg gagacccgtg cactcttgtt ttgcttagga atcagggaga 180
   agaccgaaag ggaaaaggat ttgaacatgc tggattcgga ctgcagaaga acaccaactt 240
   gtgacggtca ccacggtcag atgcgggctc ggattggaat gttcaagcac aacatggaaa 300
   gcttatcaag tctactttca tatggatccg gaattatagt catatctgtt ctgaggccgc 360
   cgtaatcatt gttttcttac cgagacattt cctgcctttt ctgcccatgg tgctgcgtca 420
   ccctattttg gcccaatggg tcgtgtatca agttaggtcc attagggacg catcctaggg 480
   ttgcagcacg accccaatac ccttgtggtc gtcctcccat gtttataaac cccctagccg 540
   ccaccaagaa cagcgggttt tgtttagatc aagtttagct ctcgctactt gcttgcaagc 600
   gcgcgtgcta gttcagccgc ccgtcttctt gtcttcggaa ccccaccata ttggagtttg 660
   atctttaaac ctacatttag atctggtaat tcagtacttg ttctacttgt tcttgctagt 720
   tcttcgattg cttgcaggac gagtgcccta gtggccaggg tgtcacgctc cacaagatcg 780
   tgacagccat aggaggtggt gtatcggttg ctaaggcgca gcgtctttgg aaggctgtag 840
   tcgggccgtg aacgtcgtct cctcccccaa tcgagttatt ccacaccctc tcatcgaaag 900
   atcgggcaat cacccaacgg gtgcacatca gttggtaatc agagcaaggt ttatcggtga 960
   gagatttact tttcttcgct gttttcttat ctcctatagt ccagaaaaag ccaaaaaaat 1020
   agtagattag ttttaccgca atcctataaa ccattgagca tttactagta ctacttagtt 1080
   agggcttgtt gagtttttgg ttgcatcggt tgtgtcgagt tgctggtctt agtttattcc 1140
   tttagagttt tgagttctac cacgttttgg tcaccacgag atccaccatc accaaaaaca 1200
   tctctggttc gtttttgcca ccacggatac atatcatatc cgatttggaa gtttaaatac 1260
   aatctggaaa gcttatctta tcttctttcc aacggatctg accttatctc aaaattcgtt 1320
   ctgagcgctc cgcaatcatc gtagagattt ctggactttc tatattaaca agatttgtta 1380
   aatctgattt aaaggggttg ttagcaatat ctttattgtt tgggttgtca tagtgaaaaa 1440
   aagggtttag gcccctgcaa aaaaaaacag aagaagaaga aaaaaaaaga atagaaaaga 1500
   aaaaaaagga aaagaagaag aagggggctg aatctctaaa tctgttgttt ctctttgtgc 1560
   tgtgctagtt gttctttttc agtgactacc tttgtgccta ggctcacgtc tctagcctgg 1620
   tttagcctag gaccagcaca gtaccaccgt tgaacgatta ttcagcttgc ttttgtaact 1680
   aacgtggtac tagtgtattc cttgcttcag cccacctaca actctacata tttcgactac 1740
   agtttgacag gtcgtgttgc tgcggcaccg atacacttat tccacggttg cagacttgtt 1800
   ggttgctgac ccctcctgtt gtgcaaggta agaattggta agagcttgtg tggcaggttg 1860
   agagtgagcg ccttgcagta gctacatcct aatagttgta gagtttttat tccttcacat 1920
   tttttttctt tgttgcctct gttcgtctaa ccatggcagg attggaggtt gatgatgctt 1980
   ctcgtaatat gccacactct cctcgcacca agggtatcat acaacacttt gtaaggctgg 2040
   tgaaaacgca cacggaaggt cttgataatg acatgcaggt gacgaatgaa aagatggggc 2100
   aattggaggc cacacagatc gacacaaaca ccaaacttgc aaatgtggaa atgacagttg 2160
   ctcatattga caagagcctt gtcgcactct tgaggcgatt tgatgagatg catgctaata 2220
   ccaatggtgg gcgtgatgag ggcgccgaag gtaactggga tgactatgtt gctgatactg 2280
   aacaagatga ccaagaagca cctaatcgcc ggcgactacg tactaaccgt agaggtatgg 2340
   gtggttttca ccgacgtgag gtacatggta atgatgatgc ttttagtaag gttaaattta 2400
   aaatacctcc ttttgatggt aaatatgacc ctgatgctta cattacttgg gagattgcgg 2460
   ttgatcaaaa gtttgcatgc catgaatttc ctgagaatgc gcgggttaga gctgctacta 2520
   gtgagtttac tgaatttgct tctgtttggt ggatagaaca tggtaagaag aatcctaata 2580
   acatgccaca aacttgggat gcgttgaaac gggtcatgcg ggctagattt gttccttctt 2640
   attatgcacg tgatatgtta aacaagttgc aacaattgag acagggtact aaaagtgtag 2700
   aagaatatta tcaggaatta caaatgggta tgctgcgttg taacatagag gagggtgagg 2760
   aatctgctat ggctagattt ttgggcgggt taaataggga aattcaggac atccttgctt 2820
   ataaagatta tgctaatgta acccgattgt ttcatcttgc ttgcaaagct gaaagggaag 2880
   tgcagggacg acgtgctagt gcaaggtcta atgtttctgc aggaaaatct acaccatggc 2940
   aacagcgcac gactacgtcc atgaccggcc gtacactagc accaactccc tcgccaagtc 3000
   gaccagcacc cccgccttcc tccagcgaca aaccacgtgc atcttccaca aattcagcaa 3060
   ccaaatctgc ccagaaacca gcaggtagtg cctcttcagt agcctccacg ggtagaacaa 3120
   gagatgttct gtgttatcga tgcaagggct atggacacgt gcagcgtgat tgtcctaatc 3180
   agcgtgtttt ggtggtaaaa gacgatggtg ggtattcctc tgctagtgat ttggatgaag 3240
   ctacacttgc tttgcttgcg gctgatgatg caggcactaa ggaaccaccc gaagaacaga 3300
   ttggtgcaga tgatgcagag cattatgaga gcctcattgt acagcgtgtg cttagtgcac 3360
   aaatggagaa ggcagagcag aatcagcgac atacgttgtt tcaaacaaag tgtgtcatta 3420
   aggagcgttc atgtcgtttg atcattgatg gaggtagctg caacaacttg gctagcagcg 3480
   acatggtgga gaagcttgca cttacgacca aaccgcaccc gcatccatat cacattcaat 3540
   ggctcaacaa tagtggtaag gtcaaggtaa ccaagctggt acgaattaat tttgctattg 3600
   gttcatatcg tgatgttgtt gactgtgatg ttgtgcctat ggatgcttgt aatattctgc 3660
   taggtagacc atggcaattt gattcagatt gtatgcatca tggtagatca aatcaatatt 3720
   ctctcataca ccatgataag aaaattattt tgcttcccat gtcccctgag gctattgtgc 3780
   gtgatgatgt tgctaaagct accaaagcta aaactgagaa caacaagaat attaaagttg 3840
   ttggtaataa caaagatggg ataaaattga aaggacattg cttgcttgca acaaaaactg 3900
   atgttaatga attatttgct tccactactg ttgcctacgc cttggtatgc aaggatgctt 3960
   tgatttcaat tcaagatatg cagcattctt tgcctcctgt tattactaac attttgcagg 4020
   agtattctga tgtatttcca agtgagatac cagaggggct gccacctata cgagggattg 4080
   agcaccaaat tgatcttatt cctggtgcat ctttgccgaa tcgtgcgcca tataggacaa 4140
   atccagagga aacaaaagaa attcagcgac aagtgcaaga actactcgac aaaggttacg 4200
   tgcgtgagtc tcttagtccg tgtgctgttc cggttatttt agtgcctaaa aaagatggaa 4260
   catggcgtat gtgtgttgat tgtagggcta ttaataatat cacgatacgt tatcgacacc 4320
   ctattccacg tttagatgat atgcttgatg aattgagtgg tgccattgtc ttttctaaag 4380
   ttgatttgcg tagtgggtac caccagattc gtatgaaatt gggagatgaa tggaaaactg 4440
   ctttcaaaac taagttcgga ttgtatgagt ggttagtcat gccttttggg ttaactaatg 4500
   cacctagcac tttcatgaga ttaatgaacg aggttttgcg tgccttcatt ggaaaatttg 4560
   tggtagtata ctttgatgac atattaatct acagcaaatc tatggatgaa catgttgatc 4620
   acatgcgtgc tgtttttaat gctttacgag atgcacgttt atttggtaac cttgagaagt 4680
   gcacattttg caccgatcga gtttcgtttc ttggttatgt tgtgactcca cagggaattg 4740
   aggttgatca agccaaggta gaagcgatac atggatggcc tatgccaaag actatcacac 4800
   aggtgcggag tttcctagga cttgctggct tctatcgccg ttttgtgaag gactttagca 4860
   ccattgctgc acctttgaat gagcttacga agaagggagt gcattttagt tggggcaaag 4920
   tacaagagca cgctttcaac gtgctgaaag ataagttgac acatgcacct ctcctccaac 4980
   ttcctgattt taataagact tttgagcttg aatgtgatgc tagtggaatt ggattgggtg 5040
   gtgttttgtt acaagaaggc aaacctgttg catattttag tgaaaaattg agtgggtctg 5100
   ttctaaatta ttctacttat gataaggaat tatatgctct tgtgcgaaca ttagaaacat 5160
   ggcagcatta tttgtggccc aaagagtttg ttattcattc tgatcatgaa tctttgaaac 5220
   atattcgtag tcaaggaaaa ctgaaccgta gacatgctaa gtgggttgaa tttatcgaat 5280
   cgtttcctta tgttattaag cacaagaaag gaaaagagaa tatcattgct gacgctttgt 5340
   ctaggagata tactttgctg aatcaacttg actacaaaat ctttggatta gagacgatta 5400
   aagaccaata tgttcatgat gctgatttta aagatgtgtt gctgcattgt aaagatggga 5460
   aaggatggaa caaatatatc gttagtgatg ggtttgtgtt tagagctaac aagctatgca 5520
   ttccagctag ctccgttcgt ttgttgttgt tacaggaagc acatggaggt ggcttaatgg 5580
   gacattttgg agcaaagaaa acggaggaca tacttgctgg tcatttcttt tggcccaaga 5640
   tgagaagaga tgtggtgaga ttggttgctc gttgcacgac atgccaaaag gcgaagtcac 5700
   ggttaaatcc acacggtttg tatttgcctc tacccgttcc tagtgctcct tgggaagata 5760
   tttctatgga ttttgtgctg ggattgccta ggactaggaa gggacgtgat agtgtgtttg 5820
   tggttgttga tagattttct aagatggcac atttcatacc atgtcataaa actgacgatg 5880
   ctactcatat tgctgatttg ttctttcgtg aaattgttcg cttgcatggt gtgcccaaca 5940
   caatcgtttc tgatcgtgat gctaaatttc ttagtcattt ttggaggact ttgtgggcaa 6000
   aattggggac taagctttta ttttctacta catgtcatcc tcaaactgat ggtcaaactg 6060
   aagttgtgaa tagaactttg tctactatgt taagggcagt tctaaagaag aatattaaga 6120
   tgtgggagga ctgtttgcct catattgaat ttgcttataa tcgatcattg cattctacta 6180
   caaagatgtg cccatttcag attgtatatg gtttgttacc tcgtgctcct attgatttaa 6240
   tgcctttgcc atcttctgaa aaactaaatt ttgatgctac taggcgtgct gaattgatgt 6300
   taaaactgca cgaaactact aaagaaaaca tagagcgtat gaatgctaga tataagtttg 6360
   ctagtgataa aggtagaaag gaaataaatt ttgaacctgg agatttagtt tggttgcatt 6420
   tgagaaagga aaggtttcct gaattacgaa aatctaaatt gttgcctcga gccgatggac 6480
   cgtttaaagt gctagagaaa attaacgaca atgcatatag gctagatctg cctgcagact 6540
   ttggggttag ccccacattt aacattgcag atttaaagcc ctacttggga gaggaagttg 6600
   agcttgagtc gaggacgact caaatgcaag aaggggagaa tgatgaagac atccacacta 6660
   ctgatgcatc tataccaata caagtaccaa tttctggtcc cattactcgc gctcgtgctc 6720
   gtcaactcaa ccatcaggtg attacactct tgagttcatg tccatcatat ttagagccat 6780
   ggagacccgt gcactcttgt tttgcttagg aatcagggag aagaccgaaa gggaaaagga 6840
   tttgaacatg ctggattcgg actgcagaag aacaccaact tgtgacggtc accacggtca 6900
   gatgcgggct cggattggaa tgttcaagca caacatggaa agcttatcaa gtctactttc 6960
   atatggatcc ggaattatag tcatatctgt tctgaggccg ccgtaatcat tgttttctta 7020
   ccgagacatt tcctgccttt tctgcccatg gtgctgcgtc accctatttt ggcccaatgg 7080
   gtcgtgtatc aagttaggtc cattagggac gcatcctagg gttgcagcac gaccccaata 7140
   cccttgtggt cgtcctccca tgtttataaa ccccctagcc gccaccaaga acagcgggtt 7200
   ttgtttagat caagtttagc tctcgctact tgcttgtaag cgcgcgtgct agttcagccg 7260
   cccgtcttct tgtcttcgga accccaccat attggagttt gattttgaaa cctacattta 7320
   gatctggtaa ttcagtactt gttctacttg ttcttgctag ttcttcgatt gcttgcagga 7380
   cgagtgccct agtggccagg gtgtcacgct ccacaagatc gtgacagcca taggaggtgg 7440
   tgtatcggtt gctaaggcgc agcgtctttg gaaggctgta gtcgggccgt gaacgtcgtc 7500
   tcctccccca atcgagttat tccacaccct ctcatcgaaa gatcgggcaa tcacccaacg 7560
   ggtgcacatc ag                                                     7572
<210> 5
   <211> 32
   <212> DNA
   <213> Artificial Sequence
<220>
   <223> Overgo telomere primer1
<400> 5
   agggtttagg gtttagggtt tagggtttag gg 32
<210> 6
   <211> 30
   <212> DNA
   <213> Artificial Sequence
<220>
   <223> Overgo telomere primer2
<400> 6
   ccctaaaccc taaaccctaa accctaaacc 30
<210> 7
   <211> 24
   <212> DNA
   <213> Artificial Sequence
<220>
   <223> Primer CentC-OVG-1-40-F Biocode 65644
<400> 7
   ggttccggtg gcaaaaactc gtgc 24
<210> 8
   <211> 24
   <212> DNA
   <213> Artificial Sequence
<220>
   <223> Primer CentC-OVG-1-40-R Biocode 65645
<400> 8
   tgtcggtgca tacaaagcac gagt 24
<210> 9
   <211> 24
   <212> DNA
   <213> Artificial Sequence
<220>
   <223> Primer CentC-OVG-51-90-F Biocode 65646
<400> 9
   gaatgggtga cgtgcgacaa cgaa 24
<210> 10
   <211> 24
   <212> DNA
   <213> Artificial Sequence
<220>
   <223> Primer CentC-OVG-51-90-R Biocode 65647
<400> 10
   ggtggtttct cgcaatttcg ttgt 24
<210> 11
   <211> 24
   <212> DNA
   <213> Artificial Sequence
<220>
   <223> Primer CentC-OVG-101-140-F Biocode 65648
<400> 11
   gttttggacc taaagtagtg gatt 24
<210> 12
   <211> 24
   <212> DNA
   <213> Artificial Sequence
<220>
   <223> Primer CentC-OVG-101-140-R Biocode 104790
<400> 12
   cacaacgaac atgcccaatc cact 24
<210> 13
   <211> 24
   <212> DNA
   <213> Artificial Sequence
<220>
   <223> Primer CRM1-LTR-OVG1-F Biocode 69509
<400> 13
   cttggtcttg gacagtacct cact 24
<210> 14
   <211> 24
   <212> DNA
   <213> Artificial Sequence
<220>
   <223> Primer CRM1-LTR-OVG2-F Biocode 69510
<400> 14
   cccttgcgat ccgactacga cgag 24
<210> 15
   <211> 24
   <212> DNA
   <213> Artificial Sequence
<220>
   <223> Primer CRM1-LTR-OVG3-F Biocode 69511
<400> 15
   tcacgaagat cgtttcctgt gcgc 24
<210> 16
   <211> 24
   <212> DNA
   <213> Artificial Sequence
<220>
   <223> Primer CRM1-LTR-OVG4-F Biocode 69512
<400> 16
   cagcgcagat tagcgcgtgt tcga 24
<210> 17
   <211> 24
   <212> DNA
   <213> Artificial Sequence
<220>
   <223> Primer CRM1-LTR-OVG5-F Biocode 69513
<400> 17
   ccaaccctag gtcgtccatt atgg 24
<210> 18
   <211> 24
   <212> DNA
   <213> Artificial Sequence
<220>
   <223> Primer CRM1-LTR-OVG6-F Biocode 69514
<400> 18
   ttcaattctc ttgcacgggc ccga 24
<210> 19
   <211> 24
   <212> DNA
   <213> Artificial Sequence
<220>
   <223> Primer CRM1-LTR-OVG1-R Biocode 69515
<400> 19
   tcaggtctac ttcatcagtg aggt 24
<210> 20
   <211> 24
   <212> DNA
   <213> Artificial Sequence
<220>
   <223> Primer CRM1-LTR-OVG2-R Biocode 69516
<400> 20
   tggcgcctcg ggcttgctcg tcgt 24
<210> 21
   <211> 24
   <212> DNA
   <213> Artificial Sequence
<220>
   <223> Primer CRM1-LTR-OVG3-R Biocode 69517
<400> 21
   tgttcgttct tcgattgcgc acag 24
<210> 22
   <211> 24
   <212> DNA
   <213> Artificial Sequence
<220>
   <223> Primer CRM1-LTR-OVG4-R Biocode 69518
<400> 22
   ttagccttag ctactctcga acac 24
<210> 23
   <211> 24
   <212> DNA
   <213> Artificial Sequence
<220>
   <223> Primer CRM1-LTR-OVG5-R Biocode 69519
<400> 23
   ccagcccaat tgcggcccat aatg 24
<210> 24
   <211> 24
   <212> DNA
   <213> Artificial Sequence
<220>
   <223> Primer CRM1-LTR-OVG6-R Biocode 69520
<400> 24
   cacctgggcc agtgactcgg gccc 24
<210> 25
   <211> 24
   <212> DNA
   <213> Artificial Sequence
<220>
   <223> Primer CRM2-LTR-OVG1-F Biocode 69521
<400> 25
   tgatgaagac atccacacta ctga 24
<210> 26
   <211> 24
   <212> DNA
   <213> Artificial Sequence
<220>
   <223> Primer CRM2-LTR-OVG2-F Biocode 69522
<400> 26
   ttgaacatgc tggattcgga ctgc 24
<210> 27
   <211> 24
   <212> DNA
   <213> Artificial Sequence
<220>
   <223> Primer CRM2-LTR-OVG3-F Biocode 69523
<400> 27
   ctgcccatgg tgctgcgtca ccct 24
<210> 28
   <211> 24
   <212> DNA
   <213> Artificial Sequence
<220>
   <223> Primer CRM2-LTR-OVG4-F Biocode 69524
<400> 28
   gcgcgtgcta gttcagccgc ccgt 24
<210> 29
   <211> 24
   <212> DNA
   <213> Artificial Sequence
<220>
   <223> Primer CRM2-LTR-OVG5-F Biocode 69525
<400> 29
   gtatcggttg ctaaggcgca gcgt 24
<210> 30
   <211> 24
   <212> DNA
   <213> Artificial Sequence
<220>
   <223> Primer CRM2-LTR-OVG1-R Biocode 69526
<400> 30
   tattggtata gatgcatcag tagt 24
<210> 31
   <211> 24
   <212> DNA
   <213> Artificial Sequence
<220>
   <223> Primer CRM2-LTR-OVG2-R Biocode 69527
<400> 31
   aagttggtgt tcttctgcag tccg 24
<210> 32
   <211> 25
   <212> DNA
   <213> Artificial Sequence
<220>
   <223> Primer CRM2-LTR-OVG3-R Biocode 69528
<400> 32
   cccattgggc caaaataggg tgacg 25
<210> 33
   <211> 24
   <212> DNA
   <213> Artificial Sequence
<220>
   <223> Primer CRM2-LTR-OVG4-R Biocode 69529
<400> 33
   ttccgaagac aagaagacgg gcgg 24
<210> 34
   <211> 24
   <212> DNA
   <213> Artificial Sequence
<220>
   <223> Primer CRM2-LTR-OVG5-R Biocode 69530
<400> 34
   ctacagcctt ccaaagacgc tgcg 24
<210> 35
   <211> 24
   <212> DNA
   <213> Artificial Sequence
<220>
   <223> Primer CentA-LTR-OVG1-F Biocode 69531
<400> 35
   tgatgagaac ataacccgca caga 24
<210> 36
   <211> 24
   <212> DNA
   <213> Artificial Sequence
<220>
   <223> Primer CentA-LTR-OVG2-F Biocode 69532
<400> 36
   aggatgatga ggacatcact gcca 24
<210> 37
   <211> 24
   <212> DNA
   <213> Artificial Sequence
<220>
   <223> Primer CentA-LTR-OVG3-F Biocode 69533
<400> 37
   aaccatctag aatttgagaa ggca 24
<210> 38
   <211> 24
   <212> DNA
   <213> Artificial Sequence
<220>
   <223> Primer CentA-LTR-OVG4-F Biocode 69534
<400> 38
   gtccagaaac tgccgagtga actc 24
<210> 39
   <211> 24
   <212> DNA
   <213> Artificial Sequence
<220>
   <223> Primer CentA-LTR-OVG5-F Biocode 65535
<400> 39
   gagagagttt cgttctccat taga 24
<210> 40
   <211> 24
   <212> DNA
   <213> Artificial Sequence
<220>
   <223> Primer CentA-LTR-OVG6-F Biocode 69536
<400> 40
   gttcttgctt gttctcgatt gctt 24
<210> 41
   <211> 24
   <212> DNA
   <213> Artificial Sequence
<220>
   <223> Primer CentA-LTR-OVG7-F Biocode 69537
<400> 41
   ttggttgtgg tagtcgggca gcca 24
<210> 42
   <211> 24
   <212> DNA
   <213> Artificial Sequence
<220>
   <223> Primer CentA-LTR-OVG1-R Biocode 69538
<400> 42
   cattaacatg gtcatatctg tgcg 24
<210> 43
   <211> 24
   <212> DNA
   <213> Artificial Sequence
<220>
   <223> Primer CentA-LTR-OVG2-R Biocode 69539
<400> 43
   tggtgtggtg tattgatggc agtg 24
<210> 44
   <211> 23
   <212> DNA
   <213> Artificial Sequence
<220>
   <223> Primer CentA-LTR-OVG3-R Biocode 69540
<400> 44
   cttttattgc cttgttgcct tct 23
<210> 45
   <211> 24
   <212> DNA
   <213> Artificial Sequence
<220>
   <223> Primer CentA-LTR-OVG4-R Biocode 69541
<400> 45
   gacttgggta gagcaggagt tcac 24
<210> 46
   <211> 24
   <212> DNA
   <213> Artificial Sequence
<220>
   <223> Primer CentA-LTR-OVG5-R Biocode 69542
<400> 46
   aggaatagaa aggagttcta atgg 24
<210> 47
   <211> 24
   <212> DNA
   <213> Artificial Sequence
<220>
   <223> Primer CentA-LTR-OVG6-R Biocode 69543
<400> 47
   acagccttga acctgcaagc aatc 24
<210> 48
   <211> 24
   <212> DNA
   <213> Artificial Sequence
<220>
   <223> Primer CentA-LTR-OVG7-R Biocode 69544
<400> 48
   tgttggagaa cgacgttggc tgcc 24
<210> 49
   <211> 24
   <212> DNA
   <213> Artificial Sequence
<220>
   <223> Primer Cent4-250-OVG1-F Biocode 69555
<400> 49
   taagtgcaaa ccattgttaa attt 24
<210> 50
   <211> 24
   <212> DNA
   <213> Artificial Sequence
<220>
   <223> Primer Cent4-250-OVG2-F Biocode 69556
<400> 50
   cacaaaccct taactcgaaa ctat 24
<210> 51
   <211> 24
   <212> DNA
   <213> Artificial Sequence
<220>
   <223> Primer Cent4-250-OVG3-F Biocode 69557
<400> 51
   atcgaaagat aactcatatg gctt 24
<210> 52
   <211> 24
   <212> DNA
   <213> Artificial Sequence
<220>
   <223> Primer Cent4-250-OVG4-F Biocode 69558
<400> 52
   tccactaaag aaccaagatt gtga 24
<210> 53
   <211> 24
   <212> DNA
   <213> Artificial Sequence
<220>
   <223> Primer Cent4-250-OVG1-R Biocode 69559
<400> 53
   aattgtacta tctctaaaat ttaa 24
<210> 54
   <211> 24
   <212> DNA
   <213> Artificial Sequence
<220>
   <223> Primer Cent4-250-OVG2-R Biocode 69560
<400> 54
   tttagggttt ggggttatag tttc 24
<210> 55
   <211> 24
   <212> DNA
   <213> Artificial Sequence
<220>
   <223> Primer Cent4-250-OVG3-R Biocode 69561
<400> 55
   gaccataatg gtcaaaaagc cata 24
<210> 56
   <211> 24
   <212> DNA
   <213> Artificial Sequence
<220>
   <223> Primer Cent4-250-OVG4-R Biocode 69562
<400> 56
   atatgttgga cacaaatcac aatc 24
<210> 57
   <211> 24
   <212> DNA
   <213> Artificial Sequence
<220>
   <223> Primer 18-26SrDNANTS-OVG1-F Biocode 69634
<400> 57
   ccggaaataa gcaaagtcca agcg 24
<210> 58
   <211> 24
   <212> DNA
   <213> Artificial Sequence
<220>
   <223> Primer 18-26SrDNANTS-OVG2-F Biocode 69635
<400> 58
   tatgtcttgg gtgaagggca tggc 24
<210> 59
   <211> 24
   <212> DNA
   <213> Artificial Sequence
<220>
   <223> Primer 18-26SrDNANTS-OVG3-F Biocode 69636
<400> 59
   cgcaaggcga cgggcggcat ggct 24
<210> 60
   <211> 24
   <212> DNA
   <213> Artificial Sequence
<220>
   <223> Primer 18-26SrDNANTS-OVG4-F Biocode 69637
<400> 60
   cgaggggttc cccatggcgc acgg 24
<210> 61
   <211> 24
   <212> DNA
   <213> Artificial Sequence
<220>
   <223> Primer 18-26SrDNANTS-OVG1-R Biocode 69638
<400> 61
   tcggtgtctt tccacacgct tgga 24
<210> 62
   <211> 24
   <212> DNA
   <213> Artificial Sequence
<220>
   <223> Primer 18-26SrDNANTS-OVG2-R Biocode 69639
<400> 62
   gttttccctc cgttccgcca tgcc 24
<210> 63
   <211> 24
   <212> DNA
   <213> Artificial Sequence
<220>
   <223> Primer 18-26SrDNANTS-OVG3-R Biocode 69640
<400> 63
   agacgcaagg ccgaacagcc atgc 24
<210> 64
   <211> 24
   <212> DNA
   <213> Artificial Sequence
<220>
   <223> Primer 18-26SrDNANTS-OVG4-R Biocode 69641
<400> 64
   ggcctcagtt ttcggcccgt gcgc 24
<210> 65
   <211> 24
   <212> DNA
   <213> Artificial Sequence
<220>
   <223> Primer Subtelomere-266 Biocode 74794
<400> 65
   gacacatgtt tttgtcgtcg aaca 24
<210> 66
   <211> 24
   <212> DNA
   <213> Artificial Sequence
<220>
   <223> Primer Subtelomere-266 Biocode 74795
<400> 66
   ggaggcacga aatcgctgtt cgac 24
<210> 67
   <211> 24
   <212> DNA
   <213> Artificial Sequence
<220>
   <223> Primer Subtelomere-266 Biocode 74796
<400> 67
   cgaccgccac ccatgatttg acca 24
<210> 68
   <211> 24
   <212> DNA
   <213> Artificial Sequence
<220>
   <223> Primer Subtelomere-266 Biocode 74797
<400> 68
   accttaccag tctctatggt caaa 24
<210> 69
   <211> 24
   <212> DNA
   <213> Artificial Sequence
<220>
   <223> Primer Subtelomere-266 Biocode 74799
<400> 69
   tcccgtgagc tatagcacac gttt 24
<210> 70
   <211> 24
   <212> DNA
   <213> Artificial Sequence
<220>
   <223> Primer Subtelomere-266 Biocode 74800
<400> 70
   acacgttttc atggccgagc gacc 24
<210> 71
   <211> 24
   <212> DNA
   <213> Artificial Sequence
<220>
   <223> Primer Subtelomere-266 Biocode 74801
<400> 71
   ccgtgttcct ccacacgtgt tttt 24
<210> 72
   <211> 24
   <212> DNA
   <213> Artificial Sequence
<220>
   <223> Primer Subtelomere-266 Biocode 74802
<400> 72
   aaggtgctcc ggggacaaaa acac 24
<210> 73
   <211> 24
   <212> DNA
   <213> Artificial Sequence
<220>
   <223> Primer Subtelomere-266 Biocode 74803
<400> 73
   ttggcctccc gcgagctata tcac 24
<210> 74
   <211> 24
   <212> DNA
   <213> Artificial Sequence
<220>
   <223> Primer Subtelomere-266 Biocode 74803
<400> 74
   ttggccacgg aaatgtgtga tata 24
<210> 75
   <211> 24
   <212> DNA
   <213> Artificial Sequence
<220>
   <223> Primer Subtelomere-266 Biocode 74805
<400> 75
   ttatgtatcc gacctgccac cttc 24

<210> 76
   <211> 24
   <212> DNA
   <213> Artificial Sequence
<220>
   <223> Primer Subtelomere-266 BIocode 74806
<400> 76
   ctccccggtc taaaacgaag gtgg 24
<210> 77
   <211> 24
   <212> DNA
   <213> Artificial Sequence
<220>
   <223> Primer Subtelomere-266 Biocode 74807
<400> 77
   gccacccgtg agctatagca cacg 24
<210> 78
   <211> 24
   <212> DNA
   <213> Artificial Sequence
<220>
   <223> Primer Subtelomere-266 Biocode 74808
<400> 78
   taggtttcca taaaatcgtg tgct 24
<210> 79
   <211> 24
   <212> DNA
   <213> Artificial Sequence
<220>
   <223> Primer 180-knob-OVG-21-60-F Biocode 65650
<400> 79
   tgtcgaaaat agccatgaac gacc 24
<210> 80
   <211> 24
   <212> DNA
   <213> Artificial Sequence
<220>
   <223> Primer 180-knob-OVG-21-60-R Biocode 65651
<400> 80
   cggtattatt ggaaatggtc gttc 24
<210> 81
   <211> 24
   <212> DNA
   <213> Artificial Sequence
<220>
   <223> Primer 180-knob-OVG-71-110-F Biocode 65652
<400> 81
   cctacggatt tttgaccaag aaat 24
<210> 82
   <211> 24
   <212> DNA
   <213> Artificial Sequence
<220>
   <223> Primer 180-knob-OVG-71-110-R Biocode 65653
<400> 82
   atttctagtg gagaccattt cttg 24
<210> 83
   <211> 24
   <212> DNA
   <213> Artificial Sequence
<220>
   <223> Primer 180-knob-OVG-141-180-F Biocode 65654
<400> 83
   atgtggggtg aggtgtatga gcct 24
<210> 84
   <211> 24
   <212> DNA
   <213> Artificial Sequence
<220>
   <223> Primer 180-knob-OVG-141-180-R Biocode 65655
<400> 84
   atgagcctct ggtcgatgat caat 24
<210> 85
   <211> 24
   <212> DNA
   <213> Artificial Sequence
<220>
   <223> Primer 5S-rDNA-OVG-1-40-F Biocode 65656
<400> 85
   ggatgcgatc ataccagcac taaa 24
<210> 86
   <211> 24
   <212> DNA
   <213> Artificial Sequence
<220>
   <223> Primer 5S-rDNA-OVG-1-40-R Biocode 65657
<400> 86
   tgatgggatc cggtgcttta gtgc 24
<210> 87
   <211> 24
   <212> DNA
   <213> Artificial Sequence
<220>
   <223> Primer 5S-rDNA-OVG-61-100-F Biocode 65658
<400> 87
   cttgggcgag agtagtacta ggat 24
<210> 88
   <211> 24
   <212> DNA
   <213> Artificial Sequence
<220>
   <223> Primer 5S-rDNA-OVG-61-100-R Biocode 65659
<400> 88
   tcccaggagg tcacccatcc tagt 24
<210> 89
   <211> 24
   <212> DNA
   <213> Artificial Sequence
<220>
   <223> Primer 5S-rDNA-OVG-161-200-F Biocode 65660
<400> 89
   accatagtaa aaatgggtga ccgt 24
<210> 90
   <211> 23
   <212> DNA
   <213> Artificial Sequence
<220>
   <223> Primer 5S-rDNA-OVG-161-200-R Biocode 65661
<400> 90
   taatttaaca cgagaacggt cac 23
<210> 91
   <211> 24
   <212> DNA
   <213> Artificial Sequence
<220>
   <223> Primer 5S-rDNA-OVG-261-230-F Biocode 65662
<400> 91
   ccgtgggcga gccgagcacg gagg 24
<210> 92
   <211> 24
   <212> DNA
   <213> Artificial Sequence
<220>
   <223> Primer 5S-rDNA-OVG-261-230-R Biocode 65663
<400> 92
   tcctcttatg cccacacctc cgtg 24
<210> 93
   <211> 24
   <212> DNA
   <213> Artificial Sequence
<220>
   <223> Primer 350-knob-OVG-31-70-F Biocode 65664
<400> 93
   ctcaaatgac gtttctatga tatt 24
<210> 94
   <211> 24
   <212> DNA
   <213> Artificial Sequence
<220>
   <223> Primer 350-knob-OVG-31-70-R Biocode 65665
<400> 94
   tgaatacaat gccctcaata tcat 24
<210> 95
   <211> 24
   <212> DNA
   <213> Artificial Sequence
<220>
   <223> Primer 350-knob-OVG-121-160-F Biocode 65666
<400> 95
   ctaggtttcc tataatcccc tcta 24
<210> 96
   <211> 23
   <212> DNA
   <213> Artificial Sequence
<220>
   <223> Primer 350-knob-OVG-121-160-R Biocode 65667
<400> 96
   ctaggtatgc cttgaataga ggg 23
<210> 97
   <211> 24
   <212> DNA
   <213> Artificial Sequence
<220>
   <223> Primer 350-knob-OVG-161-200-F Biocode 65668
<400> 97
   atgttgttta tgtccactca agta 24
<210> 98
   <211> 24
   <212> DNA
   <213> Artificial Sequence
<220>
   <223> Primer 350-knob-OVG-161-200-R Biocode 65669
<400> 98
   atggtgtacg gtgttttact tgag 24
<210> 99
   <211> 24
   <212> DNA
   <213> Artificial Sequence
<220>
   <223> Primer 350-knob-OVG-261-300-F Biocode 65670
<400> 99
   gtgagatctg tccaaacata ggtt 24
<210> 100
   <211> 24
   <212> DNA
   <213> Artificial Sequence
<220>
   <223> Primer 350-knob-OVG-261-300-R Biocode 65671
<400> 100
   ggtgccttac aaccgtaacc tatg 24
<210> 101
   <211> 40
   <212> DNA
   <213> Artificial Sequence
<220>
   <223> Primer to b010.m7 fis31
<400> 101
   gcaaacttta tgtgatccct tcctcgctga acgagatgag 40
<210> 102
   <211> 40
   <212> DNA
   <213> Artificial Sequence
<220>
   <223> Primer to b108.h15 fis47
<400> 102
   gggacggcaa gtcacggtaa gaccagtcca accgaatgat 40
<210> 103
   <211> 40
   <212> DNA
   <213> Artificial Sequence
<220>
   <223> Primer to Cen3n.pk0001.g11
<400> 103
   ccaaacttgc tgagattact gggcaatctg ttcgctcgca 40
<210> 104
   <211> 24
   <212> DNA
   <213> Artificial Sequence
<220>
   <223> Overgo probe primer 23715-3101-3200f Biocode 103022
<400> 104
   ccaggtagtt tgaaacagta ttct 24
<210> 105
   <211> 24
   <212> DNA
   <213> Artificial Sequence
<220>
   <223> Overgo probe primer 23715-3501-3600f Biocode 103023
<400> 105
   ataaaggaaa agggcaaacc aaac 24
<210> 106
   <211> 24
   <212> DNA
   <213> Artificial Sequence
<220>
   <223> Overgo probe primer 23715-1401-1500f Biocode 103024
<400> 106
   gatgcccaca ttatagtgat tagc 24
<210> 107
   <211> 24
   <212> DNA
   <213> Artificial Sequence
<220>
   <223> Overgo probe primer 23715-2901-3000f Biocode 103025
<400> 107
   ccacatatag ctgctgcata tgcc 24
<210> 108
   <211> 24
   <212> DNA
   <213> Artificial Sequence
<220>
   <223> Overgo probe primer 23715-3701-3800f Biocode 103026
<400> 108
   cggatctaac acaaacatga acag 24
<210> 109
   <211> 24
   <212> DNA
   <213> Artificial Sequence
<220>
   <223> Overgo probe primer23715-1-100f Biocode 103027
<400> 109
   cgatgaattt tctcgggtgt tctc 24
<210> 110
   <211> 24
   <212> DNA
   <213> Artificial Sequence
<220>
   <223> Overgo probe primer 23715-101-200f Biocode 103028
<400> 110
   cctgcagccc taataattca gaag 24
<210> 111
   <211> 24
   <212> DNA
   <213> Artificial Sequence
<220>
   <223> Overgo probe primer 23715-301-400f Biocode 103029
<400> 111
   cacagtcgat gaatccagaa aagc 24
<210> 112
   <211> 24
   <212> DNA
   <213> Artificial Sequence
<220>
   <223> Overgo probe primer 23715-901-1000f Biocode 103030
<400> 112
   gcgtgcaatc catcttgttc aatc 24
<210> 113
   <211> 24
   <212> DNA
   <213> Artificial Sequence
<220>
   <223> Overgo probe primer 23715-3201-3300f Biocode 103031
<400> 113
   caaccacacc acatcatcac aacc 24
<210> 114
   <211> 24
   <212> DNA
   <213> Artificial Sequence
<220>
   <223> Overgo probe primer 23715-3601-3700f Biocode 103032
<400> 114
   actggcaagt tagcaatcag aacg 24
<210> 115
   <211> 24
   <212> DNA
   <213> Artificial Sequence
<220>
   <223> Overgo probe primer 23715-4901-5000f Biocode 103033
<400> 115
   catgaacgtg tcttcaacta gagg 24 <210> 116
   <211> 24
   <212> DNA
   <213> Artificial Sequence
<220>
   <223> Overgo probe primer 23715-4201-4300f Biocode 103034
<400> 116
   gacggcgttt aacaggctgg catt 24
<210> 117
   <211> 24
   <212> DNA
   <213> Artificial Sequence
<220>
   <223> Overgo probe primer 23715-201-300f Biocode 103035
<400> 117
   ccaagctctt cagcaatatc acgg 24
<210> 118
   <211> 24
   <212> DNA
   <213> Artificial Sequence
<220>
   <223> Overgo probe primer 23715-601-700f Biocode 103036
<400> 118
   atactttctc ggcaggagca aggt 24
<210> 119
   <211> 24
   <212> DNA
   <213> Artificial Sequence
<220>
   <223> Overgo probe primer 23715-1001-1100f Biocode 103037
<400> 119
   atccttggcg gcaagaaagc catc 24
<210> 120
   <211> 24
   <212> DNA
   <213> Artificial Sequence
<220>
   <223> Overgo probe primer 23715-1101-1200f Biocode 103038
<400> 120
   gcaagctacc tgctttctct ttgc 24
<210> 121
   <211> 24
   <212> DNA
   <213> Artificial Sequence
<220>
   <223> Overgo probe primer 23715-1601-1700f Biocode 103039
<400> 121
   gcttcttggc catgtagatg gact 24
<210> 122
   <211> 24
   <212> DNA
   <213> Artificial Sequence
<220>
   <223> Overgo probe primer 23715-1801-1900f Biocode 103040
<400> 122
   ttcacgccga tgaacttcac cttg 24
<210> 123
   <211> 24
   <212> DNA
   <213> Artificial Sequence
<220>
   <223> Overgo probe primer 23715-5001-5087f Biocode 103041
<400> 123
   aagcttgcca acgactacgc acta 24
<210> 124
   <211> 24
   <212> DNA
   <213> Artificial Sequence
<220>
   <223> Overgo probe primer 23715-401-500f Biocode 103042
<400> 124
   ccctgatgct cttcgtccag atca 24
<210> 125
   <211> 24
   <212> DNA
   <213> Artificial Sequence
<220>
   <223> Overgo probe primer23715-801-900f Biocode 103043
<400> 125
   agagcagccg attgtctgtt gtgc 24
<210> 126
   <211> 24
   <212> DNA
   <213> Artificial Sequence
<220>
   <223> Overgo probe primer 23715-1301-1400f Biocode 103044
<400> 126
   caggatcccg taactataac ggtc 24
<210> 127
   <211> 24
   <212> DNA
   <213> Artificial Sequence
<220>
   <223> Overgo probe primer 23715-2801-2900f Biocode 103045
<400> 127
   cgacctgcag aagtaacacc aaac 24
<210> 128
   <211> 24
   <212> DNA
   <213> Artificial Sequence
<220>
   <223> Overgo probe primer 23715-3401-3500f Biocode 103046
<400> 128
   atctagaacg accgcccaac caga 24
<210> 129
   <211> 24
   <212> DNA
   <213> Artificial Sequence
<220>
   <223> Overgo probe primer 23715-3801-3900f Biocode 103047
<400> 129
   atttggggga gatctggttg tgtg 24
<210> 130
   <211> 24
   <212> DNA
   <213> Artificial Sequence
<220>
   <223> Overgo probe primer 23715-3901-4000f Biocode 103048
<400> 130
   gagggggtgt ctatttatta cggc 24
<210> 131
   <211> 24
   <212> DNA
   <213> Artificial Sequence
<220>
   <223> Overgo probe primer 23715-4801-4900f Biocode 103049
<400> 131
   catgcaagct gatctgagct tggc 24
<210> 132
   <211> 24
   <212> DNA
   <213> Artificial Sequence
<220>
   <223> Overgo probe primer 23715-2101-2200f Biocode 103050
<400> 132
   tccatgcgca ccttgaagcg catg 24
<210> 133
   <211> 24
   <212> DNA
   <213> Artificial Sequence
<220>
   <223> Overgo probe primer 23715-501-600f Biocode 103051
<400> 133
   ttccatccga gtacgtgctc gctc 24
<210> 134
   <211> 24
   <212> DNA
   <213> Artificial Sequence
<220>
   <223> Overgo probe primer 23715-1201-1300f Biocode 103052
<400> 134
   atccactagt aacggccgcc agtg 24
<210> 135
   <211> 24
   <212> DNA
   <213> Artificial Sequence
<220>
   <223> Overgo probe primer 23715-4001-4100f Biocode 103053
<400> 135
   gccacgcaat ttctggatgc cgac 24
<210> 136
   <211> 24
   <212> DNA
   <213> Artificial Sequence
<220>
   <223> Overgo probe primer 23715-701-800f Biocode 103054
<400> 136
   cgatagccgc gctgcctcgt cttg 24
<210> 137
   <211> 24
   <212> DNA
   <213> Artificial Sequence
<220>
   <223> Overgo probe primer 23715-1901-2000f Biocode 103055
<400> 137
   cacttgaagc cctcggggaa ggac 24
<210> 138
   <211> 24
   <212> DNA
   <213> Artificial Sequence
<220>
   <223> Overgo probe primer 23715-1701-1800f Biocode 103056
<400> 138
   tccttcagct tcagggcctt gtgg 24
<210> 139
   <211> 24
   <212> DNA
   <213> Artificial Sequence
<220>
   <223> Overgo probe primer 23715-2001-2100f Biocode 103057
<400> 139
   caccttggag ccgtactgga actg 24
<210> 140
   <211> 24
   <212> DNA
   <213> Artificial Sequence
<220>
   <223> Overgo probe primer 23715-2601-2700f Biocode 103058
<400> 140
   tgcggctcgg tgcggaagtt cacg 24
<210> 141
   <211> 24
   <212> DNA
   <213> Artificial Sequence
<220>
   <223> Overgo probe primer 23715-4101-4200f Biocode 103059
<400> 141
   acgcgacgct gctggttcgc tggt 24
<210> 142
   <211> 24
   <212> DNA
   <213> Artificial Sequence
<220>
   <223> Overgo probe primer 23715-3101-3200r Biocode 103060
<400> 142
   cgttctagat cggagtagaa tact 24
<210> 143
   <211> 24
   <212> DNA
   <213> Artificial Sequence
<220>
   <223> Overgo probe primer 23715-3501-3600r Biocode 103061
<400> 143
   tgtttcgttg catagggttt ggtt 24
<210> 144
   <211> 24
   <212> DNA
   <213> Artificial Sequence
<220>
   <223> Overgo probe primer 23715-1401-1500r Biocode 33332
<400> 144
   gcacacatag tgacatgcta atca 24
<210> 145
   <211> 24
   <212> DNA
   <213> Artificial Sequence
<220>
   <223> Overgo probe primer 23715-2901-3000r Biocode 103062
<400> 145
   gatatacttg gatgatggca tatg 24
<210> 146
   <211> 24
   <212> DNA
   <213> Artificial Sequence
<220>
   <223> Overgo probe primer 23715-3701-3800r Biocode 103063
<400> 146
   cccggtagtt ctacttctgt tcat 24
<210> 147
   <211> 24
   <212> DNA
   <213> Artificial Sequence
<220>
   <223> Overgo probe primer 23715-1-100r Biocode 103064
<400> 147
   attcgagcca atatgcgaga acac 24
<210> 148
   <211> 24
   <212> DNA
   <213> Artificial Sequence
<220>
   <223> Overgo probe primer 23715-101-200r Biocode 103065
<400> 148
   gccttcttga cgagttcttc tgaa 24
<210> 149
   <211> 24
   <212> DNA
   <213> Artificial Sequence
<220>
   <223> Overgo probe primer 23715-301-400r Biocode 103066
<400> 149
   atggtggaaa atggccgctt ttct 24
<210> 150
   <211> 24
   <212> DNA
   <213> Artificial Sequence
<220>
   <223> Overgo probe primer 23715-901-1000r Biocode 103067
<400> 150
   gaggatcgtt tcgcatgatt gaac 24
<210> 151
   <211> 24
   <212> DNA
   <213> Artificial Sequence
<220>
   <223> Overgo probe primer 23715-3201-3300r Biocode 103068
<400> 151
   tgctttttgt tcgcttggtt gtga 24
<210> 152
   <211> 24
   <212> DNA
   <213> Artificial Sequence
<220>
   <223> Overgo probe primer 23715-3601-3700r Biocode 103069
<400> 152
   acctgtacgt cagacacgtt ctga 24
<210> 153
   <211> 24
   <212> DNA
   <213> Artificial Sequence
<220>
   <223> Overgo probe primer 23715-4901-5000r Biocode 103070
<400> 153
   aattaagtca ggcgcgcctc tagt 24
<210> 154
   <211> 24
   <212> DNA
   <213> Artificial Sequence
<220>
   <223> Overgo probe primer 23715-4201-4300r Biocode 103071
<400> 154
   cttgtttcga gtagataatg ccag 24
<210> 155
   <211> 24
   <212> DNA
   <213> Artificial Sequence
<220>
   <223> Overgo probe primer 23715-201-300r Biocode 103072
<400> 155
   acatagcgtt ggctacccgt gata 24
<210> 156
   <211> 24
   <212> DNA
   <213> Artificial Sequence
<220>
   <223> Overgo probe primer 23715-601-700r Biocode 103073
<400> 156
   gatctcctgt catctcacct tgct 24
<210> 157
   <211> 24
   <212> DNA
   <213> Artificial Sequence
<220>
   <223> Overgo probe primer 23715-1001-11OOr Biocode 103074
<400> 157
   cctgcaaagt aaactggatg gctt 24
<210> 158
   <211> 24
   <212> DNA
   <213> Artificial Sequence
<220>
   <223> Overgo probe primer 23715-1101-1200r Biocode 103075
<400> 158
   aagggaaaac gcaagcgcaa agag 24
<210> 159
   <211> 24
   <212> DNA
   <213> Artificial Sequence
<220>
   <223> Overgo probe primer 23715-1601-1700r Biocode 103076
<400> 159
   tacctggtgg agttcaagtc catc 24
<210> 160
   <211> 24
   <212> DNA
   <213> Artificial Sequence
<220>
   <223> Overgo probe primer 23715-1801-1900r Biocode 103077
<400> 160
   acggctgctt catctacaag gtga 24
<210> 161
   <211> 24
   <212> DNA
   <213> Artificial Sequence
<220>
   <223> Overgo probe primer 23715-5001-5087r Biocode 103078
<400> 161
   tgaagctctt gttggctagt gcgt 24
<210> 162
   <211> 24
   <212> DNA
   <213> Artificial Sequence
<220>
   <223> Overgo probe primer 23715-401-500r Biocode 103079
<400> 162
   gtcttgtcga tcaggatgat ctgg 24
<210> 163
   <211> 24
   <212> DNA
   <213> Artificial Sequence
<220>
   <223> Overgo probe primer 23715-801-900r Biocode 103080
<400> 163
   attcggctat gactgggcac aaca 24
<210> 164
   <211> 24
   <212> DNA
   <213> Artificial Sequence
<220>
   <223> Overgo probe primer 23715-1301-1400r Biocode 103081
<400> 164
   cgcttcgcta ccttaggacc gtta 24
<210> 165
   <211> 24
   <212> DNA
   <213> Artificial Sequence
<220>
   <223> Overgo probe primer 23715-2801-2900r Biocode 103082
<400> 165
   cgatgctcac cctgttgttt ggtg 24
<210> 166
   <211> 24
   <212> DNA
   <213> Artificial Sequence
<220>
   <223> Overgo probe primer 23715-3401-3500r Biocode 88245
<400> 166
   ggttgtgatg atgtggtctg gttg 24
<210> 167
   <211> 24
   <212> DNA
   <213> Artificial Sequence
<220>
   <223> Overgo probe primer 23715-3801-3900r Biocode 103083
<400> 167
   gttcggagcg cacacacaca caac 24
<210> 168
   <211> 24
   <212> DNA
   <213> Artificial Sequence
<220>
   <223> Overgo probe primer 23715-3901-4000r Biocode 103084
<400> 168
   tttcccttcc tcgcccgccg taat 24
<210> 169
   <211> 24
   <212> DNA
   <213> Artificial Sequence

<220>
   <223> Overgo probe primer 23715-4801-4900r Biocode 103085
<400> 169
   taaaacgacg gccagtgcca agct 24
<210> 170
   <211> 24
   <212> DNA
   <213> Artificial Sequence
<220>
   <223> Overgo probe primer 23715-2101-2200r Biocode 103086
<400> 170
   acgtcatcac cgagttcatg cgct 24
<210> 171
   <211> 24
   <212> DNA
   <213> Artificial Sequence
<220>
   <223> Overgo probe primer 23715-501-600r Biocode 103087
<400> 171
   agcgaaacat cgcatcgagc gagc 24
<210> 172
   <211> 24
   <212> DNA
   <213> Artificial Sequence
<220>
   <223> Overgo probe primer 23715-1201-1300r Biocode 103088
<400> 172
   aagccgaatt ccagcacact ggcg 24
<210> 173
   <211> 24
   <212> DNA
   <213> Artificial Sequence
<220>
   <223> Overgo probe primer 23715-4001-4100r Biocode 103089
<400> 173
   ttggacttgc tccgctgtcg gcat 24
<210> 174
   <211> 24
   <212> DNA
   <213> Artificial Sequence
<220>
   <223> Overgo probe primer 23715-701-800r Biocode 103090
<400> 174
   tgccctgaat gaactgcaag acga 24
<210> 175
   <211> 24
   <212> DNA
   <213> Artificial Sequence
<220>
   <223> Overgo probe primer 23715-1901-2000r Biocode 103091
<400> 175
   ccgactacaa gaagctgtcc ttcc 24
<210> 176
   <211> 24
   <212> DNA
   <213> Artificial Sequence
<220>
   <223> Overgo probe primer 23715-1701-1800r Biocode 103092
<400> 176
   tgctgaaggg cgagacccac aagg 24
<210> 177
   <211> 24
   <212> DNA
   <213> Artificial Sequence
<220>
   <223> Overgo probe primer 23715-2001-2100r Biocode 103093
<400> 177
   ggacatcctg tccccccagt tcca 24
<210> 178
   <211> 24
   <212> DNA
   <213> Artificial Sequence
<220>
   <223> Overgo probe primer 23715-2601-2700r Biocode 103094
<400> 178
   acatcgagac ctccaccgtg aact 24
<210> 179
   <211> 24
   <212> DNA
   <213> Artificial Sequence
<220>
   <223> Overgo probe primer 23715-4101-4200r Biocode 103095
<400> 179
   agtctaacgg acaccaacca gcga 24
<210> 180
   <211> 24
   <212> DNA
   <213> Artificial Sequence
<220>
   <223> PCR primer for bacm.pk108.h15.f unique sequence
<400> 180
   gatcgtcgaa tgggaatcca tggg 24
<210> 181
   <211> 28
   <212> DNA
   <213> Artificial Sequence
<220>
   <223> PCR primer for bacm.pk108.h15.r unique sequence
<400> 181
   ccctgagtga accatttagg aagatcag 28
<210> 182
   <211> 24
   <212> DNA
   <213> Artificial Sequence
<220>
   <223> PCR primer for bacm.pk108.h15-2 FIS47.f unique sequence
<400> 182
   tgcaacatcc aaagacccaa catg 24
<210> 183
   <211> 22
   <212> DNA
   <213> Artificial Sequence
<220>
   <223> PCR primer for bacm.pk108.h15-2 FIS47.r unique sequence
<400> 183
   ttccaacatg gttggtggtc ag 22
<210> 184
   <211> 26
   <212> DNA
   <213> Artificial Sequence
<220>
   <223> PCR primer for bacm.pk010.m07.fis31.f unique sequence
<400> 184
   tgtcatgaca tcttgttgct accctg 26
<210> 185
   <211> 22
   <212> DNA
   <213> Artificial Sequence
<220>
   <223> PCR primer for bacm.pk010.m07.fis31.r unique sequence
<400> 185
   aaacccggag tttctatgca gg 22
<210> 186
   <211> 591
   <212> DNA
   <213> Zea mays
<220>
   <221> misc feature
   <222> (1)...(591)
   <223> n = a, t, c, or g
<221> source
   <222> (1)...(591)
   <223> bacm.pk108.h15 unique sequence
<400> 186
   ttgctcgtaa cagatggttc angnnngatt gatcgtcgaa tgggaatcca tgggcaccca 60
   cttgaaattc aggttttctt tttgctacac ctagttatat tttctgtttc atacgggtct 120
   tttttcccaa gttgattttt tgtgattgtt tttgaggcac cttttaaaag aataaaatac 180
   acaaacattc ttcaaattgt ctgggaatgt catctaggtt cccaaacgat tagtttggat 240
   tcaaaacatc cctgatcttc ctaaatggtt cactcagggt tcgatccttc aaaatcagct 300
   agtccacgac catcctactt ggcagcccct acatctcttt ctcccccctc tcgttcacac 360
   cttgttaatg tccatcagca tagagtcttg ttagtgtcca cgtcgccagc caaaggattt 420
   accatggggt ttgcaccttg agtgaaccac ataacaagtt gagggacatg aaattgcaaa 480
   ttaatagctc agggatctcg ataacatgct tggacaagtt ttagcnactg ctgatgcatc 540
   ttagtcctat taaaagnntn nnnnacagtg cnacgncccc tattttacac g          591
<210> 187
   <211> 2000
   <212> DNA
   <213> Zea mays
<220>
   <221> source
   <222> (1)...(2000)
   <223> bacm.pk108.h15-2.fis47 unique sequence
<400> 187
   tgagaggctg atatgtcttc ttttttttct tattttttct aaaatcctga ttcctttcaa 60
   tgtcagtttt gaattccaaa ttcaaattta atttgattct caagcttcaa ttttaatgca 120
   acatccaaag acccaacatg aaatgcataa ttccatttta tttatcttat tttatctatt 180
   aaacaaagag tttcttaata tgaaacttat atacacaaaa gacactattc taagaaaaca 240
   attcctcata tatcatctta aatcttttgc tgaatattct ttaaacatta attctaaata 300
   gttttatttg tacaagaatt tgtgattatt tcctacgaag agatggttcc taggcactat 360
   aatgaatagt tttgctaatt aaacaattga aaatgtttct atgttctctg ttttaacctt 420
   agtttagagt ttttaacttc aagtttgaac taccaaagtt tgaacctctt tttttatttt 480
   ctttatactt ttaaaataca tttaaactca aatcttttgg agaaccttta taaatcccaa 540
   aatagggttt tgaggtgtta taaacgagga gtctgtgcat gggacggcaa gtcacggtaa 600
   gaccagtcca accgaatgat ggggcagctc ggtgtcgtct caattaatgc atctcttaag 660
   ttgctgacca ccaaccatgt tggaacctga tcagttggta cccattgtct gtttacatgt 720
   ggtagcttct aattggttcg cgtgatcttg aattttaaaa aattgaaata acatttttat 780
   aaaattagaa tctagcttgt ggtaacattg ctcactccat tgttgaatat atcaaaatgg 840
   agaaagcacg acgttaaaat gcttgcaaca tttatgtagg agtgttattt tatgtttttg 900
   cgaggagtat aatcgtagtg tcactgttga caatctttgg cgacttttag ctaaaggaga 960
   ggagagacaa tttcttgcta atgataggaa ttatagattg catatattga aagtgataga 1020
   gctagagtgc ccgatctttc ggtgagtgga gataattccg atttggtgga agtagaccct 1080
   cacgatccga ctacgacgag cgaacccgaa gcgccaatgc aatcgctgaa ccaactccca 1140
   atggttaccg accttgctta tgcgagatcg gcctgatcac gaagatcgtt tcctgtgcgc 1200
   aatcgaagaa cgaacaagaa aaagatgcga gcaatcttaa tatcactcga ggtggagttc 1260
   tgaatcacag aggacaacac gtatttgtgt gtgttctggg gtagctaaag ctagatgtaa 1320
   aacaaaactc aagttctaaa tgaaacagga ctctgactaa atagaggaga ggcgtgaaca 1380
   gtaggtcgac gctacagtac cacgtttact gttcacgact tacctaggcg ccccatccgg 1440
   gccttcctat tggaccatct tctaatcttc tgggccttcg ttctttaaca acatgatgta 1500
   gttcaattct cttgcacgag cctgagtcac tggcccaagt ggaagggtgg cgcctggact 1560
   agggaagatg gtgcctgggc tggagaaggt gctgctggtg tagatgtact catgttggtg 1620
   ttgatgtcct cctcatcctc cccttcttga actgaagtcg tcctcgacgg caactcctca 1680
   tcttctcccg catacggttt caaatctgca acattaaaat tagtggaaac accaaacttc 1740
   gcaggtaggt caaggatata agcattagca ttaatcttgg ttagtatctt aaaaggacca 1800
   gcaacacgag gcatcaattt agaacggcgc aaagtaggaa aacgatcctt tctcaaatgc 1860
   aacccaacca tatcaccagg ttcaaaagta actaggtttc tgggctttac taccaataat 1920
   ctgatattta gcattagcag caacaatgtt ttgttgagtt tgttctggag gttatcattt 1980
   gttcacatgt gcacatcatc                                              2000
<210> 188
   <211> 1541
   <212> DNA
   <213> Zea mays
<220>
   <221> source
   <222> (1)...(1591)
   <223> bacm.pk010.m07-fis31 unique sequence
<400> 188
   atcgataccc ttaattggga gataactgtt atattaatag tgaaaaatcc atcctattat 60
   aatttttgtt atatctttta catggccatc agtaatcaca gttaagagtt ggacaaaggc 120
   actatggagc gtggcccttc taattgtgtt gctgaagttc ttattaaggt ttgccaattt 180
   tttggtaggt gttccaaagc acagaaggct cttgagctaa gccaactttt aggagtcaac 240
   atttcttcaa cacaggtact tctgaagtag tcatgagtca ttgcttatct gaagtaagga 300
   gagaaagtta ttgcttttct tttctgatgc gtgaggttgt gcatgtacat tctatagagt 360
   gtgcctttca agtactacat caataatttc ttatttcttc atagacctgt agcacctgaa 420
   tgaaacctat ttttgaactc tgcatttttt gaagatgaca ttatgtatca atttagttct 480
   gtattggttg ttgccaaaaa atttgtaaga tattgagata gataatatat gtgatatttt 540
   tttatgtagt tgttttgcaa taacattttg atgaatattt ttgtatatta ttgtaacaca 600
   taggcaccta actagtgtat atataggatg gaaacacaaa caccatcgac gtcgatgatc 660
   tcttggatac cattatctag tgtaggtgta catgggtatt atgattatca tagtgtgttt 720
   atggttgtca tgacatcttg ttgctaccct gacaggtaac ctaaaaatcc aaaacacaat 780
   gcaagtgcag gaatgtatga ccaatgcaaa agctaaagta aagtgctttc acattcgttg 840
   ccaatgaaag tttgaaaatg cttgaccaga ttttatcaac taataaatac tgtatataat 900
   aggtctaaat ttgtgataac aaaacaagaa aaatagaaaa gtggggaaac aaaaaatagt 960
   agaaagaggc gtggggagag gcgcgagaga ggtggttggt tgggggtgga ggaccgtcgg 1020
   ccttccccgt ttcgtcttcc ggtcctcgtt ttatcccctc gcccttgcac tcctccgccg 1080
   cacccctttc ctccttccgc cctccgccgc gcctggcttt ctcccttccc gagaggccac 1140
   gctccatccc cgcccgcgct gcctcccctg gcgatcggcg gcgaccgcgg cccaacgaaa 1200
   gcgtcgtggt cggccggtaa gagttccttg ctttcctcta cgcgttgctc ctgccgttac 1260
   gcaaacttta tgtgatccct tcctcgctga acgagatgag aagaaaagtt gctgactttc 1320
   gttcacacgt gcacctgcat agaaactccg ggttttggct tccgttggaa aattttgata 1380
   aatgacttgc cacgcttcgt cttacttcaa attcgttcaa attaattact tgctatgctt 1440
   catcttactt caaattcgtt cgattcttgc tgggttcctc tgattatatc tttttttgta 1500
   tgatcaagcg taaagatacc gtcgacctcc cgctttttga a 1541
<210> 189
   <211> 355
   <212> DNA
   <213> qArtificial Sequence
<220>
   <223> TELO-266 consensus 355 bp repeat
<400> 189
   attttagtgt cgaaaccatg gtaaaatgaa atttgagtct cccaaccata tcaactttgc 60
   tgacatagta gaattttagt gtccaaacca tagtacacat tttggtcccc ggaggccggt 120
   aaggctattt ttggcctccc gtvacacatg ttttcatcgt caaacaacga tttcatgcct 180
   cccgtccgcc acccatgatt tggccacwga gactgctaag gctrtttatg gcctcccgta 240
   agctatagca camgttttca tggtcgagcg actattttta agtacgtgtt ccaccacccg 300
   cgttttggtc cccagagcac cttaaagttg ttcttggcct cccacgagct gtagg 355
<210> 190
   <211> 430
   <212> DNA
   <213> Artificial Sequence
<220>
   <223> TR430 subtelomeric repeat
<400> 190
   gacatggtag aattttagtg tccaaaccaa agtacacgtt ttagtccccg gaggcccgta 60
   aggctatttt ggcctcccgt gacacatgtt tttgtcgtcg aacagcgatt tcgtgcctcc 120
   cgaccgccac ccatgatttg accatagaga ctggtaaggt tgttttggcc tcccgtgagc 180
   tatagcacac gttttcatgg ccgagcgacc atttttatgt ccgtgttcct ccacacgtgt 240
   ttttgtcccc ggagcacctt aaagcggttc ttggcctccc gcgagctata tcacacattt 300
   ccgtggccaa acaaccaatt ttatgtatcc gacctgccac cttcgtttta gaccggggag 360
   gccgttatgg caattttttt gccacccgtg agctatagca cacgatttta tggaaaccta 420
   gaccctaaat                                                        430
<210> 191
   <211> 10368
   <212> DNA
   <213> Zea mays
<400> 191
   ggttccggtg gcaaaaactc gtgctttgta tgcacccgac acccgttttc ggaaagggtg 60
   acgtgcgaca acgaaattgc gcgaaaccac cccaaacatg agttttggac ctaaagtagt 120
   ggattgggca tgtttgttgc tgatgtagct gaggtgcccg atctttcggc gagtagagat 180
   aattccgatt tggcggaaga tgacccttgc gatccgacta cgacgagcaa gcccgaggcg 240
   ccaatgcaat cgctgaacca actccctgtg gttaccgacc ttgctgatgc gagatcggcc 300
   tgatcacgaa gatcgtttcc tgtgcgcaat cgaagaacga acaagaacaa gatgcgagca 360
   atctaatcta ttactcgagg gtggagttct gaatacacga ggacagcgca gatttgcgcg 420
   tgttcggaag tagctaaggc taacgtaaaa caaaactccc caaaaataaa ggaggcgcag 480
   ctcctgtata aatagagagg gggcgcagcc cctaggggcg gccaacccta ggtcgtccat 540
   tatgggccgc aattgggctg gtcgtctatc cttccgggcc ttcgttcttt aacaacatga 600
   tgtagttcaa ttctcttgca cgggcccgag tcactggccc aggtggaagg ggtggcgcct 660
   gggctggaga aggtgctgct ggtgtagatg tgctcgtgtt ggtgttgatg tcctcatcat 720
   cctccccttc ttgaactgaa gtcgtcctcg acggcaactc ctcatcttct cccgcatacg 780
   gtttcaaatc tgcaacatta aaactagtgg aaacaccaaa ctccgcaggc aggtcaagga 840
   tataagcatt atcattaatc ttggttagta ccttaaaagg accagcagca cgaggcatca 900
   atttagaacg gcgcaaagta ggaaaacgat cctttctcaa atgcaaccaa accatatcac 960
   caggttcaaa agtaactagt ttccggcctt tactaccagt aatctgatat ttagcattag 1020
   cagcagcaat gttttgttga gtttgttcat ggagattaat catttgttca acatgtgcaa 1080
   gagcatctat gtgtggggcg tccgtagcat caagcgaaaa caaagcaata ggcgccctag 1140
   gaatgtaacc ataaacaatt tgaaaagggc acatctttgt agaagaatgt gttgcatgat 1200
   tataagcaaa ctcaacatga ggtaagcaat cctcccaacg tttcaaattt ttgtctaaaa 1260
   cagccctaag catggtagac aaagttcgat taactacctc agtttgacca tcagtctgag 1320
   ggtgacaagt ggtgctaaac agcaatttag ttcctaattt attccacaga gatctccaaa 1380
   aatgactcag aaacttggca tcacgatccg agactattgt attgggaata ccgtgcaaac 1440
   gaataatctc tctaaaaaac aattcagcaa cattgctagc atcatcagtc ttatgacaag 1500
   gtatgaaatg agccattttg gagaatcgat caacaaccac aaaaatgcta tccctcccct 1560
   tcttagttct aggcaatccc aaaacaaaat ccatcgaaat atcaatccaa gggaaagtag 1620
   gaacaggcaa aggcatatac aaaccatggt tgttcaaccg tgacttagct ttctgacaag 1680
   tagtgcagcg tgcaacaagg cgctcaacat cagcgcgcat ccgaggccaa aagaagtggg 1740
   cagccaacac ctcatgtgtc ttgtagacgc caaagtgccc catgagaccg cctccatgtg 1800
   cttcctgtaa caacaaaaga cgaaccgagc tagctggaac acacagcttg ttagcgcgaa 1860
   acaggaaccc atcctgtatg tgaaatttgc cccatggttt cccattaata caatggccga 1920
   aagcatcttt aaaatcagca tcgtcaacat attgatcttt tacagtgtgc aaaccaaaga 1980
   ttttaaaatc taactgtgac agcatggtat agcgacgaga caaagcatca gcaataacat 2040
   tgtccttccc gttcttgtgt ttaataatgt aaggaaaaga ctcaatgaat tctacccatt 2100
   tagcatgacg acggttcaga tttgtttggg tacgaatatg ttttaaagcc tcatgatcag 2160
   aatgaattat gaactcacga tgccaaagat agtgctgcca tgtatgtaaa gtgcgcacta 2220
   acgcgtaaag ctccttatca taagtagaat atttcagact agcaccgctt aatttttcac 2280
   taaaataagc aactggtttt ccttcttgta ataaaacagc acctagccca ataccgctag 2340
   catcgcattc aagctcaaat actttattaa aatcaggcaa ttgcaatagg ggagcttggg 2400
   ttaacttatc tttcaaagtg ctgaacgctt cctcctgcga atcactccaa gcaaatggca 2460
   catctttctt tgtaagctca tgtagaggcg ctgcaatgga gctaaaatca cgaacaaatc 2520
   tgcggtagaa accggcaagt ccaagaaagc tccgaatttg tgtgaccgtc gtcggtgtag 2580
   gccactcccg aatggcagca atcttgctgc tatccacctc aatgccctgt ggagtaacaa 2640
   cataaccaag aaacgagaca cgtcgtgtgc aaaagatgca tttttccatg ttagcgaata 2700
   actgggcggc acgcaatgca tcaaaaacag cacttaaatg ttccaaatgc tctttcttag 2760
   atttgctgta aataaggata tcatcgaaat aaacaaccac aaacaatcct atgaagggcc 2820
   tcagaacttc attcatcact cgcataaaag tgctgggagc attagtcaat ccaaacggca 2880
   taaccaacca ttcatataaa ccaaatttcg ttttgaaggc tgttttccat tcatcaccta 2940
   gtttcattct aatctggtgg taaccactac gcaaatcaat cttagtgaaa ataatggcac 3000
   cactaagctc atctagcata tcatcaaggc gtggtatagg atagcgataa cgaatagtga 3060
   tattattaat agcacgacag tctacacaca tacgccatga cccatccttc ttgggaacaa 3120
   gtaacacagg aacagagcaa gggctaagag actcacgaat gtatcctttg tcaagcaacg 3180
   cctttacctg gcgctgaatc tccttcgtct catccggatt tgtacggtat ggtgcgcggt 3240
   ttggaagctg tgcaccggga atgaggtcga tctggtgctc aatgccacga agcggtggga 3300
   gacccggtgg taagtctttg ggaaagacat cagcgtactc ctgcaaaagg ttagcaacca 3360
   tagggggaat agccaaagat ggtgcatcat caagtgaaat gaggacacta gagcatacaa 3420
   gtgcatagca tggcaaatga gcaccgtgta gatcatcaaa atcagcacgt gtagcaagta 3480
   aaacaggagc cttcaactta atttcagaag gaacagagtg cgatggatca agttgtttag 3540
   cagttatagc agctcgggca agatcatctt taacaatttg ttcaggtgtc attggatgta 3600
   taattatttt ctgaccctta aaaatgaaag aataatgatt cgaacgacca tgatgcaagc 3660
   tatcagtatc atattgccaa ggtcgaccaa gtaacaaaga gcatgcttcc atgggaataa 3720
   catcacaatc aacaaaatca gaataagcac ccatggagaa aggaactcgc acggaacgcg 3780
   tgatttttat tttaccacca tcattaagcc attgaatgtg atatgggttc ggatgcttac 3840
   gagtgggtaa cgacaacttt tcgaccagca tggtactcgc caaattgttg caactgccgc 3900
   tgtcgataat gatgcgaatt gaccgctcct gcacaacacc ctttgtatgg aacagagtgt 3960
   gtcgctgatt cttctcgggc aaagcgacct gtgtactgag aacacgctgc acaacaagac 4020
   tttcatacct atcagcgtcg cccgggttga catgtacctc cgccttagct gcatggtcag 4080
   tggcaagtag tgcatgttca atttcttcag aatcactagc ggaagagtac tcaccatcgt 4140
   cacgaatgag taaagtgcgc ttgtttggac agtcccgaat cacatggcca aatcctctac 4200
   agcgatgaca ctgaatatcc cgtgtacgac ctgtgggtgg ggcagtgctg gctggtttgg 4260
   tcgtcttctc gcgtggagta gtggtggacg tggatggcgc agggggaact ggtgctgagg 4320
   tggatgtcga gcttcgtcct gcaaaagggt tagaatattg cttcgagcgg cgtccctgca 4380
   cttcacgttc agctttgcaa gcatattcaa ataaagtagt catatcataa tattccttat 4440
   aatcaagtat atcttggatt tcgcggttta aaccaccacg aaaacgtgcc atagcagcgt 4500
   cgttgtcctc caatatccca caacgaatca tacctttttg taactcctgg aaatagtcct 4560
   caacagattg agaaccttgc tgaaaacgct gcattttatt aagcaaatca cgagcataat 4620
   acgaaggcac aaatcgatgt cgcatcgcag ctttcaactg atcccaagtg gttggaatgg 4680
   tagtgggatg ttttatttta aactcacgcc accaaattaa agcaaaatca gtaaattcac 4740
   taatagcagc tttaacctga gaatgtgcag caatatcatg gcatgaaaat ttttgttcaa 4800
   cctctaactc ccaatcaaga tatgcagcag gatcatattt gccattaaaa ggtggaattt 4860
   taaatttaat cttagaaaat aagtcattag gggattacgg aaccacacgg cgtgcacgac 4920
   cacggcgatc tccatcgtcc cactcagtgt caccgccgta gtcctgctcc atctttgtgg 4980
   tcaacgcatc aaggcgtgct aggatggtgt cgagtgtggt gcgagtcgcc gtttgagcaa 5040
   ggtcaagctg gttgaaacgc tcggtcgtcg aagtgaccgt tgaatcaagc cgttcatgca 5100
   tcgtcctaat gtcatcagca agtccatcaa cttgtccctt tacttccagc aactgggcat 5160
   ccaccgtgtc atgtgctcct gccatagtta gcgcaaacac caaaacacca aaaaaaacga 5220
   caaaaacagg ggtgtactgc tcacaaggcg ctcacactag tgctgttatc aaattcttat 5280
   ccgttcttac caagccacag tggtgaactg caaccaacag gtggaaccgg tgaaagattg 5340
   gatgagcgat tgcctggaga aacagaaacc tactcgttgt agaaatatgt ggagttctgg 5400
   gtaggctgca ctcaagtcaa ggattagcac gaccaaacaa taatgcaaag ttgaattata 5460
   gtgcaaaaca cgaaactata ttgctggcca caagtgcaaa ggacggatgg aactagcaga 5520
   atggcagtac cgtaaatatt gtactagcga ggccactagt aggaatcaca agtgattttg 5580
   tttttctttt ttgtatgatt tttttggtat ttttctcagc acaagaagca acaaaatagg 5640
   agctacacga agtttcacct aaaacagagt tcaaatgtgg tctacagaaa atcagaaagt 5700
   tctctaaaaa gcgtgcgaga actttgaagg attttttctt tattttcctg aatttttttt 5760
   gacaattttg tcgaacccaa acagaccgaa ggtcagtttg gccggcctca gaatggtgtc 5820
   aactagctcc tgtaaaaatt tcagattttt cggacacccg agcgaaaagt tatgcccggt 5880
   ttaaggaagg tacctcaaat tatgttttca aacgaccgga atgaaacaac cgtatccttt 5940
   ctccttcgtt gttttttgtt tctgtttttt tttttacgta accgaaggag aaaaacaagg 6000
   aaacgatgtt gactcggttt gttttttttt ctgttttttt ttctgttttt tttcgtaacc 6060
   gaaggagaaa atcaaggaaa acagccgttg actcggtttg ttttttctgt ttttttttga 6120
   cgtaaccgaa ggagaaaaac aaggaaacaa tgttgactcg gtttgtggtg tgatcaaacg 6180
   agagatggtg gcggcgctag ggtttgaatg gtggaagaac acaatgcaac cagcaacaaa 6240
   tgacgcgaaa gcacacaaat tcaacaatgc agattattga aagaaagtgc gaggctcaaa 6300
   agggtgctgg gataagatct aacctgaatt tttatgtggt tttgtggact gtaggaaaaa 6360
   aaaacgctcg ataaactcac cgatcaacct agaaatctga taccaattga tgaagctgag 6420
   gtgcccgatc tttcggcgag tagagataat tccgatttgg cggaagatga cccttgcgat 6480
   ccgactacga cgagcaagcc cgaggcgcca atgcaatcgc tgaaccaact ccctgtggtt 6540
   accgaccttg ctgatgcgag atcggcctga tcacgaagat cgtttcctgt gcgcaatcga 6600
   agaacgaaca agaataagat gcgagcaatc taatctatta ctcgagggtg gagttctgaa 6660
   tacacgaaga cagcgcagat tagcgcgtgt tcgagagtag ctaaggctaa cgtaaaacaa 6720
   aactcaggaa ataaaggagg cgcagctcct gaataaatag agagggggcg cagcccctag 6780
   gggcggccaa ccctaggtcg tccattatgg gccgcaattg ggctggtcgt ctattcttcc 6840
   gggccttcgt tctttaacaa catgatgtag ttcaattctc ttgcacgggc ccgagtcact 6900
   ggcccaggtg gaaggggtgg cgcctgggct ggagaaggtg ctgctggtgt agatgtgctc 6960
   gtgttggtgt tgatgtccgc atcaacatcg caatcaacat aatcagaata agaacccagc 7020
   gaaaagggga cacgtaccga acgtgttacc tttattttac caccatcatt aagccattga 7080
   atgtgatacg gatgtggatg tgtgcgagtg ggcaaggata atttctctac caacgctgta 7140
   cttgccaaat tgttgcagct gccactatcg atgatgatgc gaatcgaccg ttcgtgcacg 7200
   acgcccttgg tatggaatag agtgtgtcgc tgattttttt cggcctgggc aacctgtgtg 7260
   ctgagaacac gctgcacaac aagactctca tacctatcag cgtcgatggg atcaacgtgg 7320
   acttcctcat tttctgcatg gttagtggca atcatagcat gactagtttc ctcagaatca 7380
   ctggctgaag agtactcacc attgtcacgt ataagcaagg tacgcttgtt tgggcagtcc 7440
   cgaatcatgt gcccaaaccc tctgcaacga tggcactgaa tatcccgtgt acgtcctgtg 7500
   gaagaagcgg cgcctttggc agggggcgcc actggcttgg ccggccctgt gcgcgatgta 7560
   gtgctaggcg taggaggtgc agggctggaa ggagttgagc tgtgtgttgg accccggcct 7620
   gcaaaagagt tagtatatgt ctttgatcgt cgtccctgca cttcacgttc agctttgcaa 7680
   gcatattcaa acaatgtggt tatatcaaaa taatccttat aatcaagtat atcctgaatt 7740
   tccctgttca aaccaccacg aaaacgcgcc atagcagcgt catctgactc aaccaaacca 7800
   caacgaagca tacccttttg caactcctgg taatattcct caacagattg tgaaccttgt 7860
   tgaaaacgct gcattttgtt aagcaaatca cgagcataat aggaaggaac aaatctgtgg 7920
   cgcatggcag tttttaattg ggtccaagta atgacactgt taatgggaag tttttgttta 7980
   tactcacgcc accaaattaa agcaaaatca gtaaattcac taatggcagc cttcacttgg 8040
   ctattagcag gaatatcatg gcatgaaaat ttctgttcta cctctaattc ccaatcaaga 8100
   tatgcagcag gatcatattt accattaaaa gatggaattt taaatttaat cttagaaaat 8160
   aagtcattag ggggatgacg aaccacacga cgtgcacgac cacggcgatc tccatcgtcc 8220
   tgctcagtgt caccgccgta ttcctgctcc atctttgtgg tcaatgcatc aaggcgtgcc 8280
   aggatggtgt cgagtgtggt gcgagtcgcc gtttgagcaa ggtcaagttg gttgaaacgc 8340
   tcggtcgtcg aagtgatcgt tgaatcaagc cgttcatgca tcgtcctaat gtcagcagca 8400
   agtccatcaa cttggcccct tacttcctgc aactgggcat ccaccatgtc gtgtgctcct 8460
   gccatagtta gcgcaaacac caaaaggaga aaaaccaacg acaaaaacag gggtgtactg 8520
   ctcacaaggc gctcacacta gtgctgttat caagttctta tccgttctta ccaagccaca 8580
   gtggtgaact gcaaccaaca ggtggaaccg gtgaaagatt ggatgagcga ttgcttggag 8640
   aaacagaaac ctgctcgtcg tagaaatatg tggagttgtg ggtaggctgc actcaagtca 8700
   aggattagca cgatcaaaca ataatgcaaa gttgaattat agtgcaaaac acgaaactat 8760
   attgctggcc acaggtgcaa aggatggatg gatggaaata gcagaatggc agtaacgtaa 8820
   atattgtact agtgatgcca aaaaggcact agtacaaatc acaggtgatt ttgtttttct 8880
   tttttgtatg atttttttga tatttttctc agcacaagaa gcaacaagat aggagctaca 8940
   cgaagtttca cctaaaacag atatcagatg tggtctacag aaaatcagga agttctctga 9000
   aaagcgtgcg agaactttga cggatttttt tctttatttt cctgaatttt tttgacaatt 9060
   ttgtcgaacc ccaaacagac cgtaggtgag tttggccggg ctcagaatgg tgtcaactag 9120
   ctcctgtaaa aatttcagat tttttggaca cccgagcgaa aagttatgcc cggtttaagg 9180
   aaggtaccct caagttatgt tttcaaacga ccgggatgaa acaaccgtat cctttctcct 9240
   tcgttgtttt tttttgtttc tgtttttttt ttgacgtaac cgaaggagaa aaacaaggaa 9300
   acgatgttgc ctcggttttt ttttttctgt tttttttcgt aaccgaagga gaaaaacaag 9360
   gaaacggccg ttgactcggt ttgttttttc tgtttttttt tacgtaaccg aaggagaaaa 9420
   acaaggaaac aatgttgact cggtttgtgg cgtgatcaaa ggggagatgg tggcggcgct 9480
   aggatatgaa tggtggaaga acacaatgca accagcaaca aggaaacgcg aaagcacaca 9540
   aattcaacaa tgcagattat tgaaagaaag tgcgaggctc aaaagggtgc tgggataaga 9600
   actaacctga atttttatgt ggttttgtgg actgtaggaa aaaaaacgct cgataaactc 9660
   accgatcaac ctggaaatct gataccaatt gatgtagctg aggtgcccga tctttcggcg 9720
   agtagagata attccgattt ggcggaagat gacccttgcg atccgactac gacgagcaag 9780
   cccgaggtgc caatgcaatc gctgaaccaa ctccctgtgg ttaccgacct tgctgatgcg 9840
   agatcggcct gatcacgaag atcgtttcct gtgcgcaatc gaagaacgaa caagaacaag 9900
   atgcgagcaa tctaatctat tactcgaggg tggagttctg aatacacgag gacagcgcag 9960
   atttgcgcgt gttcggaagt agctaaggct aacgtaaaac aaaactccca aaaataaagg 10020
   aggcgcagct cctgtataaa tagagagggg gcgcagcccc taggggcggc caaccctagg 10080
   tcgtccatta tgggccgcaa ttgggctggt cgtctatcct tccgggcctt cgttctttaa 10140
   caacatgatg tagttcaatt ctcttgcacg ggcccgagtc actggcccag gtggaagggg 10200
   tggcgcctgg gctggagaag gtgctgctgg tgtagatgtg ctcgtgttgg tgttgatgtc 10260
   ctcatcacat gtttggggtg gtttcgcgca atttcgttgt cgcacgtcac acattccgaa 10320
   aacggttgtc ggggtgcata caaagcacga gtttttgaca ccggaacc             10368
<210> 192
   <211> 32
   <212> DNA
   <213> Artificial Sequence
<220>
   <223> Telo-31overgo primer1 Biocode 75319
<400> 192
   agggtttagg gtttagggtt tagggtttag gg 32
<210> 193
   <211> 31
   <212> DNA
   <213> Artificial Sequence
<220>
   <223> Telo-31overgo primer2 Biocode 39612
<400> 193
   ccctaaaccc taaaccctaa accctaaacc c 31

## Description

### FIELD OF THE INVENTION

This invention is in the field of plant biotechnology, in particular, this pertains to artificial minichromosomes and to methods of making such minichromosomes in a plant.

### BACKGROUND OF THE INVENTION

Recent advances in chromosome engineering have made it possible to alter the genome of plant, thus, altering its phenotype. When a transgene is integrated into a plant genome, it is usually in a random fashion and in an unpredictable copy number. Accordingly, research efforts have been directed toward better controlling transgene integration.

Given this need, researchers have wondered if the answer might lie in the use of artificial minichromosomes. These are man-made linear or circular DNA molecules constructed from cis-acting DNA sequence elements that provide replication and partitioning of the constructed minichromosomes.

It is believed that production of artificial chromosomes would reduce or eliminate some issues associated with random genomic integrations into a native plant chromosome, for example linkage drag due to association of the transgene with genomic material from the host plant. Artificial chromosomes may also provide means to deliver 10-100 times more genes than standard transformation vectors, and to provide large chromosomal segments for complementation and/or map-based cloning.

Three components have been identified for artificial chromosome replication, stability, and maintenance/inheritance: (i) autonomous replication sequences which function as an origin of replication; (ii) telomeres which function to stabilize and maintain the ends of linear chromosomes; and, (iii) centromeres which are the site of kinetochore assembly for proper chromosome segregation in mitosis and meiosis. Isolated centromeres from unicellular organisms, such as yeast, do not function in higher eukaryotes.

U.S. Patent 5,270,201, issued to Richards *et al.* on December 14, 1993, describes plant artificial chromosomes based on telomeres and, optionally, a centromere.

U.S. Patent 7,119,250, issued to Luo et al. on October 10, 2006, describes plant centromere compositions.

U.S. Patent 7,132,240, issued to Richards et al. on November 7, 2006, describes a method to isolate methylated centromere DNA potentially from any centromere in an organism.

U.S. Patent 7,193,128, issued to Copenhaver et al. on March 20, 2007, describes a method for generating or increasing revenue from crops using nucleic acid sequences of plant centromeres.

PCT Application having publication number WO 2007/030510 that was published on March 15, 2007 describes methods of making plants transformed with autonomous minichromosomes.

### SUMMARY OF THE INVENTION

The present invention concerns an artificial plant minichromosome comprising a functional centromere containing: (a) at least two arrays of tandem repeats of CentC in an inverted orientation wherein the first array comprises at least fifty copies of CentC and the second array comprises at least fifty copies of CentC; and, (b) at least one copy of a retrotransposable element, wherein the retrotransposable element is situated between the first and the second array.

In a second embodiment, an artificial plant minichromosome of the invention comprises a retrotransposable element selected from the group consisting of CentA, CRM1, and CRM2.

In a third embodiment, the artificial plant minichromosome of the invention also comprises at least one functional telomere.

In a fourth embodiment, the functional centromere, comprised by the artificial plant minichromosome specifically binds centromeric protein C (CENPC).

In a fifth embodiment, a corn plant can comprise any of the artificial minichromosomes of the invention.

In a sixth embodiment, the invention concerns an isolated polynucleotide comprising: (a) at least two arrays of tandem repeats of CentC in an inverted orientation wherein the first array comprises at least ten copies of CentC and the second array comprises at least ten copies of CentC; and, (b) at least one copy of a retrotransposable element, wherein the retrotransposable element is situated between the first and the second array.

In a seventh embodiment, the isolated polynucleotide of the invention comprises a retrotransposable element which is selected from the group consisting of CentA, CRM1, and CRM2.

In an eighth embodiment, the invention concerns a recombinant construct comprising any of the isolated polynucleotides of the invention as well as a transgenic corn plant comprising such recombinant constructs.

### BRIEF DESCRIPTION OF THE DRAWINGS

The invention can be more fully understood from the following detailed description and the accompanying drawings and Sequence Listing, which form a part of this application.

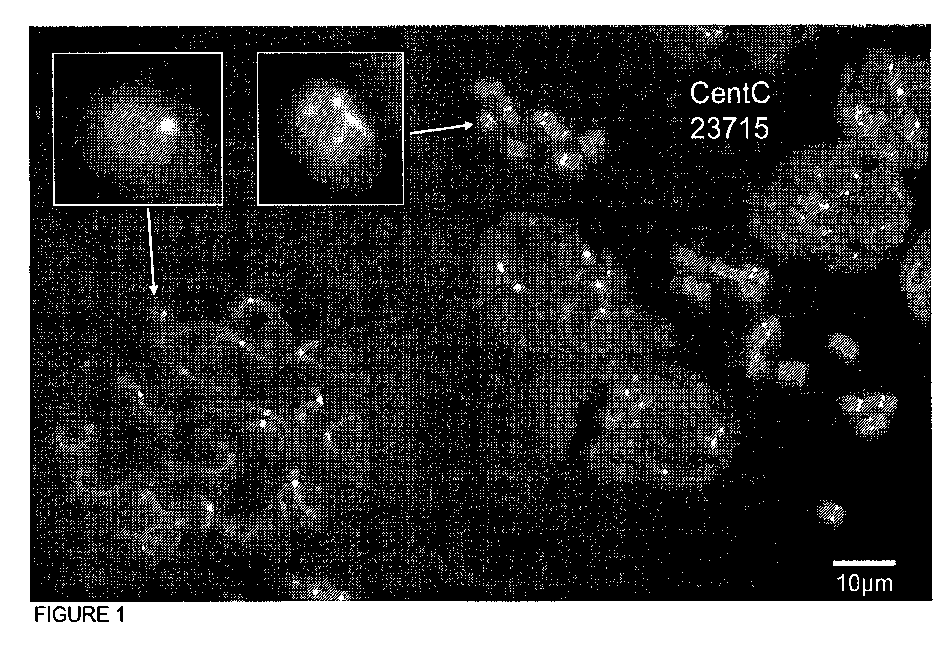

**Figure 1**. Fluorescent *in situ* hybridization (FISH) on a mitotic chromosomal spread of maize embryogenic calli from Hi-II transformation CMC3 pool 1 event #14. Calli were derived from immature embryos transformed with linearized BAC clone pool 1 retrofitted with Tn5-3. Prometaphase (left) and metaphase (right) nuclei both show the 20 native chromosomes plus 1 minichromosome (arrows and insets). Both minichromosomes are positive for the CentC (green - color; white - greyscale) centromere-specific repeat and the unique marker probe 23715 (red - color; white - greyscale) specific to the transformation construct, with both CentC and 23715 being essentially colocalized in the minichromosome (insets).

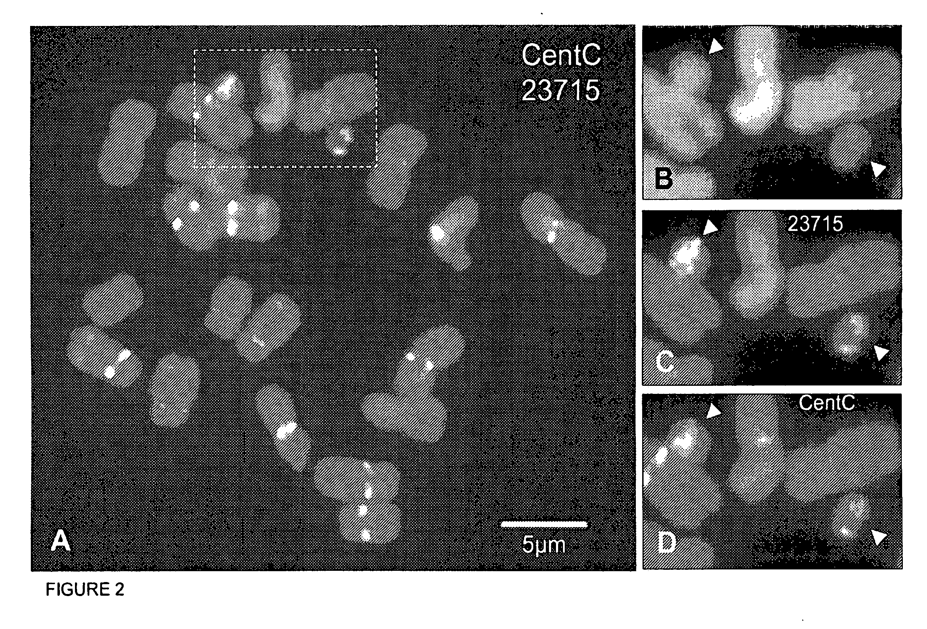

**Figure 2**. FISH on a mitotic chromosomal spread of maize embryogenic calli from Hi-II transformation CMC3 pool 1 event #14. Calli were derived from immature embryos transformed with linearized BAC clone pool 1 retrofitted with Tn5-3. Panel A shows a metaphase nucleus showing the 20 native chromosomes plus 2 minichromosomes (box). Both minichromosomes are positive for the CentC (green - color; white - greyscale) centromere-specific repeat and the unique marker probe 23715 (red - color; white - greyscale) specific to the transformation construct. Panels B-D are higher magnification of the box showing the minichromosomes (arrowheads) with: B - DAPI only; C - DAPI + 23715 probe (red - color; white - greyscale); and D - DAPI + CentC probe (green - color; white - greyscale).

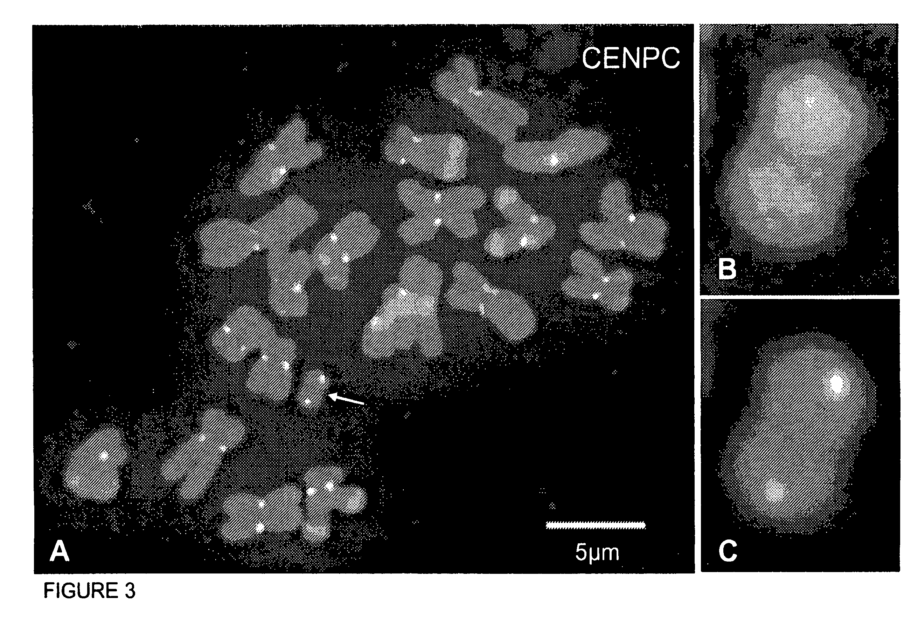

**Figure 3**. Immunofluorescence on a mitotic chromosomal spread of maize embryogenic calli from Hi-II transformation CMC3 pool 1 event #14. Calli were derived from immature embryos transformed with linearized BAC clone pool 1 retrofitted with Tn5-3. Panel A shows a metaphase nucleus showing the 20 native chromosomes plus 1 minichromosome (arrow). All centromeres of the native chromosomes and the minichromosome are positive for Centromeric Protein C, CENPC (red - color; white - greyscale), a centromere/kinetochore-specific protein. Panels B-C show higher magnification of the minichromosome with: B - DAPI only; and C - DAPI + CENPC (red - color; white - greyscale). The morphology and immunolocalization of CENPC indicates that the minichromosome is composed of two sister chromatids each having a functional centromere.

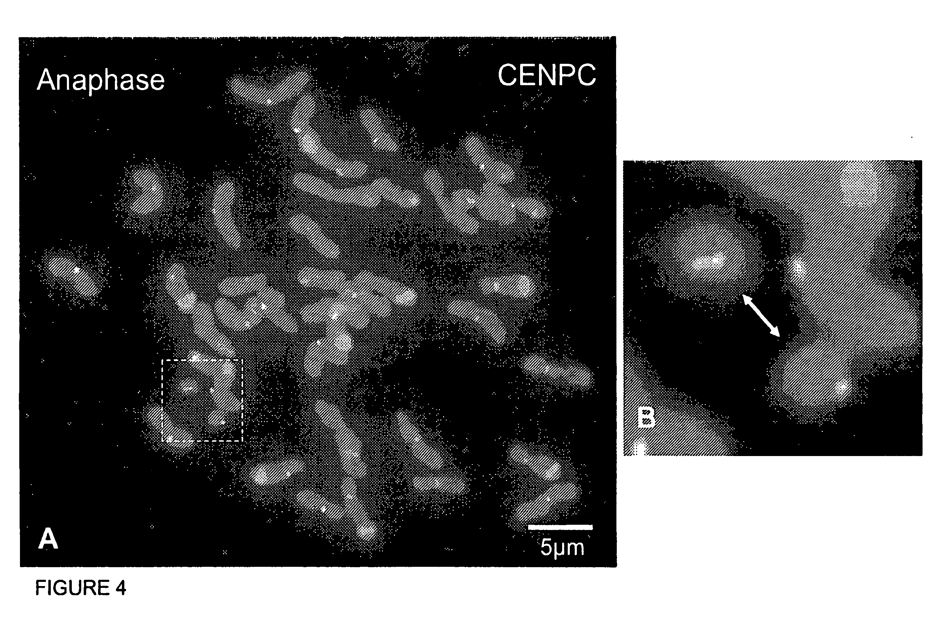

**Figure 4**. Panel A - Immunofluorescence on a mitotic chromosomal spread of maize embryogenic calli from Hi-II transformation CMC3 pool 1 event #14. Calli were derived from immature embryos transformed with linearized BAC clone pool 1 retrofitted with Tn5-3. Separation of sister chromatids of the native chromosomes and the minichromosome (box) was observed during anaphase. All centromeres of the native chromosomes and the minichromosome are positive for Centromeric Protein C, CENPC (red - color; white - greyscale) a centromere/kinetochore-specific protein. Panel B is a high magnification image of the box in A, showing the separation of the minichromosome sister chromatids (double arrow) indicating that the minichromosome, like normal chromosomes, can segregate during mitosis.

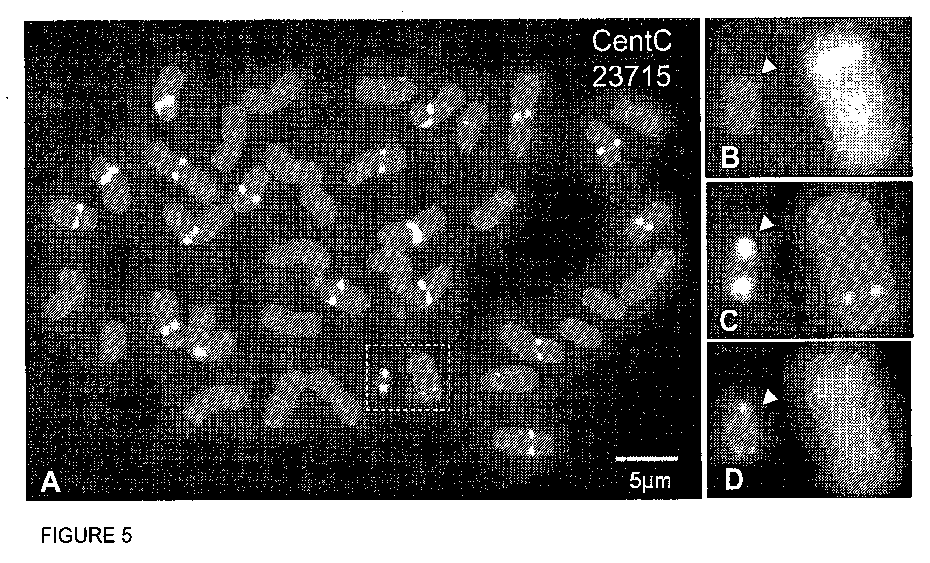

**Figure 5**. FISH on a mitotic chromosomal spread of maize embryogenic calli from Hi-II transformation CMC3 pool 3 event #12. Calli were derived from immature embryos transformed with linearized BAC clone pool 3 retrofitted with Tn5-3. A tetra-aneuploid (39 chromosomes, lacking one copy of ch 6) metaphase nucleus showing the native chromosomes plus 1 minichromosome (arrow) is shown. The minichromosome is positive for the CentC (green - color; white - greyscale) centromere-specific repeat and the unique marker probe 23715 (red - color; white - greyscale) specific to the transformation construct. Panels B-D are higher magnification of the boxed area showing the minichromosome (arrowheads) and a native chromosome with: A - DAPI only; B - DAPI + CentC probe (green - color; white - greyscale); and D - DAPI + 23715 probe (red - color; white - greyscale). Bipolar localization of CentC repeats as revealed by FISH staining at the minichromosome indicates that it is composed of two sister chromatids similar to that observed in the native chromosomes.

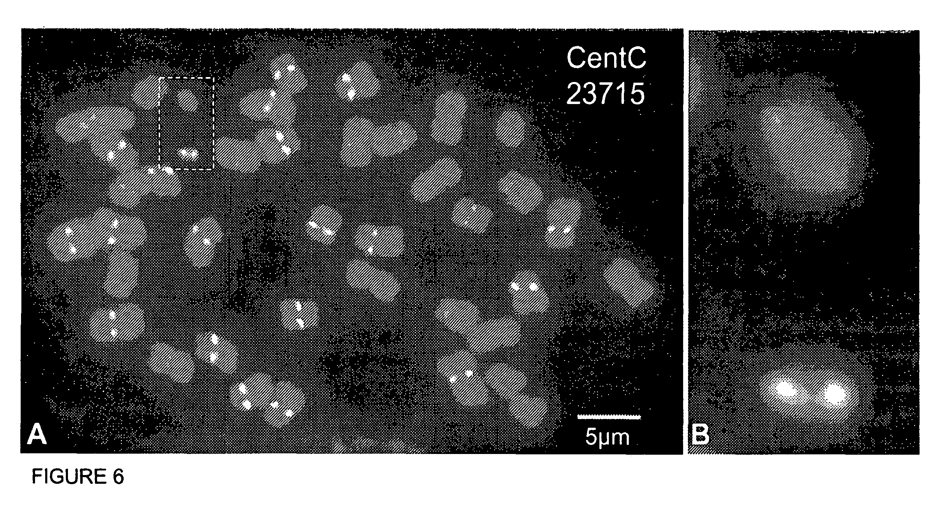

**Figure 6****.** FISH on a mitotic chromosomal spread of maize embryogenic calli from Hi-II transformation CMC3 pool 3 event #12. Calli were derived from immature embryos transformed with linearized BAC clone pool 3 retrofitted with Tn5-3. Panel A - Tetra-aneuploid (39 chromosomes, lacking one copy of ch 6) metaphase nucleus showing the native chromosomes plus 2 minichromosomes (arrows). Minichromosomes are positive for both the CentC (green - color; white - greyscale) centromere-specific repeat and the unique marker probe 23715 (red - color; white - greyscale) specific to the transformation construct. Panel B is a high magnification image of the 2 minichromosomes showing variation in the abundance of CentC repeats and the unique marker 23715.

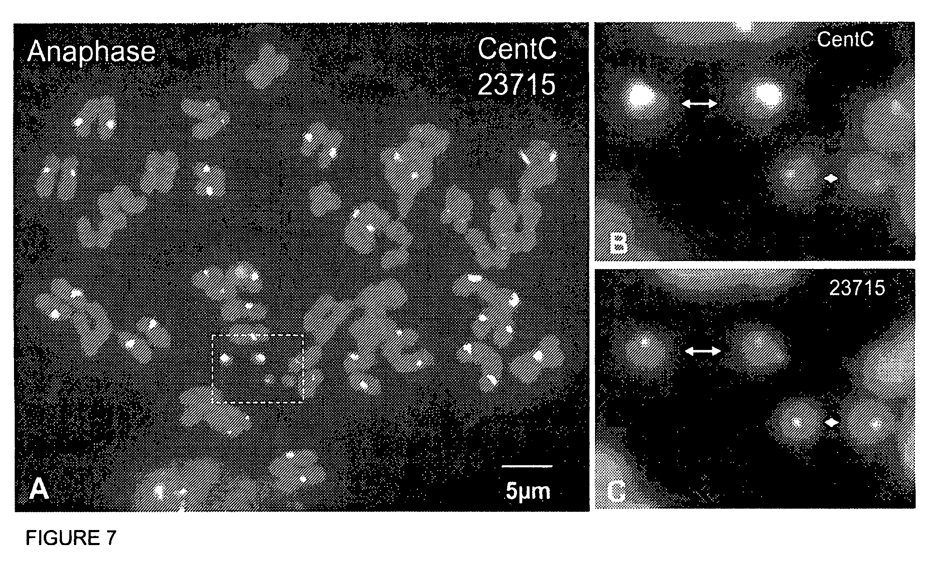

**Figure 7**. FISH on a mitotic chromosomal spread of maize embryogenic calli from Hi-II transformation CMC3 pool 3 event #12. Calli were derived from immature embryos transformed with linearized BAC clone pool 3 retrofitted with Tn5-3. Panel A - Tetra-aneuploid (39 chromosomes, lacking one copy of ch 6) nucleus showing separation of sister chromatids of the native chromosomes and the two minichromosomes (box) at early anaphase. The sister chromatids of both minichromosomes are positive for the CentC (green - color; white - greyscale) centromere-specific repeat and the unique marker probe 23715 (red - color; white - greyscale) specific to the transformation construct. Panels B-C are high magnification images of the 2 minichromosomes (double arrows) showing: B - DAPI + CentC probe (green - color; white - greyscale); and C - DAPI + 23715 probe (red - color; white - greyscale). Separation of the minichromosome sister chromatids at anaphase suggests the presence of functional centromeres, allowing for segregation during mitosis.

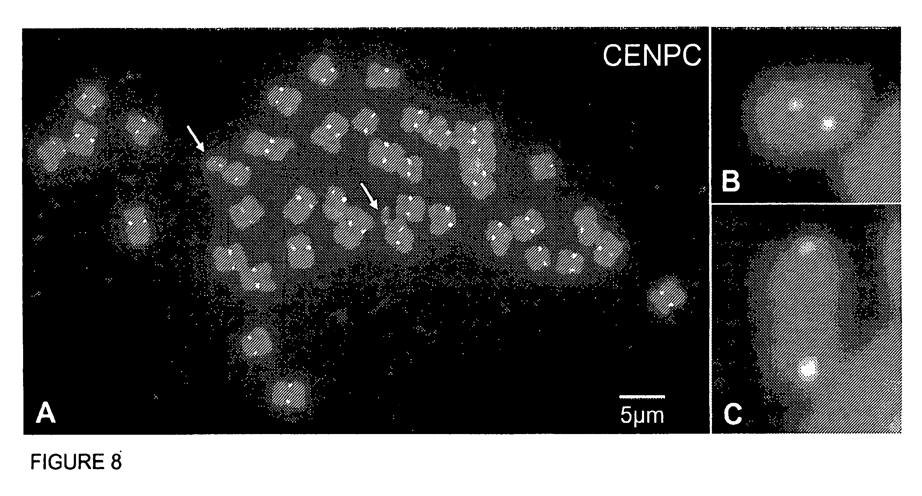

**Figure 8**. Immunofluorescence on a mitotic chromosomal spread of maize embryogenic calli from Hi-II transformation CMC3 pool 3 event #12. Calli were derived from immature embryos transformed with linearized BAC clone pool 3 retrofitted with Tn5-3. Panel A shows a tetra-aneuploid (39 chromosomes, lacking one copy of ch 6) metaphase nucleus showing 39 native chromosomes plus 2 minichromosomes (arrows). All centromeres of the native chromosomes and the minichromosomes are positive for Centromeric Protein C, CENPC (red - color; white - greyscale) a centromere/kinetochore-specific protein. Panels B-C are high magnification images of the minichromosomes. The pattern of CENPC immunolocalization, two foci per minichromosome, indicates that the minichromosome is composed of two sister chromatids and each has a functional centromere able to form a kinetochore complex.

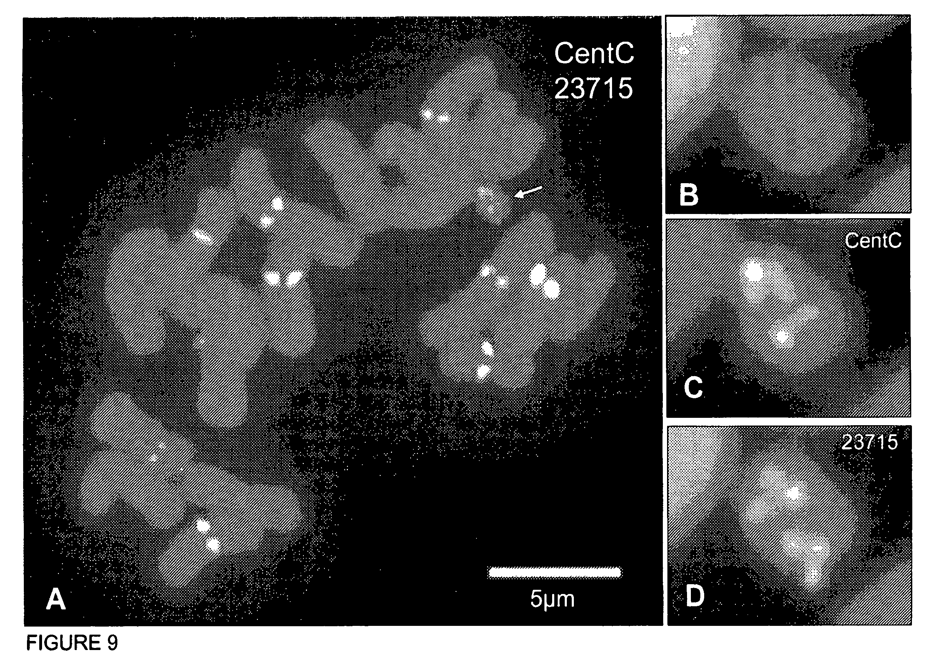

**Figure 9**. FISH on a mitotic chromosomal spread from root tips of a plant regenerated from a Hi-II maize transformation event. Plants were derived from immature embryos transformed with linearized bacm.pk128.j21 retrofitted with Tn5-3. Panel A shows an aneuploid metaphase nucleus showing 19 native chromosomes plus 1 minichromosome (arrow). The minichromosome is positive for the CentC (green - color; white - greyscale) centromere-specific repeat and the unique marker probe 23715 (red - color; white - greyscale) specific to the transformation construct. Panels B-D are higher magnifications of the minichromosome with: B - DAPI only; C - DAPI + CentC probe (green - color; white - greyscale); and D - DAPI + 23715 probe (red - color; white - greyscale).

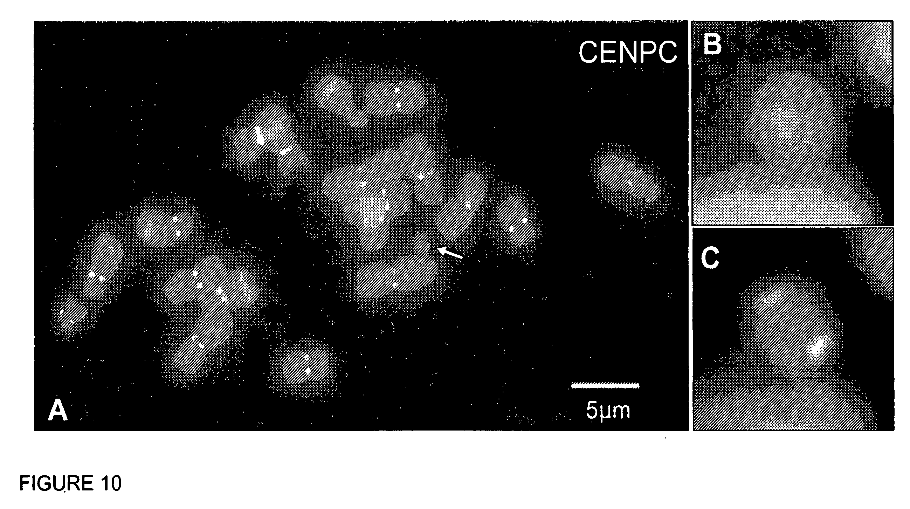

**Figure 10**. Immunofluorescence on a mitotic chromosomal spread from root tips of a plant regenerated from a Hi-II maize transformation event. Plants were derived form immature embryos transformed with linearized bacm.pk128.j21 retrofitted with Tn5-3. Panel A shows an aneuploid metaphase nucleus showing 19 native chromosomes plus 1 minichromosome (arrow). All centromeres of the native chromosomes and the minichromosome are positive for Centromeric Protein C, CENPC (red - color; white - greyscale), a centromere/kinetochore-specific protein. Panels B-C are higher magnification of the minichromosome with: B - DAPI only; and C - DAPI + CENPC. The pattern of CENPC immunolocalization, two foci per minichromosome, indicates that the minichromosome is composed of two sister chromatids and each has a functional centromere able to form a kinetochore complex.

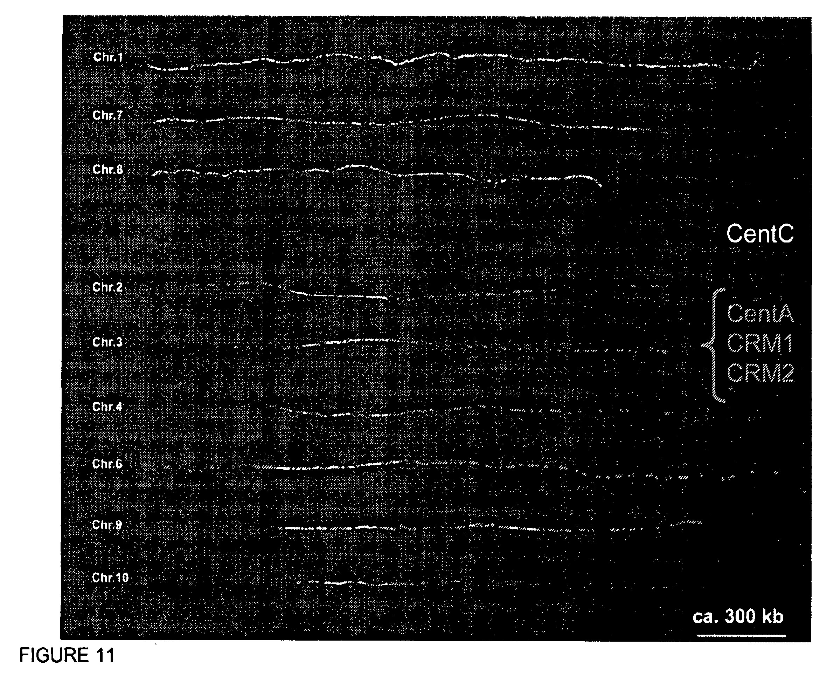

**Figure 11**. Fine structure of corn centromeres revealed by fiber-FISH. Four centromeric repeats, CentC (green - color; white - greyscale) and a sum of CentA, CRM1, and CRM2 (red - color; grey - greyscale) were used in multi-color FISH on extended DNA fibers of oat-maize addition lines containing individual corn chromosomes. This revealed megabase-long hybridization stretches, which are unique for each chromosome.

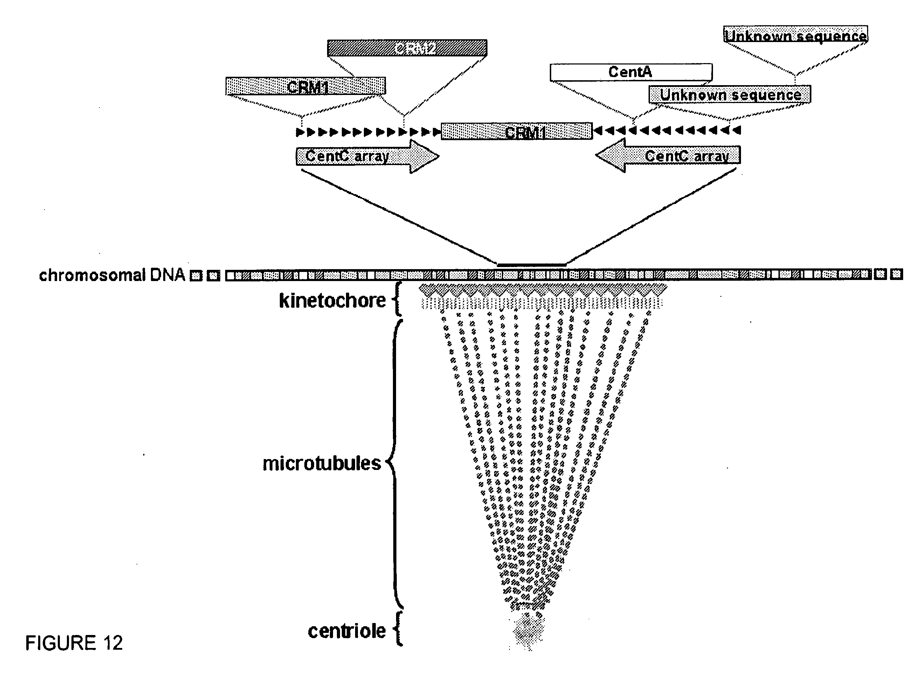

**Figure 12**. Model of a corn centromere. Centromeric organization is shown using maize centromeric repeat nomenclature. Uninterrupted arrays of CentC can be composed of several hundred to thousands of repeat elements. Other maize centromere-specific retrotransposable elements such as CentA, CRM1, and/or CRM2 can be integrated into a CentC array, into each other, and/or into itself in centromeric regions. In addition to centromere-specific retrotransposons, other retrotransposons can be integrated in the array, into elements such as CentA, CentC, CRM1, and CRM2, and/or into itself to form inserts which interrupt CentC tandem repeat arrays. This figure shows one model of the organization of maize CentC elements (arrowheads) forming two arrays of tandem head-to-tail repeats. The CentC arrays can be found in an inverted orientation to form a large segment of the centromeric DNA. Fiber-FISH along with FISH on meiotic anaphase chromosomes and blot-hybridization analysis of cloned centromeric DNA segments indicated that regions with high density of all four centromeric repeats (CentC, CRM1, CentA, and CRM2) are involved in formation of the kinetochore.

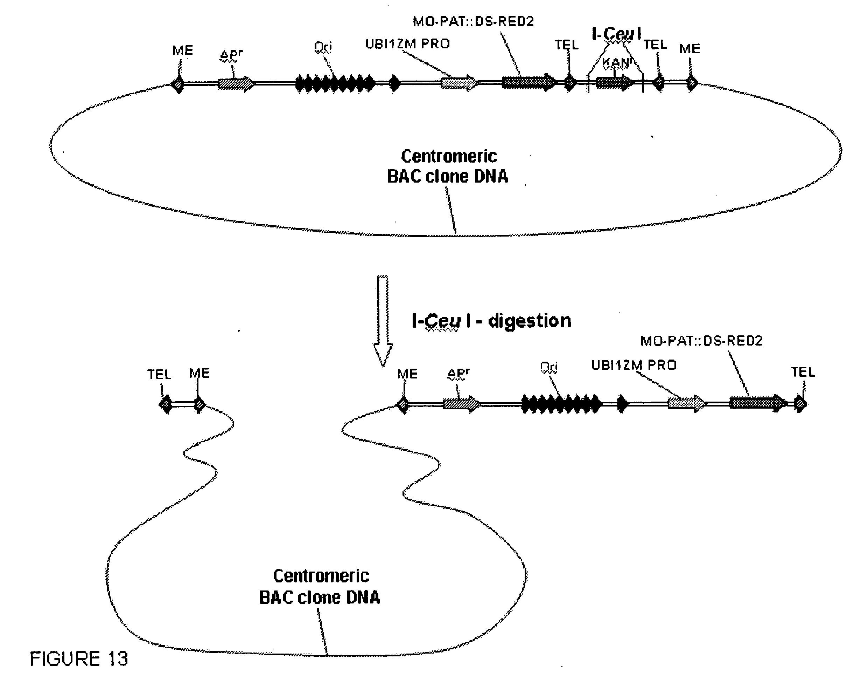

**Figure 13**. Retrofitting and conversion of a BAC clone into a linear artificial minichromosome *in vitro.* BAC clone DNA is retrofitted with custom-made transposon Tn5-3 comprising ampicillin resistance gene (AP^{r}), origin of replication (ori), selectable (MO-PAT) and visual (DS-RED2) markers under ubiquitin promoter (UBI1ZM PRO), telomeric sequences (TEL) in reverse orientation separated by a kanamycin resistance gen (KAN^{r}) gene, and sites for homing restriction enzymes I-Ppo I, I-*Ceu* I, and PI-*Sce* I. ME stands for transposon mosaic ends. Digestion of the BAC construct with homing restriction enzyme I-Ceu I converts a circular BAC into a linear DNA molecule flanked with telomeric sequences.

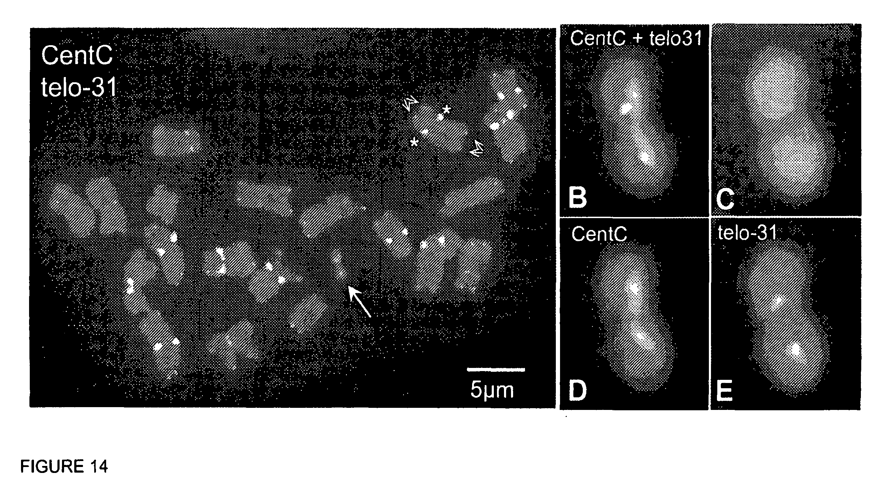

**Figure 14**. Metaphase nucleus of callus from CMC3 pool 1 event #14 probed for centromere and telomere elements. FISH analysis was done using fluorescently labeled probes for the centromere-specific CentC repeat (green - color; white - greyscale) and the telomere-specific telo-31 repeat (red - color; white - greyscale). Localization of these probes is noted for a native chromosome, CentC is denoted by asterisks (*), and telo-31 denoted by double arrows. Panels B-E show higher magnification of the minichromosome. Panel B - DAPI + Cent C + telo31 (green/red - color; white - greyscale); C - DAPI only; D - DAPI + CentC probe (green - color; white - greyscale); and E - DAPI + 23715 probe (red - color; white - greyscale). The pattern of telo-31 hybridization suggests that the minichromosome (arrow) has functional telomeres similar to the native chromosomes.

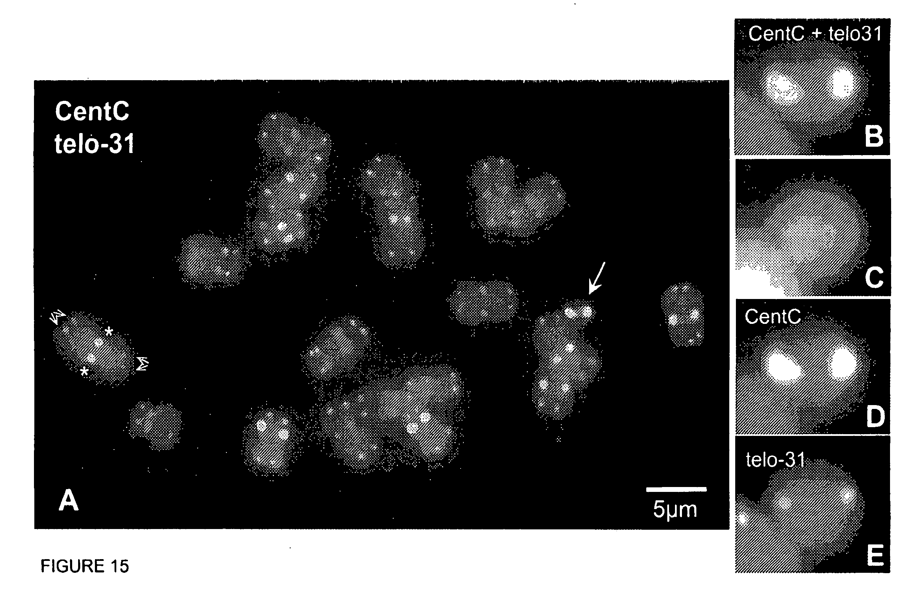

**Figure 15**. Metaphase nucleus of callus from CMC3 subpool 1.3 event #27 probed for centromere and telomere elements. FISH analysis was done using fluorescently labeled probes for the centromere-specific CentC repeat (green - color; white - greyscale) and the telomere-specific telo-31 repeat (red - color; white - greyscale). Localization of these probes is noted for a native chromosome, CentC is denoted by asterisks (*), and telo-31 denoted by double arrows. Panels B-E show higher magnification of the minichromosome. Panel B - DAPI + Cent C + telo-31 (green/red - color; white - greyscale); C⁻- DAPI only; D - DAPI + CentC probe (green - color; white - greyscale); and E - DAPI + 23715 probe (red - color; white -greyscale). The pattern of telo-31 hybridization suggests that the minichromosome (arrow) has functional telomeres similar to the native chromosomes.

### DETAILED DESCRIPTION OF THE INVENTION

As used herein and in the appended claims, the singular forms "a", "an", and "the" include plural reference unless the context clearly dictates otherwise. Thus, for example, reference to "a plant" includes a plurality of such plants, reference to "a cell" includes one or more cells and equivalents thereof known to those skilled in the art, and so forth.

In the context of this disclosure, a number of terms and abbreviations are used. The following definitions are provided.

"Open reading frame" is abbreviated ORF.

"American Type Culture Collection" is abbreviated ATCC.

The term "artificial plant minichromosome" as used herein refers to any artificially created chromosome comprising a centromere and telomeres that possesses properties comparable to those of a native chromosome, such as replication and segregation during mitosis and meiosis and therefore autonomous and transmissible in cell division. The terms artificial minichromosome, minichromosome, and artificial chromosome are used interchangeably herein.

The term "functional centromere" refers to the spindle attachment region of a eukaryotic chromosome that functions in a manner comparable to centromeres in a native chromosome. It is the most condensed and constricted region of a chromosome, to which the spindle fiber is attached during mitosis. During mitosis in a typical plant or animal cell, each chromosome divides longitudinally into two sister chromosomes that eventually separate and travel to opposite poles of the mitotic spindle. At the beginning of mitosis, when the sister chromosomes have split but are still paired, every chromosome attaches to the spindle at a specific point along its length. That point is referred to as the centromere or spindle attachment region. Centromeres are composed of highly repetitive DNA, that is, DNA sequences that are present in a genome in many copies.

The term "array" refers to an orderly arrangement of elements.

The term "tandem repeat" refers to multiple copies of the same base sequence in the same orientation. Thus, these are copies of sequences of nucleotides, which are repeated over and over again a number of times in tandem, for example, along a chromosome. Any array of tandem repeats may comprise multiple copies of a single element, or may have at least one other element interspersed within the array, or within an element of the array.

The term "inverted orientation" refers to two or more copies of the same sequence present in an inverted form.

The terms "retrotransposable element" and "retrotransposon" are used interchangeably herein and refer to a genetic element that transposes to a new location in DNA by first making an RNA copy of itself, then making a DNA copy of this RNA with a reverse transcriptase, and then inserting the DNA copy into the target DNA. Retrotransposons are genetic elements than can amplify themselves in a genome and are ubiquitous components of the DNA of many eukaryotic organisms. They are a subclass of transposon. They are particularly abundant in plants, where they are often a principal component of nuclear DNA.

The term "functional telomere" refers to structures found at the ends of chromosomes in the cells of eukaryotes. Telomeres function by protecting chromosome ends from recombination, fusion to other chromosomes, or degradation by nucleases. They permit cells to distinguish between random DNA breaks and chromosome ends. They also play a significant role in determining the number of times that a normal cell can divide. A telomere is a region of highly repetitive DNA at the end of a linear chromosome that functions as a disposable buffer. Every time linear eukaryotic chromosomes are replicated during late S-phase the DNA polymerase complex is incapable of replicating all the way to the end of the chromosome; if it were not for telomeres, this would quickly result in the loss of vital genetic information, which is needed to sustain a cell's activities.

As used herein, "nucleic acid" means a polynucleotide and includes single or double-stranded polymer of deoxyribonucleotide or ribonucleotide bases. Nucleic acids may also include fragments and modified nucleotides. Thus, the terms "polynucleotide", "nucleic acid sequence", "nucleotide sequence" or "nucleic acid fragment" are used interchangeably to denote a polymer of RNA or DNA that is single or double-stranded, optionally containing synthetic, non-natural or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric genes to produce the desired phenotype in a transformed plant. Chimeric genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential in the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

The terms "homology", "homologous", "substantially similar", "substantially identical", and "corresponding substantially" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. These terms also refer to amino acid sequences, polypeptides, or peptide fragments with or without modifications, deletions, insertions, or substitutions that do not substantially alter the functional properties relative to an initial unmodified sequence. It is therefore understood, as those skilled in the art will appreciate, that the invention encompasses more than the specific exemplary sequences.

Moreover, the skilled artisan recognizes that substantially similar nucleic acid sequences encompassed by this invention are also defined by their ability to hybridize (under moderately stringent conditions, e.g., 0.5X SSC, 0.1% SDS, 60 °C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30 °C for short probes (e.g., 10 to 50 nucleotides) and at least about 60 °C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37 °C, and a wash in 1X to 2X SSC (20X SSC = 3.0 M NaCl/0.3 M trisodium citrate) at 50 to 55 °C. Exemplary moderate stringency conditions include hybridization in 40 to 45% formamide, 1 M NaCl, 1% SDS at 37 °C, and a wash in 0.5X to 1X SSC at 55 to 60 °C. Exemplary high stringency conditions include hybridization in 50% formamide, 1 M NaCl, 1 % SDS at 37 °C, and a wash in 0.1X SSC at 60 to 65 °C.

Specificity is typically the function of post-hybridization washes, the critical factors being the ionic strength and temperature of the final wash solution. For DNA-DNA hybrids, the Tₘ can be approximated from the equation of Meinkoth et al. ((1984) Anal Biochem 138:267-284): Tₘ = 81.5 °C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1 % of mismatching; thus, Tₘ, hybridization and/or wash conditions can be adjusted to hybridize to sequences of the desired identity. For example, if sequences with ≥90% identity are sought, the Tₘ can be decreased 10 °C. Generally, stringent conditions are selected to be about 5 °C lower than the thermal melting point (Tₘ) for the specific sequence and its complement at a defined ionic strength and pH. However, severely stringent conditions can utilize a hybridization and/or wash at 1, 2, 3, or 4 °C lower than the thermal melting point (Tₘ); moderately stringent conditions can utilize a hybridization and/or wash at 6, 7, 8, 9, or 10 °C lower than the thermal melting point (Tₘ); low stringency conditions can utilize a hybridization and/or wash at 11, 12, 13, 14, 15, or 20 °C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology--Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel et al., Eds., Greene Publishing and Wiley-Interscience, New York (1995). Hybridization and/or wash conditions can be applied for at least 10, 30, 60, 90, 120, or 240 minutes.

The term "sequence identity" or "identity" in the context of nucleic acid or polypeptide sequences refers to the nucleic acid bases or amino acid residues in two sequences that are the same when aligned for maximum correspondence over a specified comparison window. The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90%, or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign^{™} program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized. The term "Clustal V method of alignment" corresponds to the alignment method labeled Clustal V (described by Higgins & Sharp (1989) CABIOS 5:151-153; Higgins et al. (1992) Comput Appl Biosci 8:189-191) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program. The term "Clustal W method of alignment" corresponds to the alignment method labeled Clustal W (described by Higgins & Sharp (1989) CABIOS 5:151-153; Higgins et al. (1992) Comput Appl Biosci 8:189-191) and found in the MegAlign^{™} v6.1 program of the LASERGENE bioinformatics computing suite (DNASTAR Inc., Madison, WI). Default parameters for multiple alignment are GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB. After alignment of the sequences using the Clustal W program, it is possible to obtain a "percent identity" by viewing the "sequence distances" table in the same program. The term "BLASTN method of alignment" is an algorithm provided by the National Center for Biotechnology Information (NCBI) to compare nucleotide sequences using default parameters.

It is well understood by one skilled in the art that many levels of sequence identity are useful in identifying polypeptides, from other species, wherein such polypeptides have the same or similar function or activity. Useful examples of percent identities include, but are not limited to, 50%, 55%, 60%, 65%, 70%, 75%, 80%, 85%, 90%, or 95%, or any integer percentage from 50% to 100%. Indeed, any integer amino acid identity from 50% to 100% may be useful in describing the present invention, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

The term "gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

The term "genome" as it applies to a plant cells encompasses not only chromosomal DNA found within the nucleus, but organelle DNA found within subcellular components (*e.g*., mitochondria, or plastid) of the cell.

A "codon-optimized gene" or "codon-preferred gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same that plant is homozygous at that locus. If the alleles present at a given locus on a chromosome differ that plant is heterozygous at that locus.

The term "coding sequence" refers to a polynucleotide sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, introns, polyadenylation recognition sequences, RNA processing sites, effector binding sites and stem-loop structures.

The term "promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". New promoters of various types useful in plant cells are constantly being discovered; numerous examples may be found in the compilation by Okamuro & Goldberg (1989) Biochemistry of Plants 15:1-82.

The term "translation leader sequence" refers to a polynucleotide sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner & Foster (1995) Mol Biotechnol 3:225-236).

The terms "3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al. (1989) Plant Cell 1:671-680.

The term "RNA transcript" refers to the product resulting from RNA polymerase-catalyzed transcription of a DNA sequence. When the RNA transcript is a perfect complementary copy of the DNA sequence, it is referred to as the primary transcript. A RNA transcript is referred to as the mature RNA when it is a RNA sequence derived from post-transcriptional processing of the primary transcript. "Messenger RNA" or "mRNA" refers to the RNA that is without introns and that can be translated into protein by the cell. "cDNA" refers to a DNA that is complementary to, and synthesized from, a mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (U.S. Patent 5,107,065). The complementarity of an antisense RNA may be with any part of the specific gene transcript, *i.e*., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (*i.e*., the coding sequence is under the transcriptional control of the promoter). Coding sequences can be operably linked to regulatory sequences in a sense or antisense orientation. In another example, the complementary RNA regions of the invention can be operably linked, either directly or indirectly, 5' to the target mRNA. or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al. Molecular Cloning: A Laboratory Manual; Cold Spring Harbor Laboratory: Cold Spring Harbor, NY (1989). Transformation methods are well known to those skilled in the art and are described *infra*.

"PCR" or "polymerase chain reaction" is a technique for the synthesis of specific DNA segments and consists of a series of repetitive denaturation, annealing, and extension cycles. Typically, a double-stranded DNA is heat denatured, the two primers complementary to the 3' boundaries of the target segment are annealed at low temperature, and then extended at an intermediate temperature. One set of these three consecutive steps is referred to as a "cycle".

The term "recombinant" refers to an artificial combination of two otherwise separated segments of sequence, *e.g*., by chemical synthesis or by the manipulation of isolated segments of nucleic acids by genetic engineering techniques.

The terms "plasmid", "vector" and "cassette" refer to an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell. "Transformation cassette" refers to a specific vector containing a foreign gene and having elements in addition to the foreign gene that facilitate transformation of a particular host cell. "Expression cassette" refers to a specific vector containing a foreign gene and having elements in addition to the foreign gene that allow for expression of that gene in a foreign host.

The terms "recombinant construct", "expression construct", "chimeric construct", "construct", and "recombinant DNA construct" are used interchangeably herein. A recombinant construct comprises an artificial combination of nucleic acid fragments, e.g., regulatory and coding sequences that are not found together in nature. For example, a chimeric construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the invention. The skilled artisan will also recognize that different independent transformation events may result in different levels and patterns of expression (Jones et al. (1985) EMBO J 4:2411-2418; De Almeida et al. (1989) Mol Gen Genet 218:78-86), and thus that multiple events must be screened in order to obtain lines displaying the desired expression level and pattern. Such screening may be accomplished by Southern analysis of DNA, Northern analysis of mRNA expression, immunoblotting analysis of protein expression, or phenotypic analysis, among others.

The term "expression", as used herein, refers to the production of a functional end-product (*e.g*., an mRNA or a protein, in either precursor or mature form).

The term "introduced" means providing a nucleic acid (*e.g*., expression construct) or protein into a cell. Introduced includes reference to the incorporation of a nucleic acid into a eukaryotic or prokaryotic cell where the nucleic acid may be incorporated into the genome of the cell, and includes reference to the transient provision of a nucleic acid or protein to the cell. Introduced includes reference to stable or transient transformation methods, as well as sexually crossing. Thus, "introduced" in the context of inserting a nucleic acid fragment (*e.g*., a recombinant DNA construct/expression construct) into a cell, means "transfection" or "transformation" or "transduction" and includes reference to the incorporation of a nucleic acid fragment into a eukaryotic or prokaryotic cell where the nucleic acid fragment may be incorporated into the genome of the cell (*e.g*., chromosome, plasmid, plastid or mitochondrial DNA), converted into an autonomous replicon, or transiently expressed (*e.g*., transfected mRNA).

The term "mature" protein refers to a post-translationally processed polypeptide (*i.e*., one from which any pre- or propeptides present in the primary translation product have been removed). "Precursor" protein refers to the primary product of translation of mRNA (*i.e*., with pre- and propeptides still present). Pre- and propeptides may be, but are not limited to, intracellular localization signals.

The term "stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms.

The term "transgenic" refers to a plant or a cell, which comprises within its genome a heterologous polynucleotide. Preferably, the heterologous polynucleotide is stably integrated within the genome such that the polynucleotide is passed on to successive generations. The heterologous polynucleotide may be integrated into the genome alone or as part of an expression construct. Transgenic is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

The term "plant" refers to whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to the following: roots, stems, shoots, leaves, pollen, seeds, tumor tissue and various forms of cells and culture (e.g., single cells, protoplasts, embryos and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the following: (1) the entire complement of genetic material (genes and non-coding sequences) that is present in each cell of an organism, or virus or organelle; and/or (2) a complete set of chromosomes inherited as a (haploid) unit from one parent. "Progeny" comprises any subsequent generation of a plant.

The instant invention concerns an artificial plant minichromosome comprising a functional centromere containing: (a) at least two arrays of tandem repeats of CentC in an inverted orientation wherein the first array comprises at least fifty copies of CentC and the second array comprises at least fifty copies of CentC; and, (b) at least one copy of a retrotransposable element, wherein the retrotransposable element is situated between the first and the second array. Preferably, the retrotransposable element is selected from the group consisting of CentA, CRM1, and CRM2.

The artificial chromosome comprises a functional centromere having arrays of tandem repeats of CentC. Each array of CentC repeats may comprise at least 30, 40, 50, 60, 70, 80, 90, 100, 120, 140, 150, 160, 180, 200, 220, 240, 250, 260, 280, 300, 320, 340, 360, 380, 400, 450, or 500 copies of CentC. Further, each array of tandem repeats of CentC may be interrupted by another sequence element, including but not limited to a retrotransposon, which is inserted between copies of CentC, or within a CentC element, or within a retrotransposon, or any other sequence element in the array. Retrotransposons include, but are not limited to, CentA, CRM1, CRM2.

In most eukaryotes the centromere, which is the site for kinetochore formation and spindle attachment in chromosomes, is embedded in heterochromatin. *S. cerevisiae* chromosomes lack satellite sequences and have small, precisely localized centromeres which specify spindle attachment with ∼125 bp of DNA (Blackburn & Szostak (1984) Ann Rev Biochem 53:163-194).

However, centromeres from other fungal lineages include arrays of repeats more similar to those found animals and plants (Fishel et al. (1988) Mol Cell Biol 8:754-763). In higher eukaryotes, cytological and biochemical studies demonstrated a physical association between tandemly repeated satellite DNAs, centromere regions, and specific centromere associated proteins (Henikoff et al. (2001) Science 293:1098-1102; Yu & Dawe (2000) J Cell Biol 151:131-142).

Despite the lack of universal sequence motifs, most centromeric satellite repeats have remarkably similar unit length between organisms, for example the basic satellite unit is 171 bp in primates, 186 bp in the fish *Sparus aurata,* and 155 bp in the insect *Chironomus*. *pallidivittatus* (Henikoff et al. (2001) Science 293:1098-1102).

Plant centromeres possess similar unit length repeats, for example, 156 bp repeat in maize (Ananiev et al. (1998) Proc Natl Acad Sci USA 95:13073-13078), 168 bp repeat in rice (Dong et al. (1998) Proc Natl Acad Sci USA 95:8135-8140), and 180 bp repeat in *Arabidopsis* (Copenhaver (2003) Chromosome Res 11:255-262). In *Arabidopsis,* centromeres typically contain 2.8-4 Mb tracts of tandemly repeated 178 bp satellite sequences (Hall et al. (2004) Curr Opin Plant Biol 7:108-114). In maize, a fully functional supernumerary B chromosome centromere contains about 500 kb of tandem repeats, wherein partial deletions reduce transmission (Alfenito & Birchler (1993) Genetics 135:589-597). Maize chromosome spreads containing the supernumerary B chromosome were hybridized with probes from various repetitive elements including CentC, CRM, and CentA, which localized to centromeric regions on the A chromosomes. These repetitive elements, predominantly found near A chromosome centromeres, hybridized to many sites distinct from the centromere on the B chromosome (Lamb et al. (2005) Chromosoma 113:337-349).

At least two examples deviate from the general rule of centromere formation on the basis of centromeric satellite DNA. First, the apparently normal functioning of alien centromeres in somatic hybrids or in oat-maize introgression lines (Ananiev et al. (1998) Proc Natl Acad Sci USA 95:13073-13078) is indicative of conservation of centromere function and corresponding protein complexes. All centromeric proteins to support the function of an alien centromere comprising unrelated centromeric satellite DNA are apparently provided by the host (Jin et al. (2004) Plant Cell 16:571-81). Second, neocentromeres are a novel class of non-repeat DNA-based centromeres recently described in humans and *Drosophila* (Williams (1998) Nat Genet 18:30-37; Choo (1997) Am J Hum Genet 61:1225-1233). Found as derivatives of normal chromosomes resulting from multiple chromosomal rearrangements, neocentromeres are formed at apparently euchromatic DNA regions devoid of the repeats typically associated with centromere function. Chromosomes with neocentromeres have variable mitotic or meiotic stability.

The nature and functioning of the centromere is not yet completely understood and requires additional analysis. To date, most artificial chromosomes have functional centromeres based on native centromeric satellite DNAs. It is possible that knob repeats, such as 180 bp and 350 bp (TRI), may be used as a component of a neocentromere. It was shown that some knobs could acquire centromere function in meiotic maize chromosomes, these neocentromeres comprised 180 bp and 350 bp tandem repeats only. The study of neocentromeres in humans and lower organisms, has unraveled a previously unsuspected phenomenon depicting the dynamic nature of centromeric DNA (Choo et al. (1997) Am J Hum Genet. 61:1225-33). At the core of this phenomenon, there appears to be no specific DNA sequence requirement for centromere function; rather, a variety of sequences that can respond to the appropriate epigenetic influence appear to provide this function.

Extensive characterizations of centromere sequences have come from studies in yeast, for example *S*. *cerevisaie* and *S*. *pombe,* and have defined functional yeast centromere elements and organization. For example, in *S*. *cerevisaie* centromeres the structure and function of three essential regions, CDEI, CDEII, and CDEIII, totaling only 125 bp, or 0.006 - 0.06% of each chromosome were described (Carbon et al. (1990) New Biologist 2:10-19; Bloom (1993) Cell 73:621-624).

*S. pombe* centromeres are between 40-100 kb and consist of repetitive elements that comprise 1-3% of each chromosome (Baum et al. (1994) Mol Cell Biol 5:747-761). Subsequent studies demonstrated that less than 1/3 of the native *S*. *pombe* centromere is sufficient for centromere function (Baum et al. (1994) Mol Cell Biol 5:747-761). In *S*. *pombe,* it was shown that an inverted repeat region was essential for centromeric function, but neither the central core nor one arm of the inverted repeat alone conferred function. Deletion of a portion of the repeated sequences that flank the central core had no effect on mitotic segregation functions, or on meiotic segregation of a minichromosome to haploid progeny, but drastically impaired centromere-mediated maintenance of sister chromatid pairing of homologues in meiosis I. There is significant variability between each of the three different chromosomes in *S. pombe*, and the centromere of any particular chromosome can contain significant variability across different strains of *S*. *pombe.* However, the basic DNA structural motif, namely, the inverted repeats, is a common parameter of the *S*. *pombe* centromere (Clarke et al. (1993) Cold Spring Harb Symp Quant Biol 58:687-695).

Centromeres from higher eukaryotes are less characterized. DNA fragments that hybridize to centromeric regions in higher eukaryotes have been identified, however generally little is known about the structure, organization, and/or functionality of these sequences. However, rice is an exception because of its different centromere size. Though some rice chromosomes have a centromere similar in size to those in other species (>1 Mb), the centromeres of several chromosomes are surprisingly small and can be fully covered by BAC contigs constructed using standard techniques. Complete sequencing of rice centromere 4 and 8 revealed the presence of inverted blocks of centromeric tandem repeats within the chromosomal segment considered the centromere, similar to the inverted repeat structure observed in yeast (Zhang et al. ( 2004) Nucl Acids Res 32:2023-2030; Wu et al. (2004) Plant Cell 16:967-976).

In many cases probes to centromere repeats correlate with centromere location both cytologically and genetically, with many of these sequences present as tandemly-repeated satellite elements and dispersed repeated sequences in arrays ranging from 300 - 5000 kb in length (Willard (1990) Trends Genet 6:410-416). *In situ* hybridization has shown the alphoid satellite 171 bp repeat to be present in each human centromere (Tyler-Smith et al. (1993) Curr Biol 390-397). Whether these repeats constitute functional centromeres is not yet determined, and it appears other genomic DNA is needed to confer heritability to the DNA. Transfection of cell lines with alphoid satellites produced new chromosomes, however these new chromosomes also contain host DNA, which could contribute to centromere activity (Haaf et al. (1992) Cell 70:681-696; Willard (1997) Nat Genet 15:345-354). Further, the new chromosomes can show alphoid DNA spread over their entire length yet have only one centromeric constriction, indicating that a block of alphoid DNA may be insufficient to confer centromere function.

Genetic characterization of centromeres from plants has used segregation analysis of chromosome fragments, including analysis of trisomic strains carrying a genetically marked telocentric fragment (e.g., Koornneef (1983) Genetica 62:33-40). Plant centromere repetitive elements which are genetically (Richards *et al.* (1991)) or physically (Alfenito et al. (1993) Genetics 135:589-597; Maluszynska et al. (1991) Plant J 1:159-166) linked to a centromere have been identified, however the importance of these sequences regarding centromere function has not been fully functionally characterized.

Cytological studies in *Arabidopsis thaliana* have correlated centromere structure with repeat sequences. Staining with a non-specific fluorescent DNA-binding agent, such as 4',6-diamidino-2-phenylindole (DAPI), allows visualization of centromeric chromatin domains in metaphase chromosomes. A fluorescent *in situ* hybridization (FISH) probe to 180 bp pALI repeat sequences colocalized with the DAPI signature near the centromeres of all five *Arabidopsis* chromosomes (Maluszynska et al. (1991) Plant J 1:159-166; Martinez-Zapater et al. (1986) Mol Gen Genet 204:417-423). A functional role for pALI was proposed, however more recent studies have not detected this sequence near the centromeres of species closely related to *Arabidopsis* (Maluszynska et al. (1993) Ann Botany 71:479-484). One species tested, *A. pumila* is believed to be an amphidiploid derived from a cross of *A*. *thaliana* with another close relative (Maluszynska et al. (1991) Plant J 1:159-166; Price et al. (1995) in Arabidopsis, Somerville & Meyerowitz (eds) Cold Spring Harbor Press, NY). Another repetitive sequence, pAt12, genetically maps to within 5cM of the centromere of chromosome 1, and the central region of chromosome 5 (Richards et al. (1991) Nucl Acids Res 19:3351-3357), but its role in centromere function remains to be established.

Plant centromere regions are composed predominantly of centromere-specific repeats, centromeric retrotransposons, and a few other repetitive elements which are mostly scattered along the plant genome. For example centromeric repeats such as CentO and CRR are known from rice. Four centromere repetitive elements have been described in maize: CentA, CentC, CRM1, and CRM2 (SEQ ID NOS: 1-4). In maize, the first tandem repeated centromere-specific element discovered was CentC (Ananiev et al. (1998) Proc Natl Acad Sci USA 95:13073-13078). CentC forms multiple tandem arrays of varying length, with some tandem arrays comprising up to one thousand copies of the CentC repeat. The CentC tandem repeat interacts with CENH3 protein in the centromeric nucleosome.

Maize centromere-specific element, CentA, appears to be a retrotransposon based on its structure and properties (Ananiev et al. (1998) Proc Natl Acad Sci USA 95:13073-13078; GenBank AF078917). Another highly conservative centromere-specific retrotransposon of maize, CRM2, was found in 2003 (Nagaki et al. (2003) Genetics 163:759-770; GenBank AY129008). A fourth centromere-specific retrotransposon, CRM1 (SEQ ID NO: 3), was identified by comparative analysis of published DNA sequences of two maize centromeric BAC clones (Nagaki et al. (2003) Genetics 163:759-770) and proprietary maize genomic DNA sequences (Ananiev (2005) unpublished). Some homology can be detected among the centromeric repeat elements from closely related species, such as sorghum and sugarcane (Miller et al. (1998) Genetics 150:1615-1623; Nagaki et al. (1998) Chromosome Res 6:295-302; Zwick et al. (2000) Am J Bot 87:1757-1764); and maize and rice (Ananiev et al. (1998) Proc Natl Acad Sci USA 95:13073-13078); Cheng et al. (2002) Plant Cell 14:1691-1704).

In addition, plant centromeres contain abundant retrotransposons (CR), in cereals many of the CR elements fall within a highly conserved phylogenetic clade of Ty3/gypsy elements (Miller et al. (1998) Theor Appl Genet 96:832-839; Presting et al. (1998) Plant J 16: 721-728; Langdon et al. (2000) Genetics 156:313-325). The DNA homology is sufficient that CR probes from sorghum or *Brachypodium sylvaticum* identify the centromeres in most or all of the chromosomes in agronomically significant cereals such as rice, maize, wheat, sorghum, barley, and rye (Aragon-Alcaide et al. (1996) Chromosoma 105:261-268; Jiang et al. (1996) Proc Natl Acad Sci USA 93:14210-14213; Miller et al. (1998) Theor Appl Genet 96:832-839).

Retrotransposons, also known as class I transposable elements, consist of two subtypes, the long terminal repeat (LTR) and the non-LTR retrotransposons. The long terminal repeat subtypes have direct LTRs that range from ∼100 bp to over 5 kb in size. LTR retrotransposons are further classified into the Ty1-copia-like (*Pseudoviridae*) and the Ty3-gypsy-like (*Metaviridae*) groups based on both their degree of sequence similarity and the order of encoded gene products. Ty1-copia and Ty3-gypsy groups of retrotransposons are commonly found in high copy number (up to a few million copies per haploid nucleus) in plants with large genomes. Ty1-copia retrotransposons are abundant in species ranging from single-cell algae to bryophytes, gymnosperms, and angiosperms. Ty3-gypsy retrotransposons are also widely distributed, including both gymnosperms and angiosperms. LTR retrotransposons make up approximately 8% of the human genome. Non-LTR retrotransposons consist of two subtypes, long interspersed nuclear elements (LINEs) and short interspersed nuclear elements (SINEs). They also can be found in high copy numbers (up to 250,000) in plant species. Plant transposons, including retrotransposons, are reviewed by Feschotte et al. (2002) Nat Rev Genet 3:329-341. Plant retrotransposons are reviewed by Kumar & Bennetzen (1999) Ann Rev Genet 33:479-532.

Centromeric retrotransposons are identifiable based on unified classification of reverse-transcribing elements used for phylogeny and taxonomy studies. Complete retroelements and retroviruses include two or more open reading frames (ORFs) that encode single proteins or polyproteins. The order of the genes in the elements varies, but are classified on the basis of amino acid alignments and key conserved residues or domains within the reverse transcriptase (RT), RNase H 15 (RH), integrase (INT) and aspartic protease (PR) genes and in a conserved cysteine-histidine (CH) zinc-finger-like domain. The retroelements also comprise long-terminal repeat (LTR) sequences that flank the internal region of the retroelement. Every family of retrotransposons has different, non-cross-hybridizing LTRs, and components within a family can vary (0-50%) in their LTR sequences. In the transposition process, the two LTRs are usually identical at the time of insertion, but as time passes substitutions can cause sequence divergence. Many retroelements are known, including centromere-specific retrotransposons (see, for example, SanMiguel et al. (1998) Nat Genet 20:43-45; Turcotte et al. (2001) Plant J 25:169-179; Feng et al. (2002) Nature 420:316; Nagaki et al. (2004) Nat Genet 36:138; Nagaki et al. (2003) Genetics 163:750-770: Wu et al. (2004) Plant Cell 16:967-976; Hansen & Haslop-Harrison (2004) Adv Bot Res 41:165-193).

There exists significant variation between centromeres of different maize chromosomes with respect to their relative size and the repeat composition. In maize CentC clusters can be as small as about 100 kb, or more than about 2000 kb in different chromosomes, but commonly in the range of about 200 kb to about 300 kb. Given the lower size range, it is possible that an entire central portion of maize centromere region could be found within a single BAC clone. The observed structural polymorphism suggests that a maize centromere is composed of redundant functional blocks, each of which may be capable of supporting centromere function. A significant (at least 10 fold) variation in centromere sizes as defined by the length and/or copy number of the CentC centromeric tandem repeats is observed among different maize chromosomes. There is also a significant variation in centromere size between homologous chromosomes from different inbreds.

Telomeres are nucleoprotein caps at the ends of linear eukaryotic chromosomes essential for chromosomal end maintenance. Telomere DNA synthesis is done by telomerase, a ribonucleoprotein with reverse transcriptase activity (McKnight et al. (2002) Plant Mol Biol 48:331-337). Telomerase adds telomeric DNA onto the 3' ends of chromosomes by copying a short template sequence within its RNA subunit. The telomeres of most organisms consist of highly conserved short asymmetric repeated sequences.

Many telomeric repeat sequences are known, including CCCCAA (C₄A₂, *Tetrahymena* & *Paramecium*); C₄A₄ (*Oxytricha & Euplotes*); C₃TA (*Trypanosoma*, *Leishmania, & Physarum);* C₁₋₃A (*Saccharomyces*); C₁₋₈T (*Dictyostelium*); and C₃TA₃ (*Arabidopsis*, human, mouse, *Caenrhabditis*). The number of repeats observed in native chromosomes varies widely between organisms, *e.g*. some ciliates have about 50 repeats, less than 350 repeats has been observed in *Arabidopsis,* and repeats totaling about 300-500 bp observed in *Saccharomyces*.

Telomere length in plants, which typically ranges from about 2-75 kb, is controlled by genetic and developmental factors. Telomeric regions have been isolated from *Arabidopsis,* and show tandem repeats heterogeneous in size (Richards & Ausubel (1988) Cell 53:127-136). A 25-fold difference in the lengths of telomeres among inbred lines of maize was found, ranging from less than 2 kb for the WF9 line to about 40 kb for the CM37 line (Burr et al. (1992) Plant Cell 4:953-960). Closer toward the centromere, the canonical telomere repeat is often found mixed with other repetitive elements of the plant genome. In contrast, *Drosophila* uses transposons at the ends its chromosomes. The transposons, HeT-A and TART elements, are found in multiple copies at the end of each chromosome. Gradual shortening of the telomeres can be reversed by transposition of new transposon repeats to the ends. Similar to telomere maintenance by telomerase, the model for transposition in *Drosophila* invokes a mechanism using an RNA transposition intermediate which is converted into end DNA by reverse transcriptase.

DNA replication is the process by which cells make one complete copy of their genetic information before cell division. In *E. coli,* mammalian viruses, and *S*. *cerevisiae*, initiation of DNA replication is controlled by transacting initiator proteins that interact with cis-acting DNA replicator sequences. For *S*. *cerevisiae*, replicators encompass 100-200 bp and include the major replication origin sites where DNA synthesis begins. These replicators contain a conserved 11 bp autonomous replicating sequence (ARS) that binds the origin recognition complex (ORC) to nucleate formation of prereplication complexes (Gilbert (2001) Science 294:96-100).

In higher eukaryotes DNA replication can be initiated simultaneously in hundreds or thousands of chromosomal sites. Defined origin sequences are not required, many potential replication origins exist consisting of broad zones of closely spaced initiation sites, some of which may be used more frequently.

However, several specific eukaryotic origins of replication are known such as the origin of replication for 18S-26S rDNA which is located in a non-transcribed spacer (Ivessa & Zakian (2002) Genes Dev 16:2459-2464). This region is capable of promoting amplification of transgenic constructs (Hemann et al. (1994) DNA Cell Biol 13:437-445). Another specific origin is found in the downstream region of the dihydrofolate reductase (DHFR) gene in Chinese hamster ovary (CHO) cells (Altman & Fanning (2001) Mol Cell Biol 21:1098-1110). Preferential sites of replication initiation were also found in the *Drosophila* chromosome segment containing chorion genes (Levine & Spradling (1985) Chromosoma 92:136-142).

The replication machinery of plant and animal cells is likely capable of replicating any type of introgressed DNA, including integrated constructs, episomes, entire chromosomes, or their fragments (Gilbert (2001) Science 294:96-100).

Artificial minichromosomes are linear or circular DNA molecules constructed from cis-acting DNA sequence elements responsible for proper replication and partitioning of chromosomes to daughter cells. The cis-acting elements include: origins of replication (ori), the sites for initiation of DNA replication, also known as autonomous replication sequences (ARS); centromeres, the sites of kinetochore assembly for proper segregation of replicated chromosomes at mitosis and meiosis; and telomeres, specialized DNA repeat structures that stabilize the ends of linear chromosomes and facilitate complete replication of the chromosome ends.

Several strategies to produce eukaryotic minichromosomes are available, including but not limited to *in vivo* self-assembly of a minichromosome from component elements by the endogenous cellular chromosome maintenance machinery in the eukaryotic cell, assembly of a eukaryotic minichromosome from component elements in a prokaryotic cell, and *in vitro* assembly of a eukaryotic minichromosome from component elements.

Artificial minichromosomes were first constructed in *Saccharomyces cerevisiae* (Murray et al. (1986) Mol Cell Biol 6:3166-3172; Blackburn & Szostak (1984) Ann Rev Biochem 53:163-194). A circular plasmid comprising the yeast 125 bp centromere, an origin of replication, a selectable marker, and a palindromic arrangement of two stretches of telomeric DNA was assembled by conventional recombinant DNA techniques and introduced into yeast by spheroplast transformation where it resolved into a simple linear molecule. Linear constructs 50 kb in length containing a centromere, an origin of replication, and two telomeres replicated and segregated at mitosis with ∼99% accuracy, and retained in dividing cultures for at least 20 generations. The generation of YACs indicated the potential to assemble artificial chromosomes in other eukaryotes such as plants and animals. Experiments on YACs indicated that three cis-acting DNA sequences are needed to build an artificial chromosome: telomeres; origin(s) of replication; and a centromere.

Animal artificial chromosomes have been generated by two different approaches: generating *de novo* chromosomes from cloned DNA segments; or by fragmenting and rearranging a natural chromosome (Brown et al. (2000) Trends Biotechnol 18:402-403; Cooke (2001) Cloning Stem Cells 3:243-249; Lipps et al. (20030 Gene 304:23-33). The *de novo* approach, referred to as the assembly or bottom-up approach, generates artificial chromosomes by combining essential cloned components. Co-transfection of a mixture of human alphoid DNA, telomeres, human genomic DNA, and a selectable marker into HT1080 cells resulted in formation of minichromosomes (Harrington et al. (1997) Nat Genet 15:345-355).

Characterization of the minichromosomes revealed that they all had complex cytogenetic structures, and were stably maintained in the absence of any selection. It was concluded that the minichromosomes and their centromere(s) were formed *de novo* from input DNA via complex rearrangements. Subsequently, other groups also used HT1080 cells to introduce linear or circular DNA constructs containing human alphoid DNA and telomeres cloned in YACs, PACs, or BACs (Compton et al. (1999) Nucleic Acids Res 27:1762-1765; Grimes et al. (2001) EMBO Rep 2:910-914). Minichromosomes were observed with different frequencies and showed different mitotic stability. All minichromosomes produced were significantly bigger than the original constructs, varying from 5 to 10 Mb. Therefore, a fully functioning mammalian chromosome could be generated starting with cloned DNA serving as a backbone for *de novo* assembly.

Fragmentation and rearrangement of natural chromosomes retaining centromere and telomeric regions is another strategy for minichromosome production. Small chromosome fragments can be isolated by pulse field gel electrophoresis, retrofitted with desirable genes, and reintroduced into the host cell. Fragmented minichromosomes were observed in cancer cells, and other cell types after irradiation, however the fragments were too big for isolation and there was no way to control the gene composition.

One approach to control reduction of chromosome size was based on telomere associated chromosome fragmentation (TACF) or telomere directed truncation (TDT) (Heller et al. (1996) Proc Natl Acad Sci USA 93:7125-7130; Shen et al. (1997) Hum Mol Genet 6:1375-1382). It involves successive fragmentation of specific human host chromosomes into smaller minichromosomes using a targeting vector encompassing a terminal telomere segment, a selectable marker, and sometimes a region of homology to the target chromosome. The resulting 'engineered minichromosomes' remain autonomous and segregate normally. Minichromosomes as small as 0.5 Mb have been generated containing alphoid DNA as the functional centromere sequence in human, hamster-human somatic cell hybrid lines, or chicken cells.

Recently, human artificial chromosomes were used to create transchromosomic- cloned calves producing human immunoglobulin. A human minichromosome (HAC) vector constructed by Cre/loxP mediated chromosome translocations and telomere-directed chromosome truncations in homologous recombination-proficient chicken DT40 cells was introduced into bovine primary fetal fibroblasts by microcell-mediated chromosome transfer (MMCT). Isolated nuclei from fetal fibroblasts with HAC were transferred into enucleated mature oocytes to produce cloned calves (Kuroiwa et al. (2002) Nat Biotechnol 20:889-894). An *in vivo* approach for generation of artificial chromosomes has been developed, based on the induction of intrinsic, large-scale amplification mechanisms of mammalian cells. Targeted integration of Centromeric satellite DNA and the non-transcribed spacer of the rDNA on a specific chromosome resulted in large-scale amplification of centromeric regions. These amplified chromosomes become unstable and undergo significant rearrangements producing stable minichromosomes preferentially composed of satellite DNA (Kereso et al. (1996) Chromosome Res 4:226-239; Hadlaczky (2001) Curr Opin Mol Ther 3:125-132).

An artificial chromosome containing multiple sequence-specific recombination acceptor sites was developed (ACE platform). Sequences of interest are provided in a targeting vector, and lambda integrase enzyme used to catalyze recombination between the ACE platform and targeting vector

Similar processes have been observed in plants. Spontaneous fragmentation of native chromosomes in plants has been observed. Minichromosomes were discovered in *Arabidopsis* (Murata et al. (2006) Chromosoma, published online April 11, 2006), and maize (Brock & Pryor (1996) Chromosome 104:575-584; Kato et al. (2005) Cytogenet Genome Res 109:156-165). In some instances, minichromosomes were induced by ionizing radiation (Riera-Lizarazu et al. (2000) Genetics 156:327-339).

A physical map of rice centromere 5 has been constructed, and could be used to create a rice artificial chromosome (Nonomura & Kurata (2001) Chromosoma 110:284-291). A similar approach was proposed for the construction of an artificial chromosome for beet, *Beta procumbens* (Gindullis et al. (2001) Genome 44:846-855). Transgenic construct concatemerization, ligations, and rearrangements can be found in plant transformation events. General plant transformation with standard constructs can produce complex rearrangements, concatamerization, and construct amplification (Svitashev & Somers (2001) Genome 44:691-897; Svitashev et al. (2002) Plant J 32:443-445). Co-transformation of plants with multiple plasmids can produce transgenic loci containing combinations of the different transgenes (Wu et al. (2002) Transgenic Res 11:533-541). Similar to studies in animal cells, *de novo* assembly of artificial minichromosomes via spontaneous concatemerization and ligation of components can occur in plant cells (see Figures 1-10, and 14-15).

Kinetochores link the centromeric DNA to the spindle fiber apparatus. Human autoantibodies that bind specifically near centromeres facilitated cloning of centromere-associated proteins (CENPs, Rattner (1991) Bioassays 13:51-56). At least one of these proteins belongs to the kinesin superfamily of microtubule motors (Yen (1991) EMBO J 10:1245-1254). Yeast centromere-binding proteins have been identified through genetic and biochemical studies (Bloom (1993) Cell 73:621-624; Lechner et al. (1991) Cell 64:717-725). CENH3 is a highly conserved protein that replaces histone H3 in centromeres, is thought to recruit other proteins required for chromosome movement. CENH3 is present throughout the cell cycle and colocalizes with the kinetochore Centromeric protein C (CENPC) in meiotic cells.

Antibodies specific to centromere-associated proteins can be used to confirm centromere assembly in a DNA construct and/or minichromosome. Immunolocalization of a CENP, such as CENH3 and/or CENPC, to the centromere of a minichromosome indicates formation of a functional centromere comprised of centromeric DNA elements and the associated binding proteins. Antiserum to maize centromeric histone H3 (CENH3, 17kD) was made and tested on native maize chromosomes (Zhong et al. (2002) Plant Cell 14:2825-2836). Chromatin immunoprecipitation demonstrated that CentC and CRM2 interact specifically with CENH3. Approximately 38 and 33% of CentC and CRM2 were precipitated in the chromatin immunoprecipitation assay, confirming that much of CENH3 colocalizes with CentC. A maize homologue of mammalian CENPC was isolated by Dawe et al. ((1999) Plant Cell 11:1227-1238) and shown to be a component of the kinetochore in maize. A 20 amino acid conserved peptide from the amino terminal domain was used to produce antisera specific to maize CENPC, which was directly labeled and used to demonstrate that CENPC is specifically localized to the centromere of native and artificial minichromosomes in corn (see, *e.g*., Figures 3, 4, 8, and 10)

The centromeric repeat elements CentA, CentC, CRM1, and CRM2 include sequences that are substantially identical to the maize sequences for CentA, CentC, CRM1, and CRM2 of SEQ ID NOs: 1-4. Substantially identical sequences include sequences that have a high homology to each other as exemplified by having significant percent sequence identity, and/or by selectively hybridizing under stringent conditions to a CentA, a CentC, a CRM1, or a CRM2 (SEQ ID NOs: 1-4), or a complement thereof. Sequences that selectively hybridize under stringent hybridization conditions include sequences that hybridize to the target sequence at least 2-fold over background and to the substantial exclusion of non-target nucleic acids. Selectively hybridizing sequences typically have about at least 80, 85, 90, 95, 96, 97, 98, 99, or 100% sequence identity to the target sequence. Any suitable hybridization conditions and buffers known in the art can be used, examples of which have been described herein. Sequence identity may be used to compare the primary structure of two polynucleotides or polypeptide sequences. Sequence identity measures the residues in the two sequences that are the same when aligned for maximum correspondence. Sequence relationships can be analyzed using computer-implemented algorithms. The sequence relationship between two or more polynucleotides, or two or more polypeptides can be determined by determining the best alignment of the sequences, and scoring the matches and the gaps in the alignment, which yields the percent sequence identity and the percent sequence similarity. Polynucleotide relationships can also be described based on a comparison of the polypeptides each encodes. Many programs and algorithms for comparison and analysis of sequences are known. Unless otherwise stated, sequence identity/similarity values provided herein refer to the value obtained using GAP Version 10 (GCG, Accelrys, San Diego, CA) using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix (Henikoff & Henikoff (1992) Proc Natl Acad Sci USA 89:10915-10919). GAP uses the algorithm of Needleman & Wunsch (1970) J Mol Biol 48:443-453, to find the alignment of two complete sequences that maximizes the number of matches and minimizes the number of gaps. Substantially identical includes sequences having at least 80%, 85%, 90%, 91%, 92%, 93%, 94%, 95%, 96%, 97%, 98%, 99% or more sequence identity, wherein the sequences are expected to retain the native function based on the overall percent sequence identity, the sequence similarity, the overall alignment of primary sequence, the presence of conserved blocks of residues, the presence of conserved elements and/or domains, the presence of conserved functional domains, the presence of binding regions, the presence of catalytic residues, the predicted secondary and/or tertiary structure(s), the availability of known three-dimensional structures, and other criteria used by one of skill in the art to identify and predict a functional homologue of any particular sequence.

Variant polynucleotides include polynucleotides having at least one deletion, addition, and/or substitution in at least one of the 5' end, 3' end, and/or internal sites including introns or exons, as compared to the native polynucleotide. Variant polynucleotides include naturally occurring variants as well as synthetically derived polynucleotides, for example, those generated using site-directed mutagenesis. Conservative variants include sequences that maintain their function, encode the same polypeptide, or encode a variant polypeptide with substantially similar identity, function, and/or activity as the native polynucleotide. Variants can be identified with known techniques, for example, polymerase chain reaction (PCR), and/or hybridization techniques. Generally, variants of a particular polynucleotide will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular polynucleotide. Variant polynucleotides can also be evaluated by comparison of the percent sequence identity between the polypeptides encoded using standard alignment programs and parameters. When evaluated by comparison of the percent sequence identity shared by the two polypeptides each encodes, the percent sequence identity between the two encoded polypeptides is typically at least about 40%, 45%, 50%, 55%, 60%, 65%, 70%, 75%, 80%, 85%, 90%, 91 %, 92%, 93%, 94%, 95%, 96%, 97%, 98%, 99% or more sequence identity.

Variant proteins include proteins having at least one deletion, addition, and/or substitution in at least one of the N-terminal end, C-terminal end, and/or an internal site, as compared to the native polypeptide. Variant proteins possess the desired biological activity of the protein. Variants include naturally occurring polypeptides, as well as those generated by human manipulation. Biologically active variants of a protein typically have at least about 40%, 45%, 50%, 55%, 60%, 65%, 70%, 75%, 80%, 85%, 90%, 91 %, 92%, 93%, 94%, 95%, 96%, 97%, 98%, 99% or more sequence identity to the amino acid sequence for the native protein as determined by sequence alignment programs. A biologically active variant of a protein may differ from that protein by as few as 1-15 amino acid residues. Conservative substitutions generally refer to exchanging one amino acid with another having similar properties. For example, the model of Dayhoff et al. (1978) Atlas of Protein Sequence and Structure (Natl Biomed Res Found, Washington, D.C.) provides guidance on amino acid substitutions that are not expected to affect the biological activity of the protein.

Variant polynucleotides and proteins encompass sequences derived from mutagenic and/or recombinogenic procedures, such as mutagenesis and/or DNA shuffling. Methods for mutagenesis and nucleotide sequence alterations are known (see, *e.g.,* Kunkel (1985) Proc Natl Acad Sci USA 82:488-492; Kunkel et al. (1987) Methods Enzymol 154:367-382; U.S. Patent 4,873,192; Walker & Gaastra, eds. (1983) Techniques in Molecular Biology (MacMillan Publ. Co., NY) and the references cited therein). For example, one or more different recombinase coding sequences can be manipulated to create and select a new recombinase protein possessing the desired properties. Typically, libraries of recombinant polynucleotides are generated from a population of related sequences and can be homologously recombined *in vitro* or *in vivo* (see, *e.g.*, Stemmer (1994) Proc Natl Acad Sci USA 91:10747-10751; Stemmer (1994) Nature 370:389-391; Crameri et al. (1997) Nat Biotechnol 15:436-438; Moore et al. (1997) J Mol Biol 272:336-347; Zhang et al. (1997) Proc Natl Acad Sci USA 94:4504-4509; Crameri et al. (1998) Nature 391:288-291; and U.S. Patents 5,605,793, and 5,837,458). Generally, modifications in a polynucleotide encoding a polypeptide should not alter the reading frame, or create and/or alter DNA or mRNA secondary structure. See, EP Patent Application Publication No. 75,444.

Overlapping oligonucleotides, termed overgos, are primer pairs that span about 40 bp in length and are usually constituted from two 24-bp oligonucleotides that have an 8-bp overlapping region at the 3' ends. This feature allows the overgo primer pair to prime on each other and synthesize their complimentary strands with labeled nucleotides by the Klenow filling method (McPherson (1999) Genome Analysis: A Laboratory Manual, Vol. 4, pp. 207-213, ed. Birren et al., Cold Spring Harbor Laboratory Press, Cold Spring Harbor, NY). A variety of labeled nucleotides can be used, including but not limited to radioactive labeled nucleotides or fluorescent labeled nucleotides. This is useful for the generation of probes for different hybridization methods, including but not limited to colony hybridization, dot blots, Southern blots, and *in situ* hybridization, such as FISH. The major advantage of overgo probes over conventional probes for library hybridization is that the sequences for designing overgos can be selected, and thus repeated sequences present in a conventional DNA fragment probe can be avoided; therefore, the cross-hybridization problem that is frequently associated with large-genome DNA library screening can be minimized. Because of this advantage, overgo hybridization combined with the probe pooling strategy (Cai et al. (1998) Genomics 54:387-397; Chang et al. (2001) Genetics 159:1231-1242; Tao et al. (2001) Genetics 158:1711-1724; Romanov et al. (2003) Cytogenet Genome Res 101:277-281) has emerged as a method for high-throughput BAC library screening for clone identification and physical gene mapping.

In some examples genes or encoded polypeptides that can enhance or stimulate cell growth are provided with or within the DNA construct(s). Genes that enhance or stimulate cell growth include genes involved in transcriptional regulation, homeotic gene regulation, stem cell maintenance and proliferation, cell division, and/or cell differentiation such as WUS homologues (Mayer et al. (1998) Cell 95:805-815; WO01/0023575; US2004/0166563); aintegumenta (ANT) (Klucher et al. (1996) Plant Cell 8:137-153; Elliott et al. (1996) Plant Cell 8:155-168; GenBank Accession Nos. U40256, U41339, Z47554) ; clavata (e.g., CLV1, CVL2, CLV3) (WO03/093450; Clark et al. (1997) Cell 89:575-585; Jeong et al. (1999) Plant Cell 11:1925-1934; Fletcher et al. (1999) Science 283:1911-1914); Clavata and Embryo Surround region genes (*e.g.,* CLE) (Sharma et al. (2003) Plant Mol Biol 51:415-425; Hobe et al. (2003) Dev Genes Evol 213:371-381; Cock & McCormick (2001) Plant Physiol 126:939-942; Casamitjana-Martinez et al. (2003) Curr Biol 13:1435-1441); baby boom (*e.g.*, BNM3, BBM, ODP1, ODP2) (WO00/75530; Boutileir et al. (2002) Plant Cell 14:1737-1749); Zwille (Lynn et al. (1999) Dev 126:469-481); leafy cotyledon (*e.g.*, Lec1, Lec2) (Lotan et al. (1998) Cell 93:1195-1205; WO00/28058; Stone et al. (2001) Proc Natl Acad Sci USA 98:11806-11811; U.S. Patent 6,492,577); Shoot Meristem-less (STM) (Long et al. (1996) Nature 379:66-69); ultrapetala (ULT) (Fletcher (2001) Dev 128:1323-1333); mitogen activated protein kinase (MAPK) (Jonak et al. (2002) Curr Opin Plant Biol 5:415); kinase associated protein phosphatase (KAPP) (Williams et al. (1997) Proc Natl Acad Sci USA 94:10467-10472; Trotochaud et al. (1999) Plant Cell 11:393-406); ROP GTPase (Wu et al. (2001) Plant Cell 13:2841-2856; Trotochaud et al. (1999) Plant Cell 11:393-406); fasciata (*e.g.* FAS1, FAS2) (Kaya et al. (2001) Cell 104:131-142); cell cycle genes (U.S. Patent 6,518,487; WO99/61619; WO02/074909), Shepherd (SHD) (Ishiguro et al. (2002) EMBO J. 21:898-908); Poltergeist (Yu et al. (2000) Dev 127:1661-1670; Yu et al. (2003) Curr Biol 13:179-188); Pickle (PKL) (Ogas et al. (1999) Proc Natl Acad Sci USA 96:13839-13844); knox genes (e.g., KN1, KNAT1) (Jackson et al. (1994) Dev 120:405-413; Lincoln et al. (1994) Plant Cell 6:1859-1876; Venglat et al. (2002) Proc Natl Acad Sci USA 99:4730-4735); fertilization independent endosperm (FIE) (Ohad et al. (1999) Plant Cell 11:407-415), and the like. The combinations of polynucleotides include multiple copies of any one of the polynucleotides of interest, and the combinations may have any combination of up-regulating and down-regulating expression of the combined polynucleotides. The combinations may or may not be combined on one construct for transformation of the host cell, and therefore may be provided sequentially or simultaneously. The host cell may be a wild-type or mutant cell, in a normal or aneuploid state.

Site-specific recombinase systems can be used with any minichromosome system. Both integrases and recombinases capable of catalyzing both the forward and reverse reactions, are useful for introducing modifications after the DNA construct(s) or minichromosome has been established in the plant cell. Various intramolecular modifications, such as deletion or inversion of defined sequences can be done. Further, intermolecular insertions and exchanges can be done, including translocations with endogenous chromosomes comprising compatible site-specific recombination sites. The recombinase systems can also be used to establish target sites (docking sites) within the minichromosome for later site specific integration of polynucleotide(s) of interest provided by any method, including crossing or direct delivery.

Elements from recombination systems, such as recombinases, and recombination sites can be used, for example in a DNA construct, a target site, and/or a transfer cassette. A target site comprises a polynucleotide integrated into the genome, the polynucleotide comprising a promoter operably linked to at least one recombination site. A transfer cassette comprises at least a first recombination site operably linked to a polynucleotide of interest and/or a polynucleotide encoding a selection marker, wherein the first recombination site is recombinogenic with a recombination site in the target site. A targeted seed or plant has stably incorporated into its genome a DNA construct that has been generated and/or manipulated through the use of a recombination system. Site-specific recombination methods that result in various integration, alteration, and/or excision events to generate the recited DNA construct can be employed to generate a targeted seed. See, e.g., WO99/25821, WO99/25854, WO99/25840, WO99/25855, WO99/25853, WO99/23202, WO99/55851, WO01/07572, WO02/08409, and WO03/08045.

A recombinase is a polypeptide that catalyzes site-specific recombination between its compatible recombination sites, and includes naturally occurring recombinase sequences, variants, and/or fragments that retain activity. A recombination site is a nucleotide sequence that is specifically recognized by a recombinase enzyme, and encompasses naturally occurring recombination site sequences, variants, and/or fragments that retain activity. For reviews of site-specific recombinases, see Sauer (1994) Curr Op Biotech 5:521-527; Sadowski (1993) FASEB 7:760-767; Groth & Calos (2004) J Mol Biol 335:667-678; and Smith & Thorpe (2002) Mol Microbiol 44:299-307. Any recombination system, or combination of systems, can be used including but not limited to recombinases and recombination sites from the integrase and/or resolvase families, biologically active variants and fragments thereof, and/or any other naturally occurring or recombinantly produced enzyme or variant thereof that catalyzes conservative site-specific recombination between specified recombination sites, and naturally occurring or modified recombination sites or variants thereof that are specifically recognized by a recombinase to generate a recombination event.

The recombination sites employed can be corresponding sites or dissimilar sites. Corresponding recombination sites, or a set of corresponding recombination sites, are sites having an identical nucleotide sequence. A set of corresponding recombination sites, in the presence of the appropriate recombinase, will efficiently recombine with one another. Dissimilar recombination sites have a distinct sequence, comprising at least one nucleotide difference as compared to each other. The recombination sites within a set of dissimilar recombination sites can be either recombinogenic or non-recombinogenic with respect to one another. Each recombination site within the set of dissimilar sites is biologically active and can recombine with an identical site. Recombinogenic sites are capable of recombining with one another in the presence of the appropriate recombinase. Recombinogenic sites include those sites where the relative excision efficiency of recombination between the recombinogenic sites is above the detectable limit under standard conditions in an excision assay as compared to the wild type control, typically, greater than 2%, 5%, 10%, 20%, 50%, 100%, or greater. Non-recombinogenic sites will not recombine with one another in the presence of the appropriate recombinase, or recombination between the sites is not detectable. Non-recombinogenic recombination sites include those sites that recombine with one another at a frequency lower than the detectable limit under standard conditions in an excision assay as compared to the wild type control, typically, lower than 2%, 1.5%, 1%, 0.75%, 0.5%, 0.25%, 0.1%, 0.075, 0.005%, 0.001%. Any suitable non-recombinogenic recombination sites may be utilized, including a FRT site or active variant thereof, a lox site or active variant thereof, an att site or active variant thereof, any combination thereof, or any other combination of non-recombinogenic recombination sites. Directly repeated recombination sites in a set of recombinogenic recombination sites are arranged in the same orientation, recombination between these sites results in excision of the intervening DNA sequence. Inverted recombination sites in a set of recombinogenic recombination sites are arranged in the opposite orientation, recombination between these sites results in inversion of the intervening DNA sequence.

The Integrase family of recombinases has over one hundred members and includes, for example, FLP, Cre, Dre, Int, and R. For other members of the Integrase family, see for example, Esposito et al. (1997) Nucleic Acids Res 25:3605-3614; Nunes-Duby et al. (1998) Nucleic Acids Res 26:391-406; Abremski et al. (1992) Protein Eng 5:87-91; Groth & Calos (2004) J Mol Biol 335:667-678; and Smith & Thorpe (2002) Mol Microbiol 44:299-307. Other recombination systems include, for example, streptomycete bacteriophage phiC31 (Kuhstoss et al. (1991) J Mol Biol 20:897-908); bacteriophage λ (Landy (1989) Ann Rev Biochem 58:913-949, and Landy (1993) Curr Op Genet Dev 3:699-707); SSV1 site-specific recombination system from *Sulfolobus shibatae* (Maskhelishvili et al. (1993) Mol Gen Genet 237:334-342); and a retroviral integrase-based integration system (Tanaka et al. (1998) Gene 17:67-76). In some examples, the recombinase is one that does not require cofactors or a supercoiled substrate. Such recombinases include Cre, FLP, phiC31 Int, mutant λ Int, R, SSV1, Dre, or active variants or fragments thereof. FLP recombinase catalyzes a site-specific reaction between two FRT sites, and is involved in amplifying the copy number of the two-micron plasmid of *S*. *cerevisiae* during DNA replication. The FLP protein has been cloned and expressed. See, for example, Cox (1993) Proc Natl Acad Sci USA 80:4223-4227. The FLP recombinase used may be derived from the genus *Saccharomyces.* In some examples a polynucleotide synthesized using plant-preferred codons encoding the recombinase is used. FLP enzyme encoded by a nucleotide sequence comprising maize preferred codons (FLPm) that catalyzes site-specific recombination events is known (U.S. Patent 5,929,301). Additional functional variants and fragments of FLP are known. See, for example, Buchholz et al. (1998) Nat Biotechnol 16:617-618, Hartung et al. (1998) J Biol Chem 273:22884-22891, Saxena et al. (1997) Biochim Biophys Acta 1340:187-204, Hartley et al. (1980) Nature 286:860-864, Shaikh & Sadowski (2000) J Mol Biol 302:27-48, Voziyanov et al. (2002) Nucleic Acids Res 30:1656-1663, and Voziyanov et al. (2003) J Mol Biol 326:65-76. The bacteriophage P1 recombinase Cre catalyzes site-specific recombination between two lox sites. See, for example, Guo et al. (1997) Nature 389:40-46; Abremski et al. (1984) J Biol Chem 259:1509-1514; Chen et al. (1996) Somat Cell Mol Genet 22:477- 488; Shaikh et al. (1977) J Biol Chem 272:5695- 5702; and, Buchholz et al. (1998) Nat Biotechnol 16:617-618. Cre polynucleotide sequences may also be synthesized using plant-preferred codons, for example, moCre (see, *e.g.*, WO 99/25840), and other variants are known, see for example Vergunst et al. (2000) Science 290:979-982, Santoro & Schulz (2002) Proc Natl Acad Sci USA 99:4185-4190, Shaikh & Sadowski (2000) J Mol Biol 302:27-48, Rufer & Sauer (2002) Nucleic Acids Res 30:2764-2771, Wierzbicki et al. (1987) Mol Biol 195:785-794, Petyuk et al. (2004) J Biol Chem 279:37040-37048, Hartung & Kisters-Wolke (1998) J Biol Chem 273:22884-22891, Koresawa et al. (2000) J Biochem (Tokyo) 127:367-372, U.S. Patent 6,890,726, and Buchholz & Stewart (2001) Nat Biotechnol 19:1047-1052. A Cre homolog has been identified in P1-related phages, the recombinase isolated from phage D6 is known as Dre which is a tyrosine recombinase closely related to Cre, but which recognizes distinct 32 bp rox sites (Sauer & McDermott (2004) Nucleic Acids Res 32:1-10). The phiC31 integrase and variants are known (Kushtoss et al. (1991) J Mol Biol 222:897-908, WO03/066867, WO05/017170, US2005/0003540, and Sclimenti et al. (2001) Nucleic Acids Res 29:5044-5051. The λ integrase and cofactors (Hoess et al. (1980) Proc Natl Acad Sci USA 77:2482-2486, Blattner et al. (1997) Science 277:1453-1474), and variants thereof are known, including cofactor-independent Int variants (Miller et al. (1980) Cell 20:721-729, Lange-Gustafson and Nash (1984) J Biol Chem 259:12724-12732, Christ et al. (1998) J Mol Biol 288:825-836, and Lorbach et al. (2000) J Mol Biol 296:1175-1181), att site recognition variants (Dorgai et al. (1995) J Mol Biol 252:178-188, Yagu et al. (1995) J Mol Biol 252:163-167, and Dorgai et al. (1998) J Mol Biol 277:1059-1070), as well as maize codon optimized Int, variant, and cofactor sequences (WO03/08045). Other integrases and variants are known, such as HK022 integrase (Kolot et al. (1999) Mol Biol Rep 26:207-213) and variants such as att site recognition variants (Dorgai et al. (1995) J Mol Biol 252:178-188, Yagu et al. (1995) J Mol Biol 252:163-167, and Dorgai et al. (1998) J Mol Biol 277:1059-1070).

Wild-type recombination sites, mutant, or any combination of wild type and/or mutant sites can be used. Such recombination sites include, for example, wild type lox, FRT, and att sites, and mutant lox, FRT, and att sites. An analysis of the recombination activity of mutant lox sites is presented in Lee et al. (1998) Gene 216:55-65. Other recombination sites and variants are known, see for example, Hoess et al. (1982) Proc Natl Acad Sci USA 79:3398-3402; Hoess et al. (1986) Nucleic Acids Res 14:2287-2300; Thomson et al. (2003) Genesis 36:162-167; Schlake & Bode (1994) Biochemistry 33:12746-12751; Siebler & Bode (1997) Biochemistry 36:1740-1747; Huang et al. (1991) Nucleic Acids Res 19:443-448; Sadowski (1995) in Progress in Nucleic Acid Research and Molecular Biology Vol. 51, pp. 53-91; Cox (1989) in Mobile DNA, Berg & Howe (eds) American Society of Microbiology, Washington D.C., pp. 116-670; Dixon et al. (1995) Mol Microbiol 18:449-458; Umlauf & Cox (1988) EMBO J 7:1845-1852; Buchholz et al. (1996) Nucleic Acids Res 24:3118-3119; Kilby et al. (1993) Trends Genet 9:413-421; Rossant & Geagy (1995) Nat Med 1:592-594; Bayley et al. (1992) Plant Mol Biol 18:353-361; Odell et al. (1990) Mol Gen Genet 223:369-378; Dale & Ow (1991) Proc Natl Acad Sci USA 88:10558-10562; Qui et al. (1994) Proc Natl Acad Sci USA 91:1706-1710; Stuurman et al. (1996) Plant Mol Biol 32:901-913; Dale et al. (1990) Gene 91:79-85; Albert et al. (1995) Plant J 7:649-659, U.S. Patent 6,465,254, WO01/2354 WO99/55851, and WO01/11058. In some examples, sets of dissimilar and corresponding recombination sites can be used, for example sites from different recombination systems. Accordingly, any suitable recombination site or set of recombination sites may be used, including a FRT site, a biologically active variant of a FRT site, a lox site, a biologically active variant of a lox site, an att site, a biologically active variant of an att site, any combination thereof, or any other combination of recombination sites. Examples of FRT sites include, for example, the minimal wild type FRT site (FRT1), and various mutant FRT sites, including but not limited to FRT5, FRT6, and FRT7 (see U.S. Patent 6,187,994). Additional variant FRT sites are known, (see, *e.g.*, WO01/23545, and U.S. publication 2007/0015195, herein incorporated by reference). Other recombination sites that can be used include att sites, such as those disclosed in Landy (1989) Ann Rev Biochem 58:913-949, Landy (1993) Curr Op Genet Dev 3:699-707, U.S. Patent 5,888,732, WO01/07572, and Thygarajan et al. (2001) Mol Cell Biol 21:3926-3934. The site-specific recombinase(s) used depend on the recombination sites in the target site and the transfer cassette. If FRT sites are utilized, FLP recombinase is provided, when lox sites are utilized, Cre recombinase is provided, when λ att sites are used, λ Int is provided, when phiC31 att sites are used, phiC31 Int is provided. If the recombination sites used comprise sites from different systems, for example a FRT and a lox site, both recombinase activities can be provided, either as separate entities, or as a chimeric recombinase, for example FLP/Cre (see, *e.g.*, WO 99/25840).

A marker provides for the identification and/or selection of a cell, plant, and/or seed expressing the marker. Markers include, *e.g.*, screenable, visual, and/or selectable marker. A selection marker is any marker, which when expressed at a sufficient level, confers resistance to a selective agent. For example visual markers can be used to identify transformed cells comprising the introduced DNA construct(s). In one example the visual marker is a fluorescent protein. Such fluorescent proteins include but are not limited to yellow fluorescent protein (YFP), green fluorescent protein (GFP), cyan fluorescent protein (CFP), and red fluorescent protein (RFP). In still other examples, the visual marker is encoded by a polynucleotide having maize preferred codons. In further examples, the visual marker comprises GFPm, AmCyan, ZsYellow, or DsRed. See, Wenck et al. (2003) Plant Cell Rep. 22:244-251.

Selection markers and their corresponding selective agents include, but are not limited to, herbicide resistance genes and herbicides; antibiotic resistance genes and antibiotics; and other chemical resistance genes with their corresponding chemical agents. Bacterial drug resistance genes include, but are not limited to, neomycin phosphotransferase II (nptII) which confers resistance to kanamycin, paromycin, neomycin, and G418, and hygromycin phosphotransferase (hph) which confers resistance to hygromycin B. See also, Bowen (1993) Markers for Plant Gene Transfer, Transgenic Plants, Vol. 1, Engineering and Utilization; Everett et al. (1987) Bio/Technology 5:1201-1204; Bidney et al. (1992) Plant Mol Biol 18:301-313; and WO97/05829.

Resistance may also be conferred to herbicides from several groups, including amino acid synthesis inhibitors, photosynthesis inhibitors, lipid inhibitors, growth regulators, cell membrane disrupters, pigment inhibitors, seedling growth inhibitors, including but not limited to imidazolinones, sulfonylureas, triazolopyrimidines, glyphosate, sethoxydim, fenoxaprop, glufosinate, phosphinothricin, triazines, bromoxynil, and the like. See, for example, Holt (1993) Ann Rev Plant Physiol Plant Mol Biol 44:203-229; and Miki et al. (2004) J Biotechnol 107:193-232. Selection markers include sequences that confer resistance to herbicides, including but not limited to, the bar gene, which encodes phosphinothricin acetyl transferase (PAT) which confers resistance to glufosinate (Thompson et al. (1987) EMBO J 6:2519-2523); glyphosate oxidoreductase (GOX), glyphosate N-acetyltransferase (GAT), and 5-enol pyruvylshikimate-3-phosphate synthase (EPSPS) which confer resistance to glyphosate (Barry et al. (1992) in Biosynthesis and Molecular Regulation of Amino Acids in Plants, B.K. Singh et al. (Eds) pp.139-145; Kishore et al. (1992) Weed Tech 6:626-634; Castle (2004) Science 304:1151-1154; Zhou et al. (1995) Plant Cell Rep 15:159-163; WO97/04103; WO02/36782; and WO03/092360). Other selection markers include dihydrofolate reductase (DHFR), which confers resistance to methotrexate (see, *e.g.*, Dhir et al. (1994) Improvements of Cereal Quality by Genetic Engineering, R.J. Henry (ed), Plenum Press, New York; and Hauptmann et al. (1988) Plant Physiol 86:602- 606). Acetohydroxy acid synthase (AHAS or ALS) mutant sequences lead to resistance to imidiazolinones and/or sulfonylureas such as imazethapyr and/or chlorsulfuron (see, *e.g.*, Zu et al. (2000) Nat Biotechnol 18:555-558; U.S. Patents 6,444,875, and 6,660,910; Sathasivan et al. (1991) Plant Physiol 97:1044-1050; Ott et al. (1996) J Mol Biol 263:359-368; and Fang et al. (1992) Plant Mol Biol 18:1185-1187).

In addition, chemical resistance genes further include tryptophan decarboxylase which confers resistance to 4-methyl tryptophan (4-mT) (Goodijn et al. (1993) Plant Mol Biol 22:907-912); and bromoxynil nitrilase which confers resistance to bromoxynil. The selection marker may comprise cyanamide hydratase (Cah), see, for example, Greiner et al. (1991) Proc Natl Acad Sci USA 88:4260-4264; and Weeks et al. (2000) Crop Sci 40:1749-1754. Cyanamide hydratase enzyme converts cyanamide into urea, thereby conferring resistance to cyanamide. Any form or derivative of cyanamide can be used as a selection agent including, but not limited to, calcium cyanamide (Perlka® (SKW, Trotberg Germany) and hydrogen cyanamide (Dormex® (SKW)). See also, U.S. Patents 6,096,947, and 6,268,547. Variants of cyanamide hydratase polynucleotides and/or polypeptides will retain cyanamide hydratase activity. A biologically active variant of cyanamide hydratase will retain the ability to convert cyanamide to urea. Methods to assay for such activity include assaying for the resistance of plants expressing the cyanamide hydratase to cyanamide. Additional assays include the cyanamide hydratase colorimetric assay (see, *e.g.*, Weeks et al. (2000) Crop Sci 40:1749-1754; and U.S. Patent 6,268,547).

The present invention also concerns an isolated polynucleotide comprising: (a) at least two arrays of tandem repeats of CentC in an inverted orientation wherein the first array comprises at least ten copies of CentC and the second array comprises at least ten copies of CentC; and, (b) at least one copy of a retrotransposable element, wherein the retrotransposable element is situated between the first and the second array. Suitable retrotransposable elements are discussed above.

The isolated polynucleotides comprise at least two arrays of tandem repeats of CentC. Each array of CentC repeats may comprise at least 10, 15, 20, 25, 30, 40, 50, 60, 70, 80, 90, 100, 120, 140, 150, 160, 180, 200, 220, 240, 250, 260, 280, or 300 copies of CentC. Further, each array of tandem repeats of CentC may be interrupted by another sequence element, including but not limited to a retrotransposon, which is inserted between copies of CentC, or within a CentC element, or within a retrotransposon, or any other sequence element in the array. Retrotransposons include, but are not limited to, CentA, CRM1, and CRM2.

A polynucleotide includes any nucleic acid molecule, and comprises naturally occurring, synthetic, and/or modified ribonucleotides, deoxyribonucleotides, and combinations of ribonucleotides and deoxyribonucleotides. Polynucleotides encompass all forms of sequences including, but not limited to, single-stranded, double-stranded, linear, circular, branched, hairpins, stem-loop structures, and the like.

Also within the scope of the invention is a recombinant construct comprising any of the isolated polynucleotides of the invention.

A recombinant DNA construct comprises a polynucleotide which when present in the genome of a plant is heterologous or foreign to that chromosomal location in the plant genome. In preparing the DNA construct, various fragments may be manipulated to provide the sequences in a proper orientation and/or in the proper reading frame. Adapters or linkers may be employed to join the fragments. Other manipulations may be used to provide convenient restriction sites, removal of superfluous DNA, or removal of restriction sites. For example, *in vitro* mutagenesis, primer repair, restriction, annealing, resubstitutions, transitions, transversions, or recombination systems may be used. Polynucleotides of interest refer to any nucleic acid molecule included in the DNA construct(s) for any purpose, including but not limited to untranslated regions, regulatory regions, transcription initiation regions, translation initiation regions, introns, exons, polynucleotides encoding an RNA, selection markers, screenable markers, phenotypic markers, polynucleotides encoding a recombinase, recombination sites, target sites, transfer cassettes, restriction sites, recognition sites, insulators, enhancers, spacer/stuffer sequences, origins of replication, telomeric sequence, operators, and the like, can be provided in a DNA construct(s). The construct can include 5' and 3' regulatory sequences operably linked to the appropriate sequences. The DNA construct(s) can include in the 5' to 3' direction of transcription at least one of the following, a transcriptional and translational initiation region, the polynucleotide, and a transcriptional and translational termination region functional in plants. Alternatively, the DNA construct(s) may lack at least one 5' and/or 3' regulatory element. For example, DNA construct(s) may be designed such that upon introduction into a cell and in the presence of the appropriate recombinase a recombination event at the target site operably links the 5' and/or 3' regulatory regions to the appropriate sequences of the DNA construct(s).

Regulatory elements can be used in a variety of ways depending on the polynucleotide element, recombination site, transfer cassette and/or target site employed. In some examples intervening sequences can be present between operably linked elements and not disrupt the functional linkage. For example, an operable linkage between a promoter and a polynucleotide of interest allows the promoter to initiate and mediate transcription of the polynucleotide of interest. In some examples a translational start site is operably linked to a recombination site. In some examples, a recombination site is within an intron.

A cassette may additionally contain at least one additional sequence to be introduced into the plant. Alternatively, additional sequence(s) can be provided separately. DNA constructs can be provided with a plurality of restriction sites or recombination sites for manipulation of the various components and elements. DNA constructs may additionally contain selectable marker genes.

A transcriptional initiation region may be native, analogous, foreign, or heterologous to the plant host or to the polynucleotide of interest, and may be a natural sequence, a modified sequence, or a synthetic sequence. A number of promoters can be used to express a coding sequence.

A variety of promoters useful in plants is reviewed in Potenza et al. (2004) In Vitro Cell Dev Biol Plant 40:1-22. In some examples, the promoter expressing the selection marker is active in the seed. Promoters active in the seed include constitutive promoters, for example, the core promoter of the Rsyn7 promoter and other constitutive promoters disclosed in WO99/43838 and U.S. Patent 6,072,050; the core CaMV 35S promoter (Odell et al. (1985) Nature 313:810-812); the MVV (mirabilis mosaic virus) promoter (Dey & Maiti (1999) Plant Mol Biol 40:771-782); rice actin (McElroy et al. (1990) Plant Cell 2:163-171); ubiquitin (Christensen et al. (1989) Plant Mol Biol.12:619-632, and Christensen et al. (1992) Plant Mol Biol 18:675-689); pEMU (Last et al. (1991) Theor Appl Genet 81:581-588); MAS (Velten et al. (1984) EMBO J 3:2723-2730); ALS promoter (U.S. Patent 5,659,026), and the like. Other constitutive promoters include those disclosed in, *e.g.*, U.S. Patents 5,608,149; 5,608,144; 5,604,121; 5,569,597; 5,466,785; 5,399,680; 5,268,463; 5,608,142; and 6,177,611.

The promoter may be a tissue-preferred promoter, to target enhanced expression within a particular plant tissue. In some examples, a seed-preferred promoter is used to express the selection marker. Seed-preferred promoters include both seed-specific promoters, active during seed development, as well as seed-germinating promoters, active during seed germination. See Thompson et al. (1989) BioEssays 10:108. Seed-preferred promoters include, but are not limited to, Cim1 (cytokinin-induced message); cZ19B1 (maize 19 kDa zein); milps (myo-inositol-1-phosphate synthase) (see WO00/11177, and U.S. Patent 6,225,529), bean β-phaseolin, napin, β-conglycinin, soybean lectin, cruciferin, maize 15 kDa zein, 22 kDa zein, 27 kDa zein, waxy, shrunken 1, shrunken 2, globulin 1, end1, and end2 (WO00/12733), and the like.

A chemical-regulated promoter can be used to modulate expression in the seed through the application of an exogenous chemical regulator. The promoter may be a chemical-inducible promoter, where application of the chemical induces gene expression, or a chemical-repressible promoter, where application of the chemical represses gene expression. Chemical-inducible promoters include, but are not limited to, the maize ln2-2 promoter, activated by benzenesulfonamide herbicide safeners; the maize GST promoter, activated by hydrophobic electrophilic compounds (*e.g.*, some pre-emergent herbicides); and the tobacco PR-1 a promoter, activated by salicylic acid. Other chemical-regulated promoters of interest include steroid-responsive promoters (see, for example, the glucocorticoid-inducible promoter in Schena et al. (1991) Proc Natl Acad Sci USA 88:10421-10425 and McNellis et al. (1998) Plant J 14:247-257) and tetracycline-inducible and tetracycline-repressible promoters (see, *e.g.*, Gatz et al. (1991) Mol Gen Genet 227:229-237, and U.S. Patents 5,814,618, and 5,789,156).

The DNA construct(s) can comprise expression units. Expression units can have elements including, but not limited to, introns, enhancers, leaders insulators, spacers, regions encoding an RNA, marker genes, recombination sites, termination regions, sequences encoding recombinases, enhancers, linkers, recognition sites, *etc.* In addition, the DNA constructs can comprise transfer cassettes, target sites, or any portions or combinations thereof. The DNA construct(s) can be modified in a variety of ways including but limited to site-specific recombination/integration methods or transposon-based transpositions, to provide a number of variations in the DNA construct(s). Polynucleotide sequences may be modified for expression in the plant. See, *e.g.*, Campbell & Gowri (1990) Plant Physiol 92:1-11. Methods for synthesizing plant-preferred genes include, *e.g.*, U.S. Patents 5,380,831, 5,436,391, and Murray et al. (1989) Nucleic Acids Res 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to average levels for a given host, as calculated by reference to endogenous genes expressed in the host. The sequence may also be modified to avoid secondary mRNA structures. Cassettes may additionally contain 5' leader sequences in the DNA cassette which may act to enhance translation. Translation leaders include, *e.g*., pimaizeavirus leaders such as EMCV leader (Elroy-Stein et al. (1989) Proc Natl Acad Sci USA 86:6126-6130); potyvirus leaders such as TEV leader (Gallie et al. (1995) Gene 165:233-238), MDMV leader (Kong et al. (1988) Arch Virol 143:1791-1799), and human immunoglobulin heavy-chain binding protein (BiP) (Macejak et al. (1991) Nature 353:9094); untranslated leader from the coat protein RNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol 84:965-968. Other methods or sequences known to enhance translation can also be utilized, such as introns, and the like.

Sequences of interest include, *e.g.*, zinc fingers, kinases, heat shock proteins, transcription factors, DNA repair, agronomic traits, insect resistance, disease resistance, herbicide resistance, sterility, oil, protein, starch, digestibility, kernel size, maturity, nutrient composition, levels or metabolism, and the like. Insect resistance genes may encode resistance to pests such as rootworm, cutworm, European Maize Borer, and the like. Such genes include, *e.g., B. thuringiensis* toxic protein genes (U.S. Patents 5,366,892; 5,747,450; 5,736,514; 5,723,756; 5,593,881; Geiser et al. (1986) Gene 48:109) and the like. Disease resistance traits include detoxification genes, such as against fumonosin (U.S. Patent 5,792,931); avirulence (avr) and disease resistance (R) genes (Jones et al. (1994) Science 266:789; Martin et al. (1993) Science 262:1432; Mindrinos et al. (1994) Cell 78:1089); and the like. Herbicide resistance traits include genes coding for resistance to herbicides including sulfonylurea-type herbicides (*e.g.*, the S4 and/or Hra mutations in ALS), herbicides that act to inhibit action of glutamine synthase, such as phosphinothricin or basta (*e.g.*, the bar gene), EPSPS (U.S. Patents 6,867,293; 5,188,642; and 5,627,061), GOX (Zhou et al. (1995) Plant Cell Rep 15:159-163), and GAT (U.S. Patent 6,395,485). Antibiotic resistance genes may also be used, such as the nptII gene which encodes resistance to the antibiotics kanamycin and geneticin. Sterility genes can also be used, for example as an alternative to detasseling, including male tissue-preferred genes and genes with male sterility phenotypes such as QM (*e.g.*, U.S. Patent 5,583,210), kinases, and those encoding compounds toxic to either male or female gametophytic development.

Reduction of the activity of specific genes, silencing and/or suppression may be desired. Many techniques for gene silencing are known, including but not limited to antisense technology (see, *e.g.,* Sheehy et al. (1988) Proc Natl Acad Sci USA 85:8805-8809; and U.S. Patents 5,107,065; 5,453, 566; and 5,759,829); cosuppression (*e.g.*, Taylor (1997) Plant Cell 9:1245; Jorgensen (1990) Trends Biotech 8:340-344; Flavell (1994) Proc Natl Acad Sci USA 91:3490-3496; Finnegan et al. (1994) Bio/Technology 12:883-888; and Neuhuber et al. (1994) Mol Gen Genet 244:230-241); RNA interference (Napoli et al. (1990) Plant Cell 2:279-289; U.S. Patent 5,034,323; Sharp (1999) Genes Dev 13:139-141; Zamore et al. (2000) Cell 101:25-33; Javier (2003) Nature 425:257-263; and, Montgomery et al. (1998) Proc Natl Acad Sci USA 95:15502-15507), virus-induced gene silencing (Burton et al. (2000) Plant Cell 12:691-705; and Baulcombe (1999) Curr Op Plant Bio 2:109-113); target-RNA-specific ribozymes (Haseloff et al. (1988) Nature 334: 585-591); hairpin structures (Smith et al. (2000) Nature 407:319-320; WO99/53050; WO02/00904; and WO98/53083); ribozymes (Steinecke et al. (1992) EMBO J 11:1525; U.S. Patent 4,987,071; and, Perriman et al. (1993) Antisense Res Dev 3:253); oligonucleotides mediated targeted modification (*e.g.*, WO03/076574: and WO99/25853); Zn-finger targeted molecules (*e.g.*, WO01/52620; WO03/048345; and WO00/42219); and other methods, or combinations of the above methods.

The termination region may be native with the transcriptional initiation region, may be native with the operably linked DNA sequence of interest, or may be derived from another source. Convenient termination regions are available from the Ti-plasmid of *A*. *tumefaciens,* such as the octopine synthase and nopaline synthase termination regions. See also Guerineau et al. (1991) Mol Gen Genet 262:141-144; Proudfoot (1991) Cell 64:671-674; Sanfacon et al. (1991) Genes Dev 5:141-149; Mogen et al. (1990) Plant Cell 2:1261-1272; Munroe et al. (1990) Gene 91:157-158; Ballas et al. (1989) Nucleic Acids Res 17:7891-7903; and Joshi et al. (1987) Nucleic Acids Res 15:9627-9639.

The mixture can further comprise a polynucleotide encoding a polypeptide that stimulates cell growth. Examples of polypeptides that stimulate cell growth include, but are not limited to, a wuschel, a baby boom, a RepA, or a Lec1.

Any method for introducing a sequence into a plant can be used, as long as the polynucleotide or polypeptide gains access to the interior of at least one cell. Methods for introducing sequences into plants are known and include, but are not limited to, stable transformation, transient transformation, virus-mediated methods, and sexual breeding. Stably incorporated indicates that the introduced polynucleotide is integrated into a genome and is capable of being inherited by progeny. Transient transformation indicates that an introduced sequence does not integrate into a genome such that it is heritable by progeny from the host. The plants and seeds employed may have a DNA construct stably incorporated into their genome. Any protocol may be used to introduce the DNA construct, any component of site-specific recombination systems, a polypeptide, or any other polynucleotide of interest. Providing comprises any method that brings together any polypeptide and/or a polynucleotide with any other recited components. Any means can be used to bring together a target site, transfer cassette, and appropriate recombinase, including, for example, stable transformation, transient delivery, and sexual crossing (see, e.g., WO99/25884). In some examples, the recombinase may be provided in the form of the polypeptide or mRNA. A series of protocols may be used in order to bring together the various components. For instance, a cell can be provided with at least one of these components via a variety of methods including transient and stable transformation methods; co-introducing a recombinase DNA, mRNA or protein directly into the cell; employing an organism (*e.g.,* a strain or line) that expresses the recombinase; or growing/culturing the cell or organism carrying a target site, crossing to an organism expressing an active recombinase protein, and selecting events in the progeny. A simple integration pattern is produced when the transfer cassette integrates predominantly at the target site. Any promoter, including constitutive, inducible, developmentally, temporal, and/or spatially regulated promoter, *etc.*, that is capable of regulating expression in the organism may be used.

Transformation protocols as well as protocols for introducing polypeptides or polynucleotide sequences into plants may vary depending on the type of plant or plant cell targeted for transformation. Suitable methods of introducing polypeptides and polynucleotides into plant cells include microinjection (Crossway et al. (1986) Biotechniques 4:320-334, U.S. Patent 6,300,543; and U.S. Applications 11/427,947 and 11/427,371 all of which are herein incorporated by reference), electroporation (Riggs et al. (1986) Proc Natl Acad Sci USA 83:5602-5606, *Agrobacterium*-mediated transformation (U.S. Patents 5,563,055; and 5,981,840), direct gene transfer (Paszkowski et al. (1984) EMBO J 3:2717-2722), and ballistic particle acceleration (U.S. Patents 4,945,050; 5,879,918; 5,886,244; and 5,932,782; Tomes et al. (1995) in Plant Cell, Tissue, and Organ Culture: Fundamental Methods, ed. Gamborg & Phillips (Springer-Verlag, Berlin); McCabe et al. (1988) Biotechnology 6:923-926); and Lec1 transformation (WO00/28058). Also see Weissinger et al. (1988) Ann Rev Genet 22:421-477; Sanford et al. (1987) Particulate Science and Technology 5:27-37 (onion); Christou et al. (1988) Plant Physiol 87:671-674 (soybean); Finer & McMullen (1991) In Vitro Cell Dev Biol 27P:175-182 (soybean); Singh et al. (1998) Theor Appl Genet 96:319-324 (soybean); Datta et al. (1990) Biotechnology 8:736-740 (rice); Klein et al. (1988) Proc Natl Acad Sci USA 85:4305-4309 (maize); Klein et al. (1988) Biotechnology 6:559-563 (maize); U.S. Patents 5,240,855; 5,322,783; and, 5,324,646; Klein et al. (1988) Plant Physiol 91:440- 444 (maize); Fromm et al. (1990) Biotechnology 8:833-839 (maize); Hooykaas-Van Slogteren et al. (1984) Nature 311:763-764; U.S. Patent 5,736,369 (cereals); Bytebier et al. (1987) Proc Natl Acad Sci USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Rep 9:415-418; and Kaeppler et al. (1992) Theor Appl Genet 84:560-566 (whisker-mediated transformation); D'Halluin et al. (1992) Plant Cell 4:1495-1505 (electroporation); Li et al. (1993) Plant Cell Rep 12:250-255; Christou & Ford (1995) Ann Bot 75:407-413 (rice); Osjoda et al. (1996) Nat Biotechnol 14:745-750 (maize via A. tumefaciens); and Ch. 8, pp. 189-253 in Advances in Cellular and Molecular Biology of Plants, Vol. 5, Ed. Vasil, Kluwer Acad Publ (Dordrecht, The Netherlands) 1999.

Various compounds can be used in conjunction with any direct delivery methods for introducing into plant cells any polynucleotide, polypeptide, or combinations thereof, optionally containing other components. For example, microprojectiles for a particle gun method can be prepared by associating DNA construct(s) with the microprojectiles in the presence of a cationic lipid solution, liposome solution, cationic polymer, DNA binding protein, cationic protein, cationic peptide, cationic polyamino acid, or combination thereof. In some examples, microprojectiles for a particle gun method are prepared by associating DNA construct(s) with the microprojectiles in the presence of Tfx-10, Tfx-20, Tfx-50, Lipofectin, Lipofectamine, Cellfectin, Effectene, Cytofectin GSV, Perfect Lipids, DOTAP, DMRIE-C, FuGENE-6, Superfect, Polyfect, polyethyleneimine, chitosan, protamine Cl, DNA binding proteins, histone H1, histone CENH3, poly-L lysine, DMSA, and the like.

The polynucleotide may be introduced into plants by contacting plants with a virus, or viral nucleic acids. Generally, such methods involve incorporating a desired polynucleotide within a viral DNA or RNA molecule. The sequence may initially be synthesized in a viral polyprotein and later processed *in vivo* or *in vitro* to produce a desired protein. Useful promoters encompass promoters utilized for transcription by viral RNA polymerases. Methods for introducing polynucleotides into plants and expressing a protein encoded, involving viral DNA or RNA molecules, are known, see, *e.g.*, U.S. Patents 5,889,191; 5,889,190; 5,866,785; 5,589,367; 5,316,931; and Porta et al. (1996) Mol Biotech 5:209-221.

Various components, including those from a site-specific recombination system, can be provided to a plant using a variety of transient methods. Such transient transformation methods include, but are not limited to, the introduction of the recombinase or active fragment or variant thereof directly, introduction of the recombinase mRNA, or using a non-integrative method, or introducing low levels of DNA into the plant. Such methods include, for example, microinjection, particle bombardment, viral vector systems, and/or precipitation of the polynucleotide wherein transcription occurs from the particle-bound DNA without substantive release from the particle or integration into the genome, such methods generally use particles coated with polyethylimine, (see, *e.g.*, Crossway et al. (1986) Mol Gen Genet 202:179-185; Nomura et al. (1986) Plant Sci 44:53-58; Hepler et al. (1994) Proc Natl Acad Sci USA 91:2176-2180; and Hush et al. (1994) J Cell Sci 107:775-784).

The transformed cells may be regenerated into plants using standard protocols and media, see *e.g.*, McCormick et al. (1986) Plant Cell Rep 5:81-84. These plants may then be grown and self-pollinated, backcrossed, and/or outcrossed, and the resulting progeny having the desired characteristic identified. Two or more generations may be grown to ensure that the characteristic is stably maintained and inherited and then seeds harvested. In this manner transformed/transgenic seed having the recited DNA construct stably incorporated into their genome are provided. A plant and/or a seed having stably incorporated the DNA construct can be further characterized for expression, site-specific integration potential, agronomics, and copy number (see, *e.g.*, U.S. Patent 6,187,994).

Fragments and variants of recombination sites, recombinases, selection markers, and nucleotide sequences of interest can be used, and unless otherwise stated, indicate that the variant or fragment retains at least some of the activity/function of the original composition. In instances where the polynucleotide encodes a protein, a fragment of a polynucleotide may encode protein fragments that retain the biological activity of the full-length protein. Fragments of a polynucleotide may range from at least about 20 nucleotides, about 50 nucleotides, about 100 nucleotides, and up to the full-length polynucleotide. A fragment of a polynucleotide that encodes a biologically active portion of a protein typically encodes at least 15, 25, 30, 50, 100, 150, 200, 250, 300, 325, 350, 375, 400, 420, or 450 contiguous amino acids, or any integer in this range up to and including the total number of amino acids present in a full-length protein. A biologically active fragment of a polypeptide can be prepared by isolating a portion of one of the polynucleotides encoding the portion of the polypeptide of interest, expressing the protein fragment, and assessing the activity.

Alternatively, a biologically active fragment of a polypeptide can be produced by selectively chemical or proteolytic cleaving of the full-length polypeptide, and the activity measured. For example, polynucleotides that encode fragments of a recombinase polypeptide can comprise nucleotide sequence comprising at least 16, 20, 50, 75, 100, 150, 200, 250, 300, 350, 400, 450, 500, 550, 600, 650, 700, 800, 900, 1,000, 1,100, 1,200, 1,300, or 1,400 nucleotides, or any integer in this range up to and including the total number of nucleotides of a full-length polynucleotide. In addition, fragments of a recombination site retain the biological activity of the recombination site, undergoing a recombination event in the presence of the appropriate recombinase. Fragments of a recombination site may range from at least about 5, 10, 15, 20, 25, 30, 35, 40 nucleotides, up to the full-length of a recombination site. For example, a full-length FRT, lox, attB, and attP sites are known and range from about 50 nucleotides to about 250 nucleotides, and fully active minimal are known and range from about 20, 25, 30, 35, 40, 45, and 50 nucleotides.

Assays to measure the biological activity of recombination sites and recombinases are known (see, *e.g.*, Senecoll et al. (1988) J Mol Biol 201:406-421; Voziyanov et al. (2002) Nucleic Acids Res 30:7; U.S. Patent 6,187,994; WO01/00158; Albert et al. (1995) Plant J 7:649-659; Hartang et al. (1998) J Biol Chem 273:22884-22891; Saxena et al. (1997) Biochim Biophy Acta 1340:187-204; and Hartley et al. (1980) Nature 280-860-864). Assays for recombinase activity generally measure the overall activity of the enzyme on DNA substrates containing recombination sites. For example, to assay for FLP activity, inversion of a DNA sequence in a circular plasmid containing two inverted FRT sites can be detected as a change in position of restriction enzyme sites (see, *e.g*., Vetter et al. (1983) Proc Natl Acad Sci USA 80:7284). Alternatively, excision of DNA from a linear molecule or intermolecular recombination frequency induced by the enzyme may be assayed (see, *e.g.*, Babineau et al. (1985) J Biol Chem 260:12313; Meyer-Leon et al. (1987) Nucleic Acids Res 15:6469; and Gronostajski et al. (1985) J Biol Chem 260:12328). Recombinase activity may also be measured by excision of a sequence flanked by recombinogenic FRT sites to activate an assayable marker gene.

### EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only.

The meaning of abbreviations is as follows: "see" means second(s), "min" means minute(s), "h" means hour(s), "d" means day(s), 'µl" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kB" means kilobase(s).

### EXAMPLE 1. Identification and isolation of centromeres from maize

To evaluate the size, composition, and structural organization of individual centromeres, labeled probes specific to a CentC, CentA, CRM1, and/or CRM2, were used individually and/or in a cocktail for fluorescent *in situ* hybridization (FISH) on maize meiotic pachytene, metaphase, anaphase I chromosomes and to extended DNA molecules (fiber-FISH). These four probes were also used for screening genomic maize BAC libraries.

### A. In situ hybridization

Multi-color FISH to maize metaphase chromosomes reveals that these four centromeric repeats are centromere-specific and co-localized in centromeric regions on all chromosomes in somatic cells. FISH analysis showed that the retrotransposons CRM1, CRM2, and CentA, occupy approximately the same region in maize centromeres. There is significant variation in repeat composition and relative size of repeat regions between centromeres of different maize chromosomes.

FISH results showed that the CentA probe had the weakest hybridization signal; the CRM1 probe showed a gradient-like hybridization pattern with the strongest signal around the primary constriction of the metaphase chromosome, with the signal gradually fading at the periphery of the centromere regions, and the CRM2 probe showed the most clear and compact hybridization signal. The strength of the FISH signal of the CentC repeats was highly dependent on the CentC copy number, which is variable between centromeres of different maize chromosomes. In some centromeres CentC is tightly clustered, showing slight overlap with the other centromeric repeats, in other chromosomes CentC repeat distribution shows more overlap with all other repeats. FISH of meiotic anaphase I chromosomes in microsporocytes with all four centromeric repeats revealed that the centromeric region at this stage is highly extended and only a small segment of the entire centromere region is actually attached to the kinetochore. All four repeats co-localized at the microtubule attachment segment, suggesting that a native functional centromere region comprises of all four centromeric repeats. Fiber-FISH on the extended DNA molecules was used to further characterize the distribution and arrangement of centromeric repeats at a higher resolution.

Oat by maize crosses generated F1 embryos that retained one or more maize chromosomes (see, *e.g.*, Riera-Lizarazu et al. (1996) Theor Appl Genet 93:123-135; Ananiev et al. (1997) Proc Natl Acad Sci USA 94:3524-3529). These lines provide a means to study individual maize chromosomes without the background complexities of the other nine maize chromosomes. A number of oat-maize addition lines are available from Ron Phillips at University of Minnesota (St. Paul, MN, USA), including Seneca 60, A188, and B73 oat-maize addition lines used herein.

DNA from oat-maize chromosome addition lines were used for analysis of centromeric regions from individual maize chromosomes. Multicolor fiber-FISH on oat-maize chromosome addition lines revealed megabase-long hybridization stretches of centromeric repeats unique for each chromosome (Figure 11). In chromosomes 1, 7, and 8 all four repeats were interspersed along the entire Centromeric region. In other chromosomes, CentC was present as relatively short stretches (about 300 kb) flanked by "loose" arrays of the other three centromeric repeats. The overall length of the centromeric regions varied greatly between different maize chromosomes as observed by FISH. CentC revealed significant polymorphism between centromeres of individual chromosomes in the abundance of this repeat, with a difference of as much as 10 fold observed within any given genotype. Chromosome 7 had the largest blocks of CentC tandem repeats in metaphase and pachytene chromosomes. Similarly the oat-maize addition line with maize chromosome 7 had the longest stretches of DNA fibers which hybridize to CentC probe. Conversely, the centromere of maize chromosome 4 had the smallest block of CentC repeats in metaphase chromosomes and the smallest tracts of CentC in oat-maize chromosome 4 addition lines, especially in maize line B73 chromosome 4. When analyzed by fiber-FISH the centromeric retrotransposons CentA, CRM1, and CRM2 showed a dotted-like pattern with large gaps between positive hybridization signals. When probes to these three retrotransposons were mixed together and used as one cocktail probe they revealed more contiguously labeled DNA fibers interspersed with blocks of CentC repeats. The flanks of contiguously labeled centromeric retrotransposons showed a dotted-like pattern along the DNA molecules indicated that centromeric retrotransposons were interspersed with other types of DNA sequences, including non-centromere specific elements. The centromeric retrotransposons can form loose arrays up to 1 Mb in centromeres of chromosomes with small blocks of CentC repeats, such as chromosome 4. The maize hybrid Zapalote chico has a supernumary B-chromosome. FISH of Zapalote chico meiotic chromosomes indicated that the functional centromere of the maize B-chromosome contains all four centromeric repeats, similar to that observed in all the A-chromosomes. However, clusters of CentC repeats can be found also in several non-centromeric sites on the long arm of the B-chromosome. Those sites are apparently free from other centromeric repeats.

The results of FISH on mitotic and meiotic chromosomes, and fiber FISH suggested that the functional native centromeric segment responsible for the formation of the kinetochore on a maize chromosome generally comprises arrays of CentC tandem repeats intermixed with three other centromeric repeats, CRM1, CRM2 and CentA (Figure 12).

### B. BAC Libraries

BAC vectors allow the cloning of large fragments of genomic DNA, up to about 300 kb in size, which can be maintained in a bacterial host, typically *E. coli.* A wide variety of BAC libraries have been generated from plant and animal species and made publicly available, see, for example the information at Clemson University Genome Institute (CUGI; see website at genome.clemson.edu) and Children's Hospital Oakland Research Institiute (CHORI; see website at chori.org). Maize genomic BAC libraries representing greater than 13X coverage using multiple enzymes for library construction from two diverse maize genotypes, B73 and Mo17, representing the Dent and Lancaster heterotic groups respectively, were screened for maize centromeric sequences.

### i. Maize Mo17 genomic BAC library

The plndigoBac536 (Shizuya, unpublished) and pBeloBAC11 (Kim et al. (1996) Genomics 34:213-218) BAC cloning vectors were developed from pBAC108L (Shizuya et al. (1992) Proc Natl Acad Sci USA 89:8794-8797). The pBAC108L is a mini-F factor based plasmid. The F factor codes for genes that regulate its own replication and copy number in the cell. Vector pBeloBAC11 was generated by introducing the LacZ gene to facilitate recombinant clone identification by blue or colorless (white) phenotypes. pBeloBAC11 has three unique cloning sites: BamHI, *Sph*I*,* and *Hind*III, which are flanked by the T7 and SP6 promoters. The rare-cutter restriction sites *Not*I, *Eag*I, *Xma*I, *Sma*I, *Bg*/I, and *Sfi*I can be used to excise the insert from pBeloBAC11. In vector pIndigoBac536, an *Eco*RI site has been modified in the chloramphenicol (CM^{R}) gene so that the *Eco*RI site in the cloning site can be used for library construction. The pBeloBAC11 and pIndigoBac536 vectors have two selection markers, LacZ and CM^{R} for transformant selection.

A proprietary maize genomic BAC library from maize Mo17 public inbred line was constructed in pBeloBAC11 or plndigoBac536 essentially as described in Kim et al. ((1996) Genomics 34:213-218) under contract with the Shizuya laboratory at the California Institute of Technology. Briefly, Mo17 genomic DNA was partially digested with *Hind*III or *Eco*RI restriction enzymes. The DNA fragments were size fractionated in agarose gel and cloned in pBeloBAC11 *Hind*III sites or pIndigoBac536 *Eco*RI sites. The average insert size was about 150 kb. The entire Mo17 genomic BAC library consists of 433 384-well plates or 166,272 total BAC clones. The first half of the library comprising 214 plates contains BAC clones with *Hind*III inserts, while the second half of the library comprising 219 plates, contains BAC clones with *Eco*RI inserts. The BAC clones are maintained in *E. coli* DH10B (BRL Life Technologies).

### ii. Maize B73 genomic BAC libraries

Two public maize B73 genomic BAC libraries were obtained. Library ZMMBBb is available from Clemson University Genome Institute (CUGI, University of Georgia, Athens, GA, USA). The ZMMBBb BAC library was created at CUGI by cloning *Hind*III partially digested maize B73 genomic DNA into the pIndigoBac536 vector comprising a chloramphenicol (CM^{R}) resistance gene. The ZMMBBb BAC library comprises 247,680 total BAC clones with an average insert size of about 137 kb, representing a 14X genomic coverage. The second B73 BAC library, CHORI-201 (ZMMBBc) created by Pieter de Jong's laboratory at Children's Hospital Oakland Research Institiute (CHORI), is available from the BACPAC Resource Center at CHORI. To construct this library, genomic DNA was isolated from maize B73 nuclei. The first segment of the library was constructed using DNA partially digested with a combination of *Eco*RI and EcoRI methylase, the second segment was constructed using *Mbo*I partially digested DNA. Size selected DNA was cloned into the pTARBAC2.1 vector (segment 1, plates 1-288) between the *Eco*RI sites and into the pTARBAC1.3 vector (segment 2, plates 289-576) between the *Bam*HI sites. The ligation products were transformed into *E*. *coli* DH10B electrocompetent cells (BRL Life Technologies). The BAC clones for each library segment in each vector have been arrayed into 288 384-well microtiter dishes. Segment 1 comprises 106,637 individual BAC clones with an average insert size of 163 kb, representing a 6.9X genomic coverage. Segment 2 comprises 105,579 individual BAC clones with an average insert size of 167 kb, representing a 7.0X genomic coverage. The total ZMMBBc library comprises 212,216 individual BAC clones with an average insert size of 165 kb, representing a 13.9X genomic coverage.

### C. BAC library screening

Maize B73 and Mo17 BAC libraries were screened with four separate probes to centromeric sequences CentA, CentC, CRM1, and CRM2. The probes were designed as OVERGO oligonucleotides 40 bp long and were unique to each centromere element. By using appropriate labels, these probes can be used for colony, and blot hybridization, and FISH and fiber-FISH.

### i. Overgo probes

Overgo probes are typically designed as two short oligonucleotides which have an 8 bp complementary overlapping region. The short oligonucleotides are typically in the range of 23-28 bp, with 24 bp being most commonly used. After annealing, the oligonucleotides form dimers with 16 bp single-stranded DNA on both sides. The partially double-stranded probe is labeled by filling the recessed 3' termini using polymerization activity of the Klenow enzyme in the presence of labeled nucleotides. The final overgo probe comprises a labeled double-stranded 40 bp probe. TABLE 1 lists primers and probes used for generating, screening, and characterization of BAC clones, DNA constructs, and maize minichromosome events.

**TABLE 1**

| **SEQ ID** | **Biocode** | **Oligo Name** | **Sequence** |
|---|---|---|---|
| 5 | | PCR-Telomere-F | AGGGTTTAGGGTTTAGGGTTTAGGGTTTAGGG |
| 6 | | PCR-Telomere-R | CCCTAAACCCTAAACCCTAAACCCTAAACC |
| 7 | 65644 | CentC-OVG-1-40f | GGTTCCGGTGGCAAAAACTCGTGC |
| 8 | 65645 | CentC-OVG-1-40r | TGTCGGTGCATACAAAGCACGAGT |
| 9 | 65646 | CentC-OVG-51-90f | GAATGGGTGACGTGCGACAACGAA |
| 10 | 65647 | CentC-OVG-51-90r | GGTGGTTTCTCGCAATTTCGTTGT |
| 11 | 65648 | CentC-OVG-101-140f | GTTTTGGACCTAAAGTAGTGGATT |
| 12 | 104790 | CentC-OVG-101-140r | CACAACGAACATGCCCAATCCACT |
| 13 | 69509 | CRM1-LTR-OVG1f | CTTGGTCTTGGACAGTACCTCACT |
| 14 | 69510 | CRM1-LTR-OVG2f | CCCTTGCGATCCGACTACGACGAG |
| 15 | 69511 | CRM1-LTR-OVG3f | TCACGAAGATCGTTTCCTGTGCGC |
| 16 | 69512 | CRM1-LTR-OVG4f | CAGCGCAGATTAGCGCGTGTTCGA |
| 17 | 69513 | CRM1-LTR-OVG5f | CCAACCCTAGGTCGTCCATTATGG |
| 18 | 69514 | CRM1-LTR-OVG6f | TTCAATTCTCTTGCACGGGCCCGA |
| 19 | 69515 | CRM1-LTR-OVG1r | TCAGGTCTACTTCATCAGTGAGGT |
| 20 | 69516 | CRM1-LTR-OVG2r | TGGCGCCTCGGGCTTGCTCGTCGT |
| 21 | 69517 | CRM1-LTR-OVG3r | TGTTCGTTCTTCGATTGCGCACAG |
| 22 | 69518 | CRM1-LTR-OVG4r | TTAGCCTTAGCTACTCTCGAACAC |
| 23 | 69519 | CRM1-LTR-OVG5r | CCAGCCCAATTGCGGCCCATAATG |
| 24 | 69520 | CRM1-LTR-OVG6r | CACCTGGGCCAGTGACTCGGGCCC |
| 25 | 69521 | CRM2-LTR-OVG1f | TGATGAAGACATCCACACTACTGA |
| 26 | 69522 | CRM2-LTR-OVG2f | TTGAACATGCTGGATTCGGACTGC |
| 27 | 69523 | CRM2-LTR-OVG3f | CTGCCCATGGTGCTGCGTCACCCT |
| 28 | 69524 | CRM2-LTR-OVG4f | GCGCGTGCTAGTTCAGCCGCCCGT |
| 29 | 69525 | CRM2-LTR-OVG5f | GTATCGGTTGCTAAGGCGCAGCGT |
| 30 | 69526 | CRM2-LTR-OVG1r | TATTGGTATAGATGCATCAGTAGT |
| 31 | 69527 | CRM2-LTR-OVG2r | AAGTTGGTGTTCTTCTGCAGTCCG |
| 32 | 69528 | CRM2-LTR-OVG3r | CCCATTGGGCCAAAATAGGGTGACG |
| 33 | 69529 | CRM2-LTR-OVG4r | TTCCGAAGACAAGAAGACGGGCGG |
| 34 | 69530 | CRM2-LTR-OVG5r | CTACAGCCTTCCAAAGACGCTGCG |
| 35 | 69531 | CentA-LTR-OVG1f | TGATGAGAACATAACCCGCACAGA |
| 36 | 69532 | CentA-LTR-OVG2f | AGGATGATGAGGACATCACTGCCA |
| 37 | 69533 | CentA-LTR-OVG3f | AACCATCTAGAATTTGAGAAGGCA |
| 38 | 69534 | CentA-LTR-OVG4f | GTCCAGAAACTGCCGAGTGAACTC |
| 39 | 65535 | CentA-LTR-OVG5f | GAGAGAGTTTCGTTCTCCATTAGA |
| 40 | 69536 | CentA-LTR-OVG6f | GTTCTTGCTTGTTCTCGATTGCTT |
| 41 | 69537 | CentA-LTR-OVG7f | TTGGTTGTGGTAGTCGGGCAGCCA |
| 42 | 69538 | CentA-LTR-OVG1r | CATTAACATGGTCATATCTGTGCG |
| 43 | 69539 | CentA-LTR-OVG2r | TGGTGTGGTGTATTGATGGCAGTG |
| 44 | 69540 | CentA-LTR-OVG3r | CTTTTATTGCCTTGTTGCCTTCT |
| 45 | 69541 | CentA-LTR-OVG4r | GACTTGGGTAGAGCAGGAGTTCAC |
| 46 | 69542 | CentA-LTR-OVG5r | AGGAATAGAAAGGAGTTCTAATGG |
| 47 | 69543 | CentA-LTR-OVG6r | ACAGCCTTGAACCTGCAAGCAATC |
| 48 | 69544 | CentA-LTR-OVG7r | TGTTGGAGAACGACGTTGGCTGCC |
| 49 | 69555 | Cent4-250-OVG1f | TAAGTGCAAACCATTGTTAAATTT |
| 50 | 69556 | Cent4-250-OVG2f | CACAAACCCTTAACTCGAAACTAT |
| 51 | 69557 | Cent4-250-OVG3f | ATCGAAAGATAACTCATATGGCTT |
| 52 | 69558 | Cent4-250-OVG4f | TCCACTAAAGAACCAAGATTGTGA |
| 53 | 69559 | Cent4-250-OVG1r | AATTGTACTATCTCTAAAATTTAA |
| 54 | 69560 | Cent4-250-OVG2r | TTTAGGGTTTGGGGTTATAGTTTC |
| 55 | 69561 | Cent4-250-OVG3r | GACCATAATGGTCAAAAAGCCATA |
| 56 | 69562 | Cent4-250-OVG4r | ATATGTTGGACACAAATCACAATC |
| 57 | 69634 | 18-26SrDNANTS-OvG1f | CCGGAAATAAGCAAAGTCCAAGCG |
| 58 | 69635 | 18-26SrDNANTS-OvG2f | TATGTCTTGGGTGAAGGGCATGGC |
| 59 | 69636 | 18-26SrDNANTS-OvG3f | CGCAAGGCGACGGGCGGCATGGCT |
| 60 | 69637 | 18-26SrDNANTS-OvG4f | CGAGGGGTTCCCCATGGCGCACGG |
| 61 | 69638 | 18-26SrDNANTS-OvG1r | TCGGTGTCTTTCCACACGCTTGGA |
| 62 | 69639 | 18-26SrDNANTS-OvG2r | GTTTTCCCTCCGTTCCGCCATGCC |
| 63 | 69640 | 18-26SrDNANTS-OvG3r | AGACGCAAGGCCGAACAGCCATGC |
| 64 | 69641 | 18-26SrDNANTS-OvG4r | GGCCTCAGTTTTCGGCCCGTGCGC |
| 65 | 74794 | subtelo-TR430-OvG2f | GACACATGTTTTTGTCGTCGAACA |
| 66 | 74795 | subtelo-TR430-OvG2r | GGAGGCACGAAATCGCTGTTCGAC |
| 67 | 74796 | subtelo-TR430-OvG3f | CGACCGCCACCCATGATTTGACCA |
| 68 | 74797 | subtelo-TR430-OvG3r | ACCTTACCAGTCTCTATGGTCAAA |
| 69 | 74799 | subtelo-TR430-OvG4f | TCCCGTGAGCTATAGCACACGTTT |
| 70 | 74800 | subtelo-TR430-OvG4r | GGTCGCTCGGCCATGAAAACGTGT |
| 71 | 74801 | subtelo-TR430-OvG5f | CCGTGTTCCTCCACACGTGTTTTT |
| 72 | 74802 | subtelo-TR430-OvG5r | AAGGTGCTCCGGGGACAAAAACAC |
| 73 | 74803 | subtelo-TR430-OvG6f | TTGGCCTCCCGCGAGCTATATCAC |
| 74 | 74804 | subtelo-TR430-OvG6r | TTGGCCACGGAAATGTGTGATATA |
| 75 | 74805 | subtelo-TR430-OvG7f | TTATGTATCCGACCTGCCACCTTC |
| 76 | 74806 | subtelo-TR430-OvG7r | CTCCCCGGTCTAAAACGAAGGTGG |
| 77 | 74807 | subtelo-TR430-OvG8f | GCCACCCGTGAGCTATAGCACACG |
| 78 | 74808 | subtelo-TR430-OvG8r | TAGGTTTCCATAAAATCGTGTGCT |
| 79 | 65650 | 180knobOvG21-60f | TGTCGAAAATAGCCATGAACGACC |
| 80 | 65651 | 180knobOvG21-60r | CGGTATTATTGGAAATGGTCGTTC |
| 81 | 65652 | 180knobOvG71-110f | CCTACGGATTTTTGACCAAGAAAT |
| 82 | 65653 | 180knobOvG71-110r | ATTTCTAGTGGAGACCATTTCTTG |
| 83 | 65654 | 180knobOvG141-180f | ATGTGGGGTGAGGTGTATGAGCCT |
| 84 | 65655 | 180knobOvG141-180r | ATGAGCCTCTGGTCGATGATCAAT |
| 85 | 65656 | 5SrDNAOvG1-40f | GGATGCGATCATACCAGCACTAAA |
| 86 | 65657 | 5SrDNAOvG1-40r | TGATGGGATCCGGTGCTTTAGTGC |
| 87 | 65658 | 5SrDNAOvG61-100f | CTTGGGCGAGAGTAGTACTAGGAT |
| 88 | 65659 | 5SrDNAOvG61-100r | TCCCAGGAGGTCACCCATCCTAGT |
| 89 | 65660 | 5SrDNAOvG161-200f | ACCATAGTAAAAATGGGTGACCGT |
| 90 | 65661 | 5SrDNAOvG161-200r | TAATTTAACACGAGAACGGTCAC |
| 91 | 65662 | 5SrDNAOvG261-230f | CCGTGGGCGAGCCGAGCACGGAGG |
| 92 | 65663 | 5SrDNAOvG261-230r | TCCTCTTATGCCCACACCTCCGTG |
| 93 | 65664 | 350knobOvG31-70f | CTCAAATGACGTTTCTATGATATT |
| 94 | 65665 | 350knobOvG31-70r | TGAATACAATGCCCTCAATATCAT |
| 95 | 65666 | 350knobOvG121-160f | CTAGGTTTCCTATAATCCCCTCTA |
| 96 | 65667 | 350knobOvG121-160r | CTAGGTATGCCTTGAATAGAGGG |
| 97 | 65668 | 350knobOvG161-200f | ATGTTGTTTATGTCCACTCAAGTA |
| 98 | 65669 | 350knobOvG161-200r | ATGGTGTACGGTGTTTTACTTGAG |
| 99 | 65670 | 350knobOvG261-300f | GTGAGATCTGTCCAAACATAGGTT |
| 100 | 65671 | 350knobOvG261-300r | GGTGCCTTACAACCGTAACCTATG |
| 101 | | b010.m7 fis31 | |
| 102 | | b108.h15 fis47 | |
| 103 | | Cen3n.pk0001.g11 | |
| 104 | 103022 | 23715-3101-3200f | CCAGGTAGTTTGAAACAGTATTCT |
| 105 | 103023 | 23715-3501-3600f | ATAAAGGAAAAGGGCAAACCAAAC |
| 106 | 103024 | 23715-1401-1500f | GATGCCCACATTATAGTGATTAGC |
| 107 | 103025 | 23715-2901-3000f | CCACATATAGCTGCTGCATATGCC |
| 108 | 103026 | 23715-3701-3800f | CGGATCTAACACAAACATGAACAG |
| 109 | 103027 | 23715-1-100f | CGATGAATTTTCTCGGGTGTTCTC |
| 110 | 103028 | 23715-101-200f | CCTGCAGCCCTAATAATTCAGAAG |
| 111 | 103029 | 23715-301-400f | CACAGTCGATGAATCCAGAAAAGC |
| 112 | 103030 | 23715-901-1000f | GCGTGCAATCCATCTTGTTCAATC |
| 113 | 103031 | 23715-3201-3300f | CAACCACACCACATCATCACAACC |
| 114 | 103032 | 23715-3601-3700f | ACTGGCAAGTTAGCAATCAGAACG |
| 115 | 103033 | 23715-4901-5000f | CATGAACGTGTCTTCAACTAGAGG |
| 116 | 103034 | 23715-4201-4300f | GACGGCGTTTAACAGGCTGGCATT |
| 117 | 103035 | 23715-201-300f | CCAAGCTCTTCAGCAATATCACGG |
| 118 | 103036 | 23715-601-700f | ATACTTTCTCGGCAGGAGCAAGGT |
| 119 | 103037 | 23715-1001-1100f | ATCCTTGGCGGCAAGAAAGCCATC |
| 120 | 103038 | 23715-1101-1200f | GCAAGCTACCTGCTTTCTCTTTGC |
| 121 | 103039 | 23715-1601-1700f | GCTTCTTGGCCATGTAGATGGACT |
| 122 | 103040 | 23715-1801-1900f | TTCACGCCGATGAACTTCACCTTG |
| 123 | 103041 | 23715-5001-5087f | AAGCTTGCCAACGACTACGCACTA |
| 124 | 103042 | 23715-401-500f | CCCTGATGCTCTTCGTCCAGATCA |
| 125 | 103043 | 23715-801-900f | AGAGCAGCCGATTGTCTGTTGTGC |
| 126 | 103044 | 23715-1301-1400f | CAGGATCCCGTAACTATAACGGTC |
| 127 | 103045 | 23715-2801-2900f | CGACCTGCAGAAGTAACACCAAAC |
| 128 | 103046 | 23715-3401-3500f | ATCTAGAACGACCGCCCAACCAGA |
| 129 | 103047 | 23715-3801-3900f | ATTTGGGGGAGATCTGGTTGTGTG |
| 130 | 103048 | 23715-3901-4000f | GAGGGGGTGTCTATTTATTACGGC |
| 131 | 103049 | 23715-4801-4900f | CATGCAAGCTGATCTGAGCTTGGC |
| 132 | 103050 | 23715-2101-2200f | TCCATGCGCACCTTGAAGCGCATG |
| 133 | 103051 | 23715-501-600f | TTCCATCCGAGTACGTGCTCGCTC |
| 134 | 103052 | 23715-1201-1300f | ATCCACTAGTAACGGCCGCCAGTG |
| 135 | 103053 | 23715-4001-4100f | GCCACGCAATTTCTGGATGCCGAC |
| 136 | 103054 | 23715-701-800f | CGATAGCCGCGCTGCCTCGTCTTG |
| 137 | 103055 | 23715-1901-2000f | CACTTGAAGCCCTCGGGGAAGGAC |
| 138 | 103056 | 23715-1701-1800f | TCCTTCAGCTTCAGGGCCTTGTGG |
| 139 | 103057 | 23715-2001-2100f | CACCTTGGAGCCGTACTGGAACTG |
| 140 | 103058 | 23715-2601-2700f | TGCGGCTCGGTGCGGAAGTTCACG |
| 141 | 103059 | 23715-4101-4200f | ACGCGACGCTGCTGGTTCGCTGGT |
| 142 | 103060 | 23715-3101-3200r | CGTTCTAGATCGGAGTAGAATACT |
| 143 | 103061 | 23715-3501-3600r | TGTTTCGTTGCATAGGGTTTGGTT |
| 144 | 33332 | 23715-1401-1500r | GCACACATAGTGACATGCTAATCA |
| 145 | 103062 | 23715-2901-3000r | GATATACTTGGATGATGGCATATG |
| 146 | 103063 | 23715-3701-3800r | CCCGGTAGTTCTACTTCTGTTCAT |
| 147 | 103064 | 23715-1-100r | ATTCGAGCCAATATGCGAGAACAC |
| 148 | 103065 | 23715-101-200r | GCCTTCTTGACGAGTTCTTCTGAA |
| 149 | 103066 | 23715-301-400r | ATGGTGGAAAATGGCCGCTTTTCT |
| 150 | 103067 | 23715-901-1000r | GAGGATCGTTTCGCATGATTGAAC |
| 151 | 103068 | 23715-3201-3300r | TGCTTTTTGTTCGCTTGGTTGTGA |
| 152 | 103069 | 23715-3601-3700r | ACCTGTACGTCAGACACGTTCTGA |
| 153 | 103070 | 23715-4901-5000r | AATTAAGTCAGGCGCGCCTCTAGT |
| 154 | 103071 | 23715-4201-4300r | CTTGTTTCGAGTAGATAATGCCAG |
| 155 | 103072 | 23715-201-300r | ACATAGCGTTGGCTACCCGTGATA |
| 156 | 103073 | 23715-601-700r | GATCTCCTGTCATCTCACCTTGCT |
| 157 | 103074 | 23715-1001-1100r | CCTGCAAAGTAAACTGGATGGCTT |
| 158 | 103075 | 23715-1101-1200r | AAGGGAAAACGCAAGCGCAAAGAG |
| 159 | 103076 | 23715-1601-1700r | TACCTGGTGGAGTTCAAGTCCATC |
| 160 | 103077 | 23715-1801-1900r | ACGGCTGCTTCATCTACAAGGTGA |
| 161 | 103078 | 23715-5001-5087r | TGAAGCTCTTGTTGGCTAGTGCGT |
| 162 | 103079 | 23715-401-500r | GTCTTGTCGATCAGGATGATCTGG |
| 163 | 103080 | 23715-801-900r | ATTCGGCTATGACTGGGCACAACA |
| 164 | 103081 | 23715-1301-1400r | CGCTTCGCTACCTTAGGACCGTTA |
| 165 | 103082 | 23715-2801-2900r | CGATGCTCACCCTGTTGTTTGGTG |
| 166 | 88245 | 23715-3401-3500r | GGTTGTGATGATGTGGTCTGGTTG |
| 167 | 103083 | 23715-3801-3900r | GTTCGGAGCGCACACACACACAAC |
| 168 | 103084 | 23715-3901-4000r | TTTCCCTTCCTCGCCCGCCGTAAT |
| 169 | 103085 | 23715-4801-4900r | TAAAACGACGGCCAGTGCCAAGCT |
| 170 | 103086 | 23715-2101-2200r | ACGTCATCACCGAGTTCATGCGCT |
| 171 | 103087 | 23715-501-600r | AGCGAAACATCGCATCGAGCGAGC |
| 172 | 103088 | 23715-1201-1300r | AAGCCGAATTCCAGCACACTGGCG |
| 173 | 103089 | 23715-4001-4100r | TTGGACTTGCTCCGCTGTCGGCAT |
| 174 | 103090 | 23715-701-800r | TGCCCTGAATGAACTGCAAGACGA |
| 175 | 103091 | 23715-1901-2000r | CCGACTACAAGAAGCTGTCCTTCC |
| 176 | 103092 | 23715-1701-1800r | TGCTGAAGGGCGAGACCCACAAGG |
| 177 | 103093 | 23715-2001-2100r | GGACATCCTGTCCCCCCAGTTCCA |
| 178 | 103094 | 23715-2601-2700r | ACATCGAGACCTCCACCGTGAACT |
| 179 | 103095 | 23715-4101-4200r | AGTCTAACGGACACCAACCAGCGA |
| 180 | | PCRbacmpk108h15f | GATCGTCGAATGGGAATCCATGGG |
| 181 | | PCRbacmpk108h15r | CCCTGAGTGAACCATTTAGGAAGATCAG |
| 182 | | PCRbacmpk108h15-2.fis47f | TGCAACATCCAAAGACCCAACATG |
| 183 | | PCRbacmpk108h15-2.fis47r | TTCCAACATGGTTGGTGGTCAG |
| 184 | | PCRbacmpk010m07fis3 1f | TGTCATGACATCTTGTTGCTACCCTG |
| 185 | | PCRbacmpk010m07fis3 1r | AAACCCGGAGTTTCTATGCAGG |
| 192 | 75319 | Telo-31overgo primer1 | AGGGTTTAGGGTTTAGGGTTTAGGGTTTAGGG |
| 193 | 39612 | Telo-31overgo primer2 | CCCTAAACCCTAAACCCTAAACCCTAAACCC |

### ii. BAC library screening results

Colony hybridization screening identified a pool of approximately 8000 BAC clones which hybridized to at least one of the four centromere-specific probes. The 8000 BAC clones were classified into 4 groups based on their hybridization profile (Table 2).

**TABLE 2**

| **Group** | **Total** |
|---|---|
| All BACs containing CentA | 842 |
| All BACs containing CentC | 2479 |
| All BACs containing CRM2 | 2968 |
| All BACs containing CRM1 | 6012 |

Based on centromeric repeat composition the BAC clones were further classified into 15 sets based on the combination of probes which hybridized to each particular BAC clone (Table 3).

**TABLE 3**

| **Group** | **# of BACs** |
|---|---|
| BACs containing CentA & CentC & CRM1 & CRM2 | 247 |
| | |
| BACs containing CentA & CentC & CRM2; not CRM1 | 6 |
| BACs containing CentA & CentC & CRM1; not CRM2 | 45 |
| BACs containinq CentA & CRM1 & CRM2; not CentC | 116 |
| BACs containing CentC & CRM1 & CRM2; not CentA | 730 |
| BACs containinq CentA & CentC; not CRM1 not CRM2 | 4 |
| BACs containing CentA & CRM1; not CentC not CRM2 | 131 |
| BACs containinq CentA & CRM2; not CentC not CRM1 | 27 |
| BACs containinq CentC & CRM2; not CentA not CRM1 | 97 |
| BACs containing CentC & CRM1; not CentA not CRM2 | 829 |
| BACs containinq CRM1 & CRM2; not CentA not CentC | 749 |
| | |
| BACs containinq CentC; not CentA not CRM1 not CRM2 | 521 |
| BACs containing CRM2; not CentA not CentC not CRM1 | 966 |
| BACs containinq CRM1; not CentA not CentC not CRM2 | 3165 |
| BACs containing CentA; not CentC not CRM1 not CRM2 | 266 |

The BAC clones were further classified based on the summation of BAC clones which hybridized to each particular probe (Table 4).

**TABLE 4**

| | |
|---|---|
| All BACs containinq CentA | 842 |
| All BACs containinq CentA & CentC | 302 |
| All BACs containinq CentA, CentC, & CRM1 | 292 |
| All BACs containing CentA, CentC, & CRM2 | 253 |
| All BACs containing CentA & CRM1 | 539 |
| All BACs containing CentA, CRM1, & CRM2 | 363 |
| All BACs containing CentA & CRM2 | 396 |
| All BACs containing CentA, CentC, CRM1, & CRM2 | 247 |
| All BACs containinq CentC | 2479 |
| All BACs containing CentC & CRM1 | 1851 |
| All BACs containing CentC & CRM2 | 1080 |
| All BACs containing CentC, CRM1, & CRM2 | 977 |
| All BACs containinq CRM1 | 6012 |
| All BACs containing CRM1 & CRM2 | 1842 |
| All BACs containinq CRM2 | 2968 |

One group of 247 BAC clones contains all four centromeric repeats. They comprise 0.15% of maize genome or can be present on a segment of DNA about 300 kb per centromere on average. This group of BAC clones was identified as the core set to be used first in experiments to construct a maize minichromosome. DNA was purified from all 247 BACs in the core set, digested with *Xmnl* or *Rsa*I, blotted and hybridized with each of the four centromeric repeats. Southern blot hybridization, confirmed that clones in this core set contained all four centromeric repeats. The BACs showed general differences in restriction fragment composition and hybridization patterns, and were further classified into 87 groups on the basis of restriction fragment similarities. One representative from each of the 87 groups (Table 5) was taken to generate core set DNA constructs and/or pools of BAC core set constructs for transformation and minichromosome assembly.

**TABLE 5**

| **No.** | **Name** | **Insert (kb)** | **No.** | **Name** | **Insert (kb)** |
|---|---|---|---|---|---|
| 1 | bacm.pk101.n23 | 50 | 45 | bacm2.pk002.g7 | 125 |
| 2 | bacm2.pk064.e15 | 50 | 46 | bacm.pk135.l7 | 125 |
| 3 | bacm.pk036.e13 | 60 | 47 | bacm.pk090.o5 | 125 |
| 4 | bacm2.pk179.e1 | 70 | 48 | bacm2.pk100.j24 | 130 |
| 5 | bacm.pk030.a6 | 70 | 49 | bacm2.pk013.c9 | 130 |
| 6 | bacm2.pk179.b18 | 75 | 50 | bacm.pk166.n7 | 130 |
| 7 | bacm.pk133.b11 | 75 | 51 | bacm.pk043.o23 | 130 |
| 8 | bacm2.pk066.m12 | 80 | 52 | bacm.pk001.n1 | 130 |
| 9 | bacm.pk119.a23 | 80 | 53 | bacm.pk106.j20 | 135 |
| 10 | bacm.pk098.h2 | 85 | 54 | bacm.pk015.d19 | 135 |
| 11 | bacm2.pk174.e4 | 90 | 55 | bacm.pk007.a2 | 140 |
| 12 | bacm2.pk116.g16 | 90 | 56 | bacm.pk148.e2 | 140 |
| 13 | bacm2.pk023.e24 | 90 | 57 | bacm.pk141.j4 | 140 |
| 14 | bacm.pk178.c10 | 90 | 58 | bacm.pk138.e14 | 140 |
| 15 | bacm.pk135.l6 | 90 | 59 | bacm.pk135.j2 | 140 |
| 16 | bacm.pk098.f3 | 90 | 60 | bacm.pk134.f15 | 140 |
| 17 | bacm.pk075.16 | 90 | 61 | bacm.pk085.k5 | 140 |
| 18 | bacm.pk066.j14 | 95 | 62 | bacm.pk077.b21 | 140 |
| 19 | bacm2.pk099.m24 | 100 | 63 | bacm.pk124.j24 | 145 |
| 20 | bacm2.pk093.h11 | 100 | 64 | bacm.pk023.i5 | 145 |
| 21 | bacm2.pk083.a2 | 100 | 65 | bacm.pk039.m16 | 150 |
| 22 | bacm.pk179.d4 | 100 | 66 | bacm2.pk169.a21 | 150 |
| 23 | bacm.pk076.m3 | 100 | 67 | bacm2.pk130.e20 | 150 |
| 24 | bacm.pk070.h17 | 100 | 68 | bacm.pk156.i17 | 150 |
| 25 | bacm.pk064.n1 | 100 | 69 | bacm.pk143.m18 | 150 |
| 26 | bacm.pk011.l8 | 100 | 70 | bacm.pk112.p1 | 150 |
| 27 | bacm.pk068.p16 | 105 | 71 | bacm.pk102.i4 | 150 |
| 28 | bacm.pk012.n20 | 105 | 72 | bacm.pk087.m4 | 150 |
| 29 | bacm.pk077.k5 | 110 | 73 | bacm.pk079.m11 | 150 |
| 30 | bacm2.pk053.g23 | 110 | 74 | bacm.pk041.e16 | 150 |
| 31 | bacm2.pk034.j8 | 110 | 75 | bacm.pk129.a4 | 150 |
| 32 | bacm.pk164.b11 | 110 | 76 | bacm.pk164.e18 | 155 |
| 33 | bacm.pk062.c14 | 110 | 77 | bacm.pk161.h1 | 155 |
| 34 | bacm.pk013.m8 | 110 | 78 | bacm.pk089.18 | 155 |
| 35 | bacm.pk056.j19 | 110 | 79 | bacm.pk076.o15 | 160 |
| 36 | bacm.pk051.g11 | 115 | 80 | bacm.pk039.a3 | 160 |
| 37 | bacm2.pk179.o14 | 120 | 81 | bacm.pk019.h24 | 160 |
| 38 | bacm2.pk096.d23 | 120 | 82 | bacm2.pk158.f12 | 160 |
| 39 | bacm2.pk070.g7 | 120 | 83 | bacm2.pk075.n6 | 170 |
| 40 | bacm2.pk034.g20 | 120 | 84 | bacm2.pk137.f2 | 175 |
| 41 | bacm2.pk012.g19 | 120 | 85 | bacm.pk093.d8 | 175 |
| 42 | bacm2.pk115.o22 | 125 | 86 | bacm.pk133.b10 | 180 |
| 43 | bacm2.pk094.f14 | 125 | 87 | bacm.pk178.o20 | 180 |
| 44 | bacm2.pk003.g6 | 125 | | | |

### D. Identification of inverted arrays of CentC repeats

BAC libraries from maize lines Mo17 and B73 were searched for inverted CentC tandem arrays. A BLAST search of a Mo17 BAC-end sequence database revealed 591 BAC ends containing CentC repeats. Of these, only 45 BAC clones contained CentC repeats on both ends, and 44 BACs had CentC repeats in the same orientation, with only one BAC having CentC repeats in an inverted orientation (bacm.pk128.j21). A second BAC clone, bacm.pk008.d20 having CentC repeats in an inverted orientation was found by Southern hybridization analysis. The Southern analysis of this clone showed a hybridization pattern very similar to the pattern observed for bacm.pk128.j21. A BLAST search of the public B73 BAC-end sequence database revealed 136 BAC ends containing CentC repeats. Of these, only 5 BAC clones contained CentC repeats on both ends, and 4 BACs had CentC repeats in the same orientation, with only one BAC having CentC repeats in inverted orientation (ZMMBBb0243L15). The DNA of bacm.pk128.j21 and bacm.pk008.d20 were digested with *Xmn*I restriction enzyme, which cleaves CentC repeats into short monomeric or dimeric fragments. A 10 kb *Xmn*I fragment was isolated, subcloned and sequenced. The sequence analysis showed that the CRM1 element (SEQ ID NO: 191) is located between two inverted CentC repeats.

### E. Isolation of centromeric BAC clones from maize chromosome 4

Maize chromosome 4 contains the shortest CentC repeat arrays. These arrays are present in a single stretch of DNA of approximately 300 kb as estimated by fiber-FISH. This segment may contain the core functional centromeric DNA sequences, and could potentially be represented by 2-4 overlapping BAC clones. Chromosome 4-specific centromeric BAC clones can be identified by finding unique DNA sequences located in the chromosome 4 centromeric region.

The maize Mo17 genomic BAC library, comprising 10,965 BAC end sequences was analyzed to identify unique BAC end sequences represented only once in the library. Eighty-one unique BAC end sequences were identified and selected for further characterization. A pair of PCR primers was designed to each of the 81 unique BAC end sequences for mapping on the oat-maize chromosome addition line panel and each unique sequence assigned to an individual maize chromosome.

The BAC end sequence of bacm.pk108.h15 (170 kb) from Mo17 was mapped to chromosome 4. This BAC was sequenced and 6 unique sequences, as well as all four centromeric repeats CentA, CentC, CRM1 and CRM2 were found. Using PCR, this BAC was assigned to a contig containing several BACs which also hybridize to CentC. Sequencing confirmed that two more BAC clones from this contig, bacm.pk010.m7 (170 kb), and bacm.pk184.c21 (150 kb) partially overlap with bacm.pk108.h15 and share some unique markers. Three unique DNA sequences were identified within these three BAC clones and their chromosome 4 localization was confirmed by PCR on oat-maize addition line DNA. Corresponding overgo probes (SEQ ID NOs: 102-104 in Table 1) were developed and used for screening of a B73 public BAC library.

Seven BAC clones from the B73 BAC library were selected based on hybridization to all three chromosome 4 specific probes. DNA from these BAC clones was digested with *Xmn*I*,* transferred to a membrane and hybridized with all four centromeric repeat probes. Four of the selected B73 BAC clones contain CentC, CRM1, and CRM2 centromere repetitive elements: bacb.0424.d20 (150 kb); bacb.0155.h15 (175 kb); bacc.0048.g5 (170 kb); and bacc.0237.m8 (125 kb). Another three B73 BAC clones contain only CRM1 and CRM2 centromere repetitive elements: bacc.0143.i9 (205 kb); bacc.0237.j16 (175 kb); and bacc.0270.c1 (180 kb). Sequencing of the bacb.0155.h15 BAC clone confirmed that it contains significant regions of homology to chromosome 4-specific Mo17 BAC clones bacm.pk010.m7, and bacm.pk108.h15.

Two groups of BAC clones representing the centromeric region of chromosome 4 from the Mo17 and B73 inbred lines were used for the production of DNA constructs for minichromosome assembly.

### F. Isolation and purification of chromosomal centromeric DNA fragments

Essentially all maize genomic DNA is heavily methylated, and this methylation pattern may play a role in the assembly, function, and/or maintenance of maize centromeres. Isolated maize genomic DNA maintaining the methylation and/or other native genomic characteristics, such as size, organization of elements, and other native nucleotide modifications, can be used to generate DNA constructs for maize minichromosome assembly.

### i. Restriction enzyme selection

Sequence analysis of maize centromeric repeats identified a large number of restriction enzymes (six cutters) with no recognition sites within any of the centromeric repeats CentA, CentC, CRM1, or CRM2 (Table 6). These restriction enzymes should digest the bulk of genomic DNA into small DNA fragments, the majority of which being about 1-20 kb in size, while centromeric DNA is expected to be significantly longer. Chromosomal centromeric regions from maize can be isolated by partial or complete digestion of maize high-molecular weight (HMW) genomic DNA with at least one of these restriction enzymes. The fraction of digested HMW genomic DNA comprising large fragments of approximately 50 kb - about 1000 kb can be purified after pulsed field gel electorphoresis (PFGE) of maize nuclei embedded in agarose blocks.

### ii. HMW maize genomic DNA preparation and characterization

HMW maize genomic DNA from Mo17 was prepared essentially as described by Liu & Whittier ((1994) Nucleic Acids Res 22:2168-2169) from DNA embedded in agarose blocks by digestion with various restriction enzymes from TABLE 6 and fractionation by PFGE. Five restriction enzymes, *Bsp*TI, *Aat*II*, Cfr*9I, *Mbi*I, *Mlu*I, were selected for the initial analyses. Of these, *Bsp*TI was selected for all further preparations. Blot-hybridization with labeled CentC centromeric probe revealed that the *Bsp*TI restriction enzyme produced a set of genomic centromeric DNA fragments ranging from about 50 kb to about 600 kb which were well-separated from the rest of the genomic DNA. Hybridization to the same DNA fragments with three other centromeric probes (CentA, CRM1, and CRM2) confirmed that these long DNA fragments comprising all four centromeric repeats have essentially no *Bsp*TI restriction sites. The hybridizing bands may represent individual centromeric DNA fragments that can be isolated and used to generate DNA constructs for minichromosome assembly.

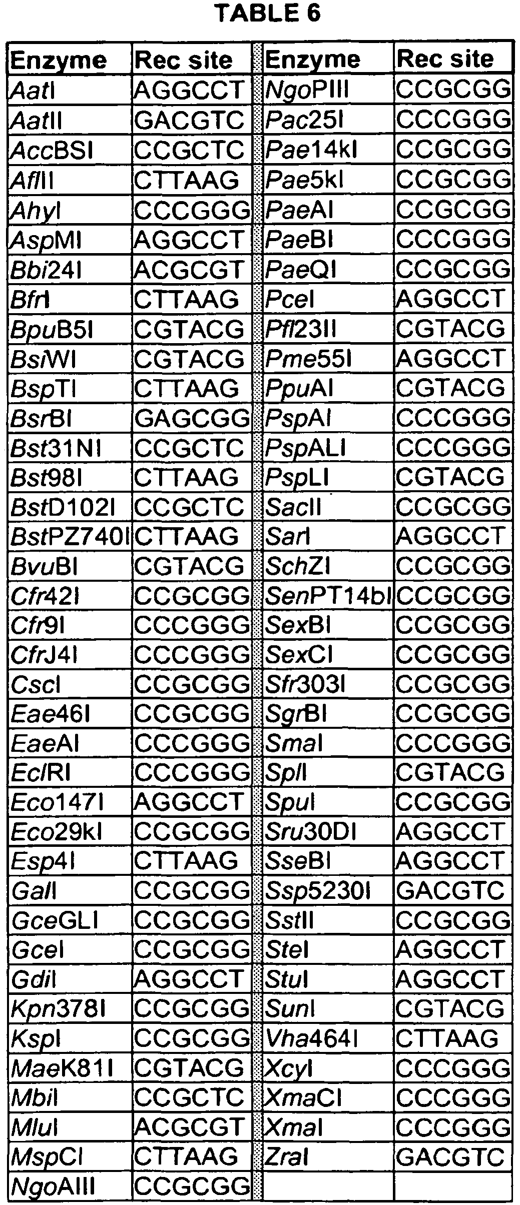

### EXAMPLE 2. Identification and isolation of telomeric sequences

Any functional telomeric region, native, cloned, or synthetic, comprising a telomeric repeat can be used to make the DNA constructs. Several telomere repeats are known, including those from *Tetrahymena, Paramecium, Oxytricha, Euplotes, Dictyostelium, Saccharomyces, Caenorhabditis, Trypanosoma, Leishmania, Physarum, Arabidopsis,* human, and mouse.

| **Telomeric Repeat** | **Exemplary Organism** |
|---|---|
| CCCCAA (C₄A₂) | *Tetrahymena, Paramecium* |
| CCCCAAAA (C₄A₄) | *Oxytricha, Euplotes* |
| CCCTA (C₃TA) | *Trypanosoma, Leishmania, Physarum* |
| C₁₋₃A | *Saccharomyces* |
| C₁₋₈T | *Dictyostelium* |
| CCCTAAA (C₃TA₃) | *Arabidopsis,* human, mouse, *Caenrhabditis* |

### A. Synthetic Telomere sequences

The highly conserved, repetitive nature of telomeric sequences allows for the chemical synthesis and/or PCR amplification of long telomeric regions suitable for vector construction. Long tracts of telomeric repeats, *e.g*., (CCCTAAA)ₙ to flank minichromosome ends can be generated.

Long stretches of tandem telomeric repeats can be produced by several rounds of PCR amplification using primer pair SEQ ID NOs: 5 & 6 by mutual priming of two complementary telomeric oligonucleotides and their products. A PCR reaction using a low concentration of the primers (< 0.1 µM) can produce DNA segments of about 100-10000 bp. Optionally, synthetic telomeric repeats can be produced by ligation of phosphorylated oligos. Telomeric DNA segments were cloned and used to produce DNA constructs.

### B. Identification and Isolation of Subtelomeric Sequences

### i. BAC clones containing telomeric repeats

BAC clones containing subtelomeric regions comprising telomeric repeats can be used to stabilize chromosomal ends of a minichromosome construct. A number of sequences were previously identified as subtelomeric repeats (Burr et al. (1992) J Plant Cell 4:953-60). The Genbank sequence database was keyword searched for telomeric and subtelomeric sequences. Selected sequences were aligned and a common repetitive element identified (Telo266, SEQ ID NO:189). Using SEQ ID NO:189, several oligonucleotides were designed and used as probes to screen the Mo17 BAC library. A number of BACs were recovered, one was selected (bacm.pk107.g1), labeled, and hybridized to pachytene chromosomes. The BAC clone sequences were found in clusters on 6 out of 20 subtelomeres in maize chromosomes. The bacm.pk107.g1 BAC insert was subcloned and sequenced. Sequence analysis revealed a common repetitive element (TR430, SEQ ID NO:190) which was used to design overgo probes (Table 1). Subtelomeric location of those repeats was confirmed by FISH to maize Mo17 and B73 pachytene chromosomes. Using the same probes, maize Mo17 genomic BAC libraries were screened by colony hybridization.

Approximately 71 BAC clones containing blocks of maize subtelomeric repeats were confirmed as having the TR430 subtelomere repeat (Table 7).

**TABLE 7**

| | | | |
|---|---|---|---|
| bacm.pk155.e24 | bacm.pk166.a12 | bacm.pk173.m16 | bacm.pk203.j15 |
| bacm2.pk022.m14 | bacam2.pk092.a9 | bacm2.pk114.i4 | bacm2.pk169.b21 |
| bacm2.pk177.j18 | bacm2.pk190.m10 | bacm2.pk220.h7 | bacm.pk001.k4 |
| bacm.pk009.c19 | bacm.pk024.j15 | bacm.pk024.k8 | bacm.pk036.g23 |
| bacm.pk038.g6 | bacm.pk061.i6 | bacm.pk062.g4 | bacm.pk064.f6 |
| bacm.pk070.j17 | bacm.pk071.c12 | bacm.pk073.m7 | bacm.pk082.m9 |
| bacm.pk101.h5 | bacm.pk107.g1 | bacm.pk110.h10 | bacm.pk112.b18 |
| bacm.pk123.e21 | bacm.pk125.n6 | bacm.pk132.h6 | bacm.pk141.p12 |
| bacm.pk142.b15 | bacm.pk146.l14 | bacm.pk148.j17 | bacm.pk154.a21 |
| bacm.pk155.p12 | bacm.pk157.d2 | bacm.pk164.n4 | bacm.pk165.n1 |
| bacm.pk169.n16 | bacm.pk171.d3 | bacm.pk172.m20 | bacm.pk172.n19 |
| bacm.pk172.n16 | bacm.pk173.e9 | bacm.pk173.i12 | bacm.pk174.g4 |
| bacm.pk176.q2 | bacm.pk184.e5 | bacm.pk185.o19 | bacm.pk189.a10 |
| bacm.pk197.m23 | bacm.pk198.f9 | bacm.pk198.k3 | bacm.pk200.c20 |
| bacm.pk208.j1 | bacm.pk213.f2 | bacm.pk214.i17 | bacm.pk214.k16 |
| bacm.pk214.l11 | bacm.pk214.m20 | bacm2.pk007.d1 | bacm2.pk034.k22 |
| bacm2.pk043.g14 | bacm2.pk043.j16 | bacm2.pk073.o7 | bacm2.pk102.o18 |
| bacm2.pk108.a3 | bacm2.pk117.h13 | bacm2.pk160.l2 | bacm.pk203.j15 |
| bacm.pk155.e24 | bacm.pk166.a12 | baacm.pk173.m16 | bacm2.pk169.b21 |
| bacm2.pk022.m14 | bacam2.pk092.a9 | bacm2.pk114.i4 | bacm.pk001.k4 |
| bacm2.pk177.j18 | bacm2.pk190.m10 | bacm2.pk220.h7 | bacm.pk036.q23 |
| bacm.pk009.c19 | bacm.pk024.j15 | bacm.pk024.k8 | |

Restriction fingerprinting with *Dpn*I and blot-hybridization with TR430 probes, (CCCTAAA)n probe, and to knob 180 bp repeat probes showed at least 3 types of subtelomeric BAC clones. The first type has long tracts of TR430 related repeats longer than 10-20 kb. The second of BAC clones has TR430 related repeats which have a restriction site within the unit, wherein unit size can be 800 bp or 900 bp. Some BAC clones contained both of these two repeats. The third type of BAC clones has TR430 bp related unit around 500 bp. Some of these BAC clones also have telomeric (CCCTAAA)n related repeats. Knob 180 bp repeats are also present in 37 subtelomeric BAC clones suggesting that knob 180 bp repeats can be a part of some subtelomeric regions. Representative BAC clones of each type were taken for further analyses, retrofitting experiments, and transgenic experiments: bacm.pk038.g6; bacm2.pk063.g24; bacm.pk071.c12; bacm.pk112.b18; bacm.pk142.b15; bacm.pk173.e9. BAC inserts with subtelomeric fragments can be used in DNA constructs for minichromosome assembly *in vitro,* or assembly in a plant cell.

### ii. Isolation of native chromosomal telomeric DNA fragments

Chromosomal telomeric fragments that retain at least one native genomic characteristic, such as methylation pattern, were purified from maize genomic DNA by size fractionation of maize genomic DNA digested with restriction enzymes which have a short recognition site of 4 bp or smaller. Native maize telomeric sequence comprises hundreds or thousands of tandem repeats of CCCTAAA at each telomere, this short telomere tandem repeat has no recognition site for any known restriction enzyme. Any short cutter restriction enzymes which recognize 2-4 bp sequence can be used, as long as they have no specificity to canonical telomeric tandem repeat (CCCTAAA)n. Short cutters digest most of genomic DNA onto small fragments which can be separated from larger telomeric DNA. Using a combination of two or more short cutting restriction enzymes can eliminate other non-telomeric DNA fragments not fragmented by the first enzyme. There are no known restriction enzymes having a recognition site within canonical tandem telomeric repeat.

Maize genomic DNA from Mo17 was digested with *Sau*3A restriction enzyme, most maize genomic DNA is reduced to very small fragments well below 1 kb, while the majority of telomeric DNA fragments are larger than about 15 kb as determined by blot hybridization. The overall length of *Sau*3A telomere DNA segments per haploid genome is about 400 kb, or 0.02% of the total maize haploid genome. Approximately 1 mg of total maize genomic DNA yields approximately 200 ng of telomeric DNA fragments in the undigested relic fraction. The genomic telomeric DNA fraction can be purified from the gel and used to generate DNA constructs for minichromosome assembly.

### EXAMPLE 3. Origin of Replication

The DNA constructs are retrofitted with DNA segments carrying replication origins to enable proper replication of the construct and/or minichromosome in the nuclei of transgenic plant cells. Any origin of replication which functions in a plant cell can be used. Available origins of replication are known and include plant origins of replication, and viral origins of replication. Optionally, if a construct will be maintained in a non-plant host cell at least one appropriate origin of replication can be included in the construct, for example bacterial and/or yeast origin(s) of replication.

### A. Non-transcribed spacer of 18-26S rDNA

A well-established eukaryotic replication origin is the non-transcribed spacer of 18-26S rDNA (Ivessa & Zakian (2002) Genes Dev 16:2459-2464) which is likely functional in plants (Hemandez et al. (1993) EMBO J 12:1475-85). The 18-26S rDNA NTS DNA sequences can be isolated from a variety of different plant species, such as *Zea mays, Triticum aestivum, Avena sativa, Hordeum vulgare, Arabidopsis thaliana,* and/or *Glycine max.* These sequences are cloned into constructs as single or multiple dispersed copies. Eukaryotic chromosomes typically have multiple origins of replication, therefore inclusion of multiple origins of replication in the DNA constructs may be useful. Unless otherwise stated, the 18-26S rDNA NTS sequence from maize is used in the DNA constructs (Toloczyki & Feix (1986) Nucleic Acids Res 14:4969-86).

### B. Wheat dwarf virus (WDV) initiator protein (Rep)

Wheat dwarf virus (WDV) initiator protein (Rep) and its cognate origin of replication can be used for generating DNA constructs for minichromosome assembly. The wheat dwarf virus (WDV) initiator protein (Rep) and its cognate origin of replication can be used to support replication of minichromosome constructs in maize cells. The WDV origin of replication can be provided on the DNA construct (in cis), while genes needed for initiator Rep protein and cell cycle stimulating RepA protein can be provided by co-transformation on independent plasmid constructs (in trans) (Sanz-Burgos & Gutierrez (1998) Virology 243:119-129.).

### EXAMPLE 4. Polynucleotides and polypeptides that stimulate growth

Polynucleotides and/or polypeptides that enhance cell growth by promoting cell division, entrance into S phase, stimulate cell division and/or growth in culture, or improve transformation can be provided before, during, or after introducing DNA constructs comprising maize centromeric sequence and/or subtelomeric fragment. Any such composition, or combination thereof can be used including polynucleotides, polypeptides, and/or other factors using any suitable delivery method.

### A. Replication associated protein A.

Replication protein A from wheat dwarf virus (WDV) can be provided to enhance cell growth and/or recovery of transgenic events. Both RepA that retains replication activity and a modified RepA that does not support viral replication can be used. For example, a plasmid carrying nos promoter::RepA can be co-delivered into plant cells with the DNA construct(s). Transient expression of RepA during first three days is expected to be sufficient to stimulate cell division and enhance event recovery (see, for example, WO00/50614).

### B. Cyclins

Cyclin proteins, involved in cell cycle modulation may enhance cell growth and recovery of transgenic events. For example, maize cyclin D can stimulate cell division and callus growth in culture and improve maize transformation. Ectopic expression of E2F induced cell proliferation in *Arabidopsis,* this effect was enhanced by co-expression of DPa (de Veylder et al. (2002) EMBO J 21:1360-1368). Many cell cycle homologues, including cyclin D, cyclin E, wee1, Rb, Rbr3, E2F/DP, and the like have been isolated from plants (U.S. Patent 6,518,487; WO99/61619; WO00/37645; WO02/074909; Xie et al. (1996) EMBO J 15:4900-4908), and can be introduced into vectors for delivery into plant cells.

### C. Wuschel

Genes that trigger specific developmental pathways are also useful in enhancing cell growth. For example, members of the WOX family, such as wuschel (WUS) appear to stimulate cell division in both cells expressing WUS and adjacent cells. A construct comprising a polynucleotide encoding a WUS polypeptide can be used to stimulate cell division by co-transformation with the DNA construct(s). Several WUS homologues are known in plants, such as *Arabidopsis* and maize (*e.g*., Mayer et al. (1998) Cell 95:805-815; WO01/0023575; and US2004/0166563), and can be used to enhance the growth of transformed cells. For example, a construct comprising a maize WUS gene was constructed:
PHP21139 ubi pro::ubi 5' UTR::ubi intron::WUS::pinII

### D. Ovule development Protein 2

Other genes of interest include those related to the AP2/ERF family of transcription factors which are preferentially expressed in developing embryos and seeds, including Ovule development Protein 2 (ZmODP2) which is expressed early in maize embryogenesis. When ectopically expressed, ODP2 may stimulate cell growth in a variety of tissues, including non-embryonic tissues, which can facilitate the recovery of transgenic events. This gene family includes baby boom (BBM, BNM3, ODP2) which has been shown to induce ectopic somatic embryos in plants (Boutilier et al. (2002) Plant Cell 14:1737-1749). BBM/ODP2 homologues are known, including homologues from maize (WO00/75530) and can be delivered to plant cells to enhance cell growth. For example, a construct comprising a maize ODP2 gene was constructed:
PHP21875 ubi pro::ubi 5' UTR::ubi intron::ODP2::pinII

### E. Knotted-1

Homeobox genes, including members of the knox gene family, such as KN1, KNAT1, and STM function in meristem initiation and/or maintenance in plants (Jackson et al. (1994) Dev 120:405-413; Lincoln et al. (1994) Plant Cell 6:1859-1876; Venglat et al. (2002) Proc Natl Acad Sci USA 99:4730-4735). Many knox family members are known in plants, including homologues from maize (Vollbrecht & Hake (1991) Nature 350:241-243; Kerstetter et al. (1994) Plant Cell 6:1877-1887; Serikawa et al. (1996) Plant Mol Biol 32:673-683) and can be used to construct vectors for delivery into plant cells.

### F. Lec1

Leafy cotyledon genes, such as Lec1 and Lec2, are involved in the regulation of embryogenesis and transcriptional activity in plants (Meinke et al. (1994) Plant Cell 6:1049-1064; Lotan et al. (1998) Cell 93:1195-1205; WO00/28058; Stone et al. (2001) Proc Natl Acad Sci USA 98:11806-11811; U.S. Patent 6,492,577). Many homologues are known which can be used to construct vectors for delivery into plant cells.

### G. Combination of growth stimulating polynucleotides

A combination of polynucleotides and/or polypeptides that enhance cell growth by promoting cell division, entrance into S phase, stimulate cell division and/or growth in culture, or improve transformation can be provided before, during, or after introducing DNA constructs comprising maize centromeric sequence and/or subtelomeric fragment. For example polynucleotides encoding a maize ODP2 (PHP21875) and a maize WUS (PH121139) can be used in transformation experiments with DNA construct(s) comprising maize centromeric and/or subtelomeric regions. In general ODP2 and/or WUS in particle bombardment co-transformation of immature maize embryos, as described in Example 6D, showed a significant increase in the frequency of transgenic events as determined by BAR^{R} phenotype and fluorescent marker protein (DsRed) expression. On average 1008 events/4800 primary embryos (21 %) were observed when were provided in the transformation mixture. Without PHP21139 or PHP21875, 8 events/706 primary embryos (∼ 1 %) were observed. Further analyses of transgenic events indicated that the ODP2 and/or WUS co-bombarded constructs were not integrated into the genome or assembled minichromosomes.

### EXAMPLE 5. Vector construction

Vectors, circular or linear, for delivery into plant cells using any standard transformation protocol are constructed using standard molecular biology protocols, see *e.g.* Sambrook et al. (1989) Molecular Cloning: A Laboratory Manual, 2nd Ed., Cold Spring Harbor Laboratory Vols. 1-3. Vectors for the transformation of plant cells are constructed by combining isolated chromosomal elements, optionally with other polynucleotides of interest, using standard techniques. The vectors include those designed to be maintained in a convenient host system such as *E*. *coli, Agrobacterium,* or yeast, as well as in plant cells. Typically, the construct further comprises a selectable and/or screenable marker that functions in plant cells to aid in the maintenance, identification, and/or selection of plant cells comprising the minichromosome construct. Further, the construct typically comprises several unique restriction sites where additional polynucleotides of interest can be cloned. The construct may also comprise site-specific recombination sites useful for recombinational cloning, and/or for later targeting and/or modification of the minichromosome. DNA constructs derived from maize BAC clones comprising centromeric sequences for direct delivery or *Agrobacterium*-mediated plant transformation are described below. Various components can be supplied either on the BAC clone construct, and/or in trans on separate DNA constructs.

### A. Markers

A variety of markers can be used to identify transformed cells comprising the introduced DNA construct(s). Visual markers include fluorescent proteins, such as AmCyan, ZsYellow, or DsRed (ClonTech Laboratories, Inc., Mountain View, CA, USA). Selectable markers include PAT, BAR, GAT, and the like.

An expression cassette, PHP 23715, for delivery to plant cells comprising a red fluorescent protein (DsRed2) and a PAT selectable marker was constructed comprising the following operably linked components:
ubi pro::ubi 5'UTR::ubi intron::DsRed2::moPAT::pinll

A DNA construct, PHP 23714, comprising a cyan fluorescent protein (AmCyan) for delivery to plant cells is constructed comprising the following operably linked components:
ubi pro::ubi 5'UTR::ubi intron::AmCyan1::moPAT::pinll

### B. Agrobacterium vectors

*Agrobacterium* binary plasmids are made using the hybrid system described by Komari et al. ((1996) Plant J 10:165-174). Derivatives of pSB11 are built as intermediate T-DNA constructs containing the desired configuration between the T-DNA border sequences. Plasmid pSB11 is obtained from Japan Tobacco Inc. (Tokyo, Japan). Construction of pSB11 from pSB21, and construction of pSB21 from starting vectors, is described by Komari et al. ((1996) Plant J 10:165-174). Description of integration of the T-DNA plasmid into the superbinary plasmid pSB1 by homologous recombination can be found in EP672752 A1. The plasmid pSB1 is also obtained from Japan Tobacco Inc. These plasmids are used for *Agrobacterium*-mediated transformation after making the co-integrant in LBA4404. Electro-competent cells of the *Agrobacterium* strain LBA4404 harboring pSB1 are created using the protocol as described by Lin (1995) in Methods in Molecular Biology, ed. Nickoloff, J.A. (Humana Press, Totowa, NJ). Cells and DNA are prepared for electroporation by mixing 1 µl plasmid DNA (·100nag) with 20 µl of competent cells in a Life Technologies (now Whatman Biometra) 0.15 cm electrode gap cuvette ((Whatman Biometra #11608-031). Electroporation is performed in a Cell-Porator Electroporation device using the Pulse Control unit (Whatman Biometra #11604-014) at the 330 µF setting along with the Voltage Booster (Whatman Biometra #11612-017) set at 4 kW. The system delivers approximately 1.8 kV to the *Agrobacterium* cells. Successful recombination is verified by restriction analysis of the co-integrant plasmid following isolation and transformation back into *E. coli* DH5α cells for amplification.

### C. In vitro Assembly of Linear DNA Constructs via ligation

Linear DNA construct minichromosome vectors are produced by preparing the component DNA fragments such as maize centromeric sequences, selectable markers (DsRed2 and AmCyan), eukaryotic origin(s) of replication (ori), telomeric sequences (TEL), and gene conferring resistance to bialophos (PAT) under ubiquitin promoter (ubi). In one example, linear minichromosome vectors were made from centromeric BAC clone bacm.pk128.j21 which comprises inverted repeats of CentC tandem arrays flanking a CRM1 centromeric repeat element. DNA fragments were generated from bacm.pk128.j21 by digestion with *Not*I and agarose gel purification. The purified fragment comprising the centromeric region was combined with specific restriction digest fragments comprising selectable marker(s) and replication origin: ubi pro::ubi 5' UTR::ubi intron::DsRed::moPAT-18S-26S rDNA NTS (*Not*l/*Spe*l), and a second selectable marker cassette: ubi pro::ubi 5' UTR::ubi intron::AmCyan::moPAT (*Not*l/*Sma*l), and telomeric sequences (*Spe*l/*Xho*l or *Sma*l/*Kpn*l) from their constructs. DNA fragments were prepared such that each fragment comprised unique recognition sites for *in vitro* assembly of a unique linear structure during ligation. The assembled linearized vector comprises:
TEL-(*Spe*l)-ubi pro::ubi 5' UTR:ubi intron::AmCyan::moPAT-(*Not*l)-bacm.pk128.j21-(*Not*l)-ori-ubi pro::ubi 5' UTR::ubi intron::DsRed::moPAT-(*Sma*l)-TEL

### D. DNA constructs - Circular Retrofitted BAC clone vectors

Any centromeric and/or subtelomeric BAC clone or chromosomal fragment clone can be retrofitted with additional components for plant transformation.

The EPICENTRE EZ::TN™ pMOD™-2 MCS Transposon Construction Vector system (EpiCentre Madison, WI, USA) is used to retrofit polynucleotides of interest into existing BAC clones. The pMOD-2 is a pUC based plasmid with a colE1 origin of replication and multiple cloning site (MCS) between the hyperactive 19 bp mosaic ends (ME) recognized by EZ-Tn5 transposase. The Tn5-2 transposon integrates randomly into each target DNA, therefore each transposistion reaction generates a small library of constructs representing different integration sites. DNA preparations of individual retrofitted clones or a group of clones can be used for transformation of plant cells.

### i. Centromeric BACs

Two representatives of CentC-only BACs, bacm.pk018.I13 and bacm2.pk174.o21, were selected based on their restriction enzyme digest and Southern hybridization patterns. These BAC clones were retrofitted using the EPICENTRE EZ::TN™ pMOD™-2 MCS construction system to generate circular DNA constructs for plant transformation and minichromosome assembly.

The MCS was used to insert a DNA fragment comprising selectable markers: ubi pro::ubi 5' UTR::ubi intron::DsRed::moPAT with or without a maize 18-26S rDNA NTS ori to produce a first version of a custom transposon construct, Tn5-1s. After cloning the DNA sequences of interest, the transposon is generated by *Psh*Al restriction enzyme digest. Upon integration BAC constructs are transformed into *E. coli*, positive clones selected by colony hybridization with the transposon probes, and DNA isolated from selected positive clones.

### ii. Subtelomeric BACs

Six representative BAC clones were selected from the subtelomeric BAC pool: bacm.pk038.g06, bacm2.pk063.g24, bacm.pk071.c12, bacmpk112.b18, bacm.pk142.b15, and bacm.pk173.e09. New custom Tn5-2 transposon constructs comprising 18-26S rDNA NTS ori-ubi pro::ubi 5' UTR::ubi intron::DsRed::moPAT, a Kan^{r} gene, and sites for three different homing restriction enzymes: I-*Ppo*I, I-*Ceu*I, and PI-*Sce*I, were built and used to retrofit the subtelomeric BAC clones. The retrofitted BAC constructs are transformed into *E. coli* and selected on kanamycin and chloramphenicol, DNA is isolated from selected positive clones.

### E. DNA Constructs - Linearized retrofitted BAC clones

Additional custom Tn5-3 transposon constructs were generated. These Tn5-3 vectors comprise 18-26S rDNA NTS ori-ubi pro::ubi 5' UTR::ubi intron::DsRed::moPAT. The constructs also comprise a Kan^{r} gene flanked by two DNA segments in inverted orientation each composed of two recognition sites for the homing restriction enzymes I-CeuI and PI-SceI, and telomeric sequence comprising arrays of telomeric repeats. After cloning the DNA sequences of interest, the transposon is generated by *Psh*Al restriction enzyme digest. Upon integration BAC constructs are transformed into *E. coli* and selected on kanamycin and chloramphenicol, DNA is isolated from selected positive clones. Recombinant retrofitted BAC DNA is digested *in vitro* with homing restriction enzyme (I-*Ceu*I or PI-*Sce*I) converting the circular DNA into a linear DNA construct flanked with telomeric sequences in the correct orientation, and removing the fragment comprising Kan^{r} (Figure 13).

Three types of centromeric BAC clones were retrofitted with this Tn5-3 vector:
1. Centromeric BAC clone with inverted blocks of centromeric CentC repeats flanking a CRM1 centromeric element bacm.pk128.j21, no CentA or CRM2 sequences;
2. Centromeric BAC clones belonging to the core set of centromeric BAC clones containing all four centromere-specific repeats CentA, CentC, CRM1, and CRM2 (Table 8); and,
3. Centromeric BAC clones from maize chromosome 4 (Table 9).

DNA samples from each BAC clone are fractionated in an agarose gel and the band containing linear retrofitted BAC construct excised. DNA is electroeluted from the agarose and used for biolistic transformation of Hi-II immature embryos 8-11 DAP (days after pollination). Optionally, these constructs can be used for microinjection of the DNA, or converted into vectors for *Agrobacterium*-mediated transformation.

**TABLE 8**

| **Pool 1** | **Pool 2** | **Pool 3** | **Pool 4** |
|---|---|---|---|
| bacm.pk007.a2 | bacm.pk011.l8 | bacm.pk001.n1 | bacm.pk109.h24 |
| bacm.pk036.e13 | bacm.pk012.n20 | bacm.pk023.i5 | bacm.pk039.a3 |
| bacm.pk066.j14 | bacm.pk013.m8 | bacm.pk043.o23 | bacm.pk039.m16 |
| bacm.pk075.16 | bacm.pk062.c14 | bacm.pk051.g11 | bacm.pk041.e16 |
| bacm.pk076.m3 | bacm.pk064.n1 | bacm.pk056.j19 | bacm.pk077.b21 |
| bacm.pk119.a23 | bacm.pk068.p16 | bacm.pk076.o15 | bacm.pk079.m11 |
| bacm.pk133.b10a | bacm.pk070.h17 | bacm.pk087.m4 | bacm.pk085.k5 |
| bacm.pk133.b10b | bacm.pk090.o5 | bacm.pk089.18 | bacm.pk098.h2 |
| bacm.pk133.b11 | bacm.pk098.f3 | bacm.pk093.d8 | bacm.pk102.i4 |
| bacm.pk135.i6 | bacm.pk135.17 | bacm.pk106.j20 | bacm.pk112.p1 |
| bacm.pk178.c10 | bacm2.pk002.g7 | bacm.pk129.a4 | bacm.pk124.j24 |
| bacm2.pk023.e24 | bacm2.pk003.g6 | bacm.pk134.f15 | bacm.pk143.m18 |
| bacm2.pk064.e15 | bacm2.pk012.g19 | bacm.pk135.j2 | bacm.pk148.e2 |
| bacm2.pk066.m12 | bacm2.pk013.c9 | bacm.pk138.e14 | bacm.pk156.i17 |
| bacm2.pk083.a2 | bacm2.pk034.g20 | bacm.pk141.j4 | bacm.pk164.b11 |
| bacm2.pk093.h11 | bacm2.pk053.g23 | bacm.pk161.h1 | bacm.pk166.n7 |
| bacm2.pk099.m24 | bacm2.pk070.g7 | bacm.pk164.e18 | bacm.pk178.o20 |
| bacm2.pk116.g16 | bacm2.pk094.f14 | bacm.pk179.d4 | bacm2.pk034.j8 |
| bacm2.pk174.e4 | bacm2.pk096.d23 | bacm2.pk130.e20 | bacm2.pk075.n6 |
| bacm2.pk179.b18 | bacm2.pk100.j24 | bacm2.pk137.f2 | bacm2.pk115.o22 |
| bacm2.pk179.e1 | bacm2.pk179.014 | bacm2.pk158.f12 | bacm2.pk169.a21 |

**TABLE 9**

| **Chromosome 4-specific B73-pool** | **Chromosome 4-specific Mo17-pool** |
|---|---|
| baccpk0143i9 | bacm.pk010m7 |
| bacbpk0155h15 | bacm.pk108h15 |
| bacbpk0424d20 | bacm.pk184c21 |

### F. DNA constructs - Retrofitted multiple BAC combination vectors

Centromeric BAC clones belonging to the core set of centromeric BAC clones containing all four centromere-specific repeats CentA, CentC, CRM1, and CRM2 (Table 8) were also retrofitted with the Tn5-2 vector. Tn5-2 constructs comprising ori-ubi pro::ubi 5' UTR::ubi intron::DsRed2::moPAT, a Kan^{r} gene, and sites for three homing restriction enzymes: I-*Ppo*l, I-*Ceu*l, and PI-*Sce*l. The retrofitted BACs were cut with homing restriction enzymes I-Ceul and PI-Scel, separated by pulsed field gel electrophoresis (PFGE) under standard conditions: 1% agarose, 1X TAE, initial pulse 5 sec, final pulse 10 sec, total run time 12 hrs at 12°C. Large fragments were purified, and subjected to ligation to form multimeric DNA constructs up to 1 Mb long.

### EXAMPLE 6: Plant Transformation

Any suitable plant transformation method can be used. Similarly any plant cell and/or tissue that can be transformed, cultured, and/or regenerated into a plant can be used. These plant cells and tissues, as well as culture media and conditions, suitable transformation methods, and regeneration media and conditions are well known.

### A. Cell types

A variety of maize cell types were evaluated for their potential as targets for minichromosome generation and construct delivery, including Black Mexican Sweet (BMS) suspension cells, meristem cells, the zygote, scutellar cells in the immature embryo, cells in cultured somatic embryos, the central cell and early endosperm cells. Methods are available to produce haploid embryos by crossing a given genotype to the RWS line, or other inducer line. Haploid immature embryos could be a good target for minichromosome delivery, either into scutellar cells 10-12 days after pollination (DAP) or into the exposed apical meristems of coleoptilar stage embryos (7-8 DAP). Important comparisons on the behavior of introduced minichromosomes into either a diploid or haploid environment could be performed, moreover, if minichromosome introduction is followed by chemically-induced chromosome doubling (*e.g*. colchicine, or nitrous oxide), these doubled-haploid embryos can be rapidly regenerated to produce a minichromosome-containing inbred. All of the aforementioned diploid and haploid cell types can be converted into suspension cultures and/or protoplasts, or established suspension cultures, such as BMS are suitable and can be used for transformation. Suspension cells and/or protoplasts may provide easy accessibility and optical clarity for microscopic monitoring after DNA construct delivery. Any suitable method for delivery of the construct to the plant protoplast culture can be used, including standard electroporation and PEG-mediated direct delivery methods, see *e.g*., Ch. 8, pp. 189-253 in Advances in Cellular and Molecular Biology of Plants, Vol. 5, Ed. Vasil, Kluwer Acad Publ (Dordrecht, The Netherlands) 1999.

### B. Microinjection of maize

Any suitable method for microinjection of plant cells, tissues, and/or embryos can be used. Further, any composition or combination/mixture of compositions can be injected, including polynucleotides, polypeptides, cofactors, chemicals, adjuvants, and the like. Direct delivery into a zygote provides an opportunity to produce a transgenic plant without the intermediate steps of tissue culture and regeneration. For example, microinjection, of maize can be done essentially as described in U.S. Patent 6,300,543. Briefly, immature maize ovules are sectioned to produce nucellar slabs comprising the embryo sac, which is targeted for microinjection delivery of the transformation composition. Following microinjection, the embryo sacs are cultured in the appropriate media for propagation and plant regeneration.

### C. Agrobacterium mediated transformation

*Agrobacterium* mediated transformation of maize is performed essentially as described by Zhao (WO98/32326). Briefly, immature embryos are isolated from maize ovules and the embryos contacted with a suspension of *Agrobacterium* containing a T-DNA, where the bacteria are capable of transferring the DNA construct to at least one cell of at least one of the immature embryos. Optionally, the target tissue can be co-transformed with multiple *Agrobacterium* lines comprising T-DNAs with different DNA constructs and/or polynucleotides of interest.
Step 1: Infection Step. Immature embryos are immersed in an *Agrobacterium* suspension for the initiation of inoculation.
Step 2: Co-cultivation Step. The embryos are co-cultured for a time with the *Agrobacterium.*
Step 3: Resting Step. Optionally, following co-cultivation, a resting step may be performed. The immature embryos are cultured on solid medium with antibiotic, but without a selecting agent, for elimination of *Agrobacterium* and for a resting phase for the infected cells.
Step 4: Selection Step. Inoculated embryos are cultured on medium containing a selective agent and growing transformed callus is recovered. The immature embryos are cultured on solid medium with a selective agent resulting in the selective growth of transformed cells.
Step 5: Regeneration Step. Calli grown on selective medium are cultured on solid medium to regenerate the plants.

### D. Particle Bombardment of Maize

Immature maize embryos are bombarded with a circular or linear DNA construct comprising an isolated maize centromeric sequence, and optionally subtelomeric region(s), origin(s) of replication, recombination docking site(s), polypeptide(s), and/or markers, for example a selectable marker gene such as PAT (Wohlleben et al. (1988) Gene 70:25-37) that confers resistance to the herbicide Bialaphos, or another suitable selectable marker or screenable marker(s), such as RFP and/or CFP. The construct may also comprise other marker genes, or be co-transformed with additional polynucleotide constructs comprising markers. Transformation is performed essentially as follows.

Immature maize ears from 8-11 DAP are surface sterilized in a solution of 30% bleach plus 0.5% Micro detergent for 20 minutes, and rinsed two times with sterile water. The immature embryos are excised, placed embryo axis side down (scutellum side up), 50 embryos per plate, on 560L medium for 1-3 days at 26°C in the dark. Before transformation the immature embryos are transferred on to 560Y medium for 4 hours, and then aligned within the 2.5-cm target zone in preparation for bombardment.

The DNA is precipitated onto 0.6 µm (average diameter) gold pellets using a water-soluble cationic lipid Tfx™-50 (Cat# E1811, Promega, Madison, WI, USA) as follows: prepare DNA solution on ice using 1 µg of maize centromeric DNA construct (10 µl); optionally other constructs for co-bombardment such as 50 ng (0.5 µl) PHP21875 (BBM), and 50 ng (0.5 µl) PHP21139 (WUS); mix DNA solution. To the pre-mixed DNA add 20 µl prepared gold particles (15 mg/ml) in water; 10 µl Tfx-50 in water; mix carefully. This can be stored on ice during preparation of macrocarriers, typically about 10 min. Pellet gold particles in a microfuge at 10,000rpm for 1 min, remove supernatant. Carefully rinse the pellet with 100 ml of 100% EtOH without resuspending the pellet, carefully remove the EtOH rinse. Add 20 µl of 100% EtOH and carefully resuspend the particles by brief sonication, 10 µl spotted onto the center of each macrocarrier and allowed to dry about 2 minutes before bombardment.

The sample plates of maize target embryos are bombarded twice per plate using approximately 0.5 µg of DNA per shot using the Bio-Rad PDS-1000/He device (Bio-Rad Laboratories, Hercules, CA) with a rupture pressure of 450 PSI, a vacuum pressure of 27-28 inches of Hg, and a particle flight distance of 8.5 cm.

Following bombardment, the embryos are transferred to 560P solid medium kept in the dark at 26°C for 4-6 days, then transferred to 560R selection medium containing 3 mg/L Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to 272V medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity.

### E. Particle Bombardment of Soybean

A polynucleotide, a mixture of polynucleotides, and optionally, polypeptide(s), can be introduced into embryogenic suspension cultures of soybean by particle bombardment using essentially the methods described in Parrott et al. (1989) Plant Cell Rep 7:615-617. This method, with modifications, is described below.

Seed is removed from immature pods and cotyledons less than 4mm in length are selected. The seeds are sterilized for 15 minutes in a 0.5% v/v bleach solution and then rinsed with sterile distilled water. The immature cotyledons are excised by first cutting away the portion of the seed that contains the embryo axis. The cotyledons are then removed from the seed coat by gently pushing the distal end of the seed with the blunt end of the scalpel blade. The cotyledons are then placed in petri dishes (flat side up) with SB1 initiation medium. The petri plates are incubated in the light (16 hr day; 75-80 µE) at 26°C. After 4 weeks of incubation the cotyledons are transferred to fresh SB1 medium. After an additional two weeks, globular stage somatic embryos that exhibit proliferative areas are excised and transferred to FN Lite liquid medium (Samoylov et al. (1998) In Vitro Cell Dev Biol Plant 34:8-13). About 10 to 12 small clusters of somatic embryos are placed in 250 ml flasks containing 35 ml of SB172 medium. The soybean embryogenic suspension cultures are maintained in 35 mL liquid media on a rotary shaker, 150 rpm, at 26°C with fluorescent lights (20 µE) on a 16:8 hour day/night schedule. Cultures are sub-cultured every two weeks by inoculating approximately 35 mg of tissue into 35 mL of liquid medium.

Soybean embryogenic suspension cultures are then transformed using particle gun bombardment (Klein et al. (1987) Nature 327:70; U.S. Patent 4,945,050). A BioRad Biolisticä PDS1000/HE instrument can be used for these transformations. A selectable marker gene can used to facilitate soybean transformation for example an expression cassette can be used comprising the 35S promoter from Cauliflower Mosaic Virus (Odell et al. (1985) Nature 313:810-812), the hygromycin phosphotransferase gene from plasmid pJR225 (from *E. coli*; Gritz et al. (1983) Gene 25:179-188) and the 3' region of the nopaline synthase gene from the T-DNA of the Ti plasmid of *Agrobacterium tumefaciens.*

To 50 µL of a 60 mg/mL 1 µm gold particle suspension is added (in order): 5 µL DNA (1 µg/µL), 20 µl spermidine (0.1 M), and 50 µL CaC1₂ (2.5 M). The particle preparation is agitated for three minutes, spun in a microfuge for 10 seconds and the supernatant removed. The DNA-coated particles are washed once in 400 µL 70% ethanol then resuspended in 40 µL of anhydrous ethanol. The DNA/particle suspension is sonicated three times for one second each. Five µL of the DNA-coated gold particles are then loaded on each macro carrier disk.

Approximately 300-400 mag of a two-week-old suspension culture is placed in an empty 60x15 mm petri dish and the residual liquid removed from the tissue with a pipette. Membrane rupture pressure is set at 1100 psi and the chamber is evacuated to a vacuum of 28 inches mercury. The tissue is placed approximately 8 cm away from the retaining screen, and is bombarded three times. Following bombardment, the tissue is divided in half and placed into two separate flasks with 35 ml of FN Lite medium per flask.

Five to seven days after bombardment, the liquid medium is exchanged with fresh medium. Eleven days post bombardment the medium is exchanged with fresh medium containing 50 mg/mL hygromycin. This selective medium is refreshed weekly. Seven to eight weeks post bombardment, green transformed tissue will be observed growing from untransformed, necrotic embryogenic clusters. Isolated green tissue is removed and inoculated into individual flasks to generate new, clonally propagated, transformed embryogenic suspension cultures. Each new line is treated as an independent transformation event. These suspensions are then subcultured and maintained as clusters of immature embryos, or tissue is regenerated into whole plants by maturation and germination of individual embryos.

For regeneration, events are removed from liquid culture and a maturation process is started on solid medium. Embryogenic clusters are removed from liquid SB196, blotted on sterile filter paper, and placed on solid agar media SB166 for 1-2 weeks. Tissue clumps are broken or gently squashed with spoonula. About 10-20 tissue clumps of about 4-5 mm diameter are subcultured for 3 weeks on medium SB103 or SB148, to generate embryos. Embryos are cultured for 4-6 weeks at 26°C under cool white fluorescent and Agro bulbs (40 watt) on a 16:8 hr photoperiod with light intensity of 90-120 µE/m2s. After 4-6 weeks of maturation, individual embryos are desiccated by placing into a large (60 x 25 mm) sterile petri dish sealed with fiber tape, or placed in plastic box (with no fiber tape) for 4-7 days. Desiccated embryos are planted in solid SB71-4 medium in either vented round culture vessel (RCV) or into 100x25 mm petri dish, and germinated at 26°C under cool white fluorescent and Agro bulbs (40 watt) on a 16:8 hr photoperiod with light intensity of 90-120 µE/m2s to produce plantlets. Plantlets are potted to cell pack trays and placed in an incubator at conditions of 16 hr photoperiod, 26°C/24°C day/night temperatures for about 2 weeks before transplanting to soil for seed production.

### F. Plant cell culture media

Medium 560L comprises 4.0 g/L N6 basal salts (Sigma C-1416), 1.0 ml/L Eriksson's Vitamin Mix (1000X Sigma 1511), 0.5 mg/L thiamine HCl, 20 g/L sucrose, 1.0 mg/L 2,4-D, and 2.88 g/L L-proline (brought to volume with D-I H₂O following adjustment to pH 5.8 with KOH); 2.0 g/L Gelrite^{®} (added after bringing to volume with D-I H₂O); and 8.5 mg/L silver nitrate (added after sterilizing the medium and cooling to room temperature).

Medium 560P comprises 4.0 g/L N6 basal salts (Sigma C-1416), 1.0 ml/L Eriksson's Vitamin Mix (1000X Sigma 1511), 0.5 mg/L thiamine HCl, 30 g/L sucrose, 2.0 mg/L 2,4-D, and 0.69 g/L L-proline (brought to volume with D-I H₂O following adjustment to pH 5.8 with KOH); 3.0 g/L Gelrite^{®} (added after bringing to volume with D-I H₂O); and 0.85 mg/L silver nitrate (added after sterilizing the medium and cooling to room temperature).

Medium 560Y comprises 4.0 g/L N6 basal salts (Sigma C-1416), 1.0 ml/L Eriksson's Vitamin Mix (1000X Sigma 1511), 0.5 mg/L thiamine HCl, 120 g/L sucrose, 1.0 mg/L 2,4-D, and 2.88 g/L L-proline (brought to volume with D-I H₂O following adjustment to pH 5.8 with KOH); 2.0 g/L Gelrite^{®} (added after bringing to volume with D-I H₂O); and 8.5 mg/L silver nitrate (added after sterilizing the medium and cooling to room temperature).

Medium 560R comprises 4.0 g/L N6 basal salts (Sigma C-1416), 1.0 ml/L Eriksson's Vitamin Mix (1000X Sigma 1511), 0.5 mg/L thiamine HCl, 30.0 g/L sucrose, and 2.0 mg/L 2,4-D (brought to volume with D-I H₂O following adjustment to pH 5.8 with KOH); 3.0 g/L Gelrite (added after bringing to volume with D-I H₂O); and 0.85 mg/L silver nitrate and 3.0 mg/L bialaphos (both added after sterilizing the medium and cooling to room temperature).

Medium 288J comprises: 4.3 g/L MS salts (Gibco 11117-074), 5.0 ml/L MS vitamins stock solution (0.100 g/L nicotinic acid, 0.02 g/L thiamine HCl, 0.10 g/L pyridoxine HCl, and 0.40 g/L glycine brought to volume with D-I H₂O) (Murashige & Skoog (1962) Physiol Plant.15:473), 100 mg/L myo-inositol, 0.5 mg/L zeatin, 60 g/L sucrose, and 1.0 ml/L of 0.1 mM abscissic acid (brought to volume with D-I H₂O after adjusting to pH 5.6); 3.0 g/L Gelrite (added after bringing to volume with D-I H₂O); and 1.0 mg/L indoleacetic acid and 3.0 mg/L bialaphos (added after sterilizing the medium and cooling to 60°C).

Medium 272V comprises: 4.3 g/L MS salts (Gibco 11117-074), 5.0 ml/L MS vitamins stock solution (0.100 g/L nicotinic acid, 0.02 g/L thiamine HCl, 0.10 g/L pyridoxine HCl, and 0.40 g/L glycine brought to volume with D-I H₂O), 0.1 g/L myo-inositol, and 40.0 g/L sucrose (brought to volume with D-I H₂O after adjusting pH to 5.6); and 6 g/L bacto-agar (added after bringing to volume with D-I H₂O), sterilized and cooled to 60°C.

Medium SB1 comprises MS salts (Gibco/ BRL - Cat# 11117-066, 1 pk/L), B5 vitamins stock 1 ml/L, 20 mg/L 2,4-D, 31.5 g/L sucrose, 8 g/L TC Agar, pH 5.8

B5 Vitamins 1000X Stock comprises 10 g myo-inositol, 100 mg nicotinic acid, 100 mg pyridoxine HCl, 1 g thiamine, D-I H₂O to 100 ml, aliquot and store at -20°C.

### G. DNA Isolation from Callus and Leaf Tissues

Putative transformation events can be screened for the presence of the transgene. Genomic DNA can be extracted from calli, leaves, or other tissue using plant nuclei separation, lysis, and HMW purification, or alternatively using a modification of the CTAB (cetyltriethylammonium bromide, Sigma H5882) method described by Stacey & Isaac (1994 in Methods in Molecular Biology Vol. 28, pp. 9-15, Ed. P.G. Isaac, Humana Press, Totowa, NJ). Approximately 100-200 mg of frozen tissue is ground into powder in liquid nitrogen and homogenized in 1 ml of CTAB extraction buffer (2% CTAB, 0.02 M EDTA, 0.1 M TrisHCl pH 8, 1.4 M NaCl, 25 mM DTT) for 30 min at 65°C. Homogenized samples are allowed to cool at room temperature for 15 min before a single protein extraction with approximately 1 ml 24:1 v/v chloroform:octanol is done. Samples are centrifuged for 7 min at 13,000 rpm and the upper layer of supernatant collected using wide-mouthed pipette tips. DNA is precipitated from the supernatant by incubation in 95% ethanol on ice for 1 h. DNA threads are spooled onto a glass hook, washed in 75% ethanol containing 0.2 M sodium acetate for 10 min, air-dried for 5 min and resuspended in TE buffer. Five µl RNAse A is added to the samples and incubated at 37°C for 1 h. For quantification of genomic DNA, gel electrophoresis is performed using a 0.8% agarose gel in 1x TBE buffer. One microlitre of each of the samples is fractionated alongside 200, 400, 600 and 800 ng µl-1 λ uncut DNA markers.

### EXAMPLE 7. Transformation Results

Immature Hi-II maize embryos at 8-11 DAP were transformed by particle bombardment essentially as described in Example 6D. Along with the retrofitted BAC DNA construct(s), embryos were co-transformed with ODP2, WUS, and/or ODP2+WUS vectors. At two weeks post-bombardment, transformed cells had proliferated to form an embryogenic callus with multiple somatic embryos. Some of these somatic embryos expressed the fluorescent DsRed2 marker gene indicative of stable inheritance. Individual somatic embryos were excised and propagated as independent transgenic events on Bialophos selection media. Clonally propagated callus culture was established from each event.

A primary screening of each transformation event was made utilizing FISH. Individual somatic embryos were used to make chromosomal spreads for FISH as described in Example 8. Each event was characterized using separate FISH probes to the mo-PAT/DsRed2 marker (PHP23715), and the CentC tandem centromeric repeat to detect of transgenic marker and centromeric DNA sequence inheritance, respectively.

Following the primary screening, selected transformation events of interest were transferred to regeneration media to produce plantlets, which were eventually transferred into soil to recover plants. After a period of growth, the selected plants were screened a second time by FISH analysis of root tip squashes to reaffirm inheritance.

### A. Co-transformation experiments of pooled BACs

The embryos were co-transformed with pools of DNA constructs. These pools can comprise combinations of DNA constructs derived from BAC clones comprising maize centromeric repeats, DNA constructs derived from BAC clones comprising telomeric and/or subtelomeric DNA segments, visual marker plasmid PHP23715, and polynucleotides encoding growth enhancing proteins Ovule Development Protein-2, ODP-2 (PHP21875) and Wushel (PHP21139) plasmids. DNA constructs derived from centromeric BAC clones include BAC clones having CentC only, CRM2 only, CentC and CRM2 only, and core BACs having all four centromeric repeats CentA, CentC, CRM1, and CRM2.

FISH analysis of 80 calli transformed with pooled BACs comprising centromeric DNA revealed 42 cytogenetically detectable events of new CentC clusters in additional to normal centromere sites. In some instances, the maize centromeric elements used for transformation inserted into the native chromosomes, resulting in dicentric structures. These insertions of centromeric DNA sequences varied in size (number of repeats), number of insertions per chromosome (up to 3 detectable in a single chromosome), and number of chromosomes with insertions (up to 4 chromosomes with at least one insertion) and all insertions co-localized with the RFP marker plasmids probe. This indicates that exogenous DNA fragments can be assembled into large blocks and integrated into a maize chromosome.

### B. Transformation with linear minichromosome prototype DNA constructs assembled by in vitro ligation of a centromeric BAC clone, telomeric sequences and marker sequences.

The Mo17 BAC clone, bacm.pk128.j21, with inverted orientation of CentC tandem repeats was identified as described in Example 1D. Telomeric sequences were generated by PCR amplification of telomeric oligonucleotides and cloned in a plasmid vector. A linear DNA construct was generated from this BAC clone by *in vitro* assembly with selectable markers (moPAT, AmCyan1, DsRed2), an 18-26S rRNA NTS replication origin (ori), and telomeric sequences (TEL). Each DNA fragment had recognition sites which allowed assembly of a unique structure upon ligation. The assembled linearized construct comprises: TEL-(*Spe*l)-ubi pro::ubi 5' UTR::ubi intron::AmCyan::moPAT-(*Not*l)-bacm.pk.128J21-(*Not*l)-ori-ubi pro::ubi 5' UTR::ubi intron::DsRed::moPAT-(*Sma*l)-TEL

The whole ligation mixture, containing assembled construct as well as byproducts of the ligation, was delivered into immature Hi-II embryos via biolistic transformation. More than two hundred events were propagated as individual callus clones based on the fluorescent and selectable marker selection (PAT). Three groups of clones were recovered: those which showed only red (72), only blue (83), or both (137) fluorescent markers. Events expressing both markers were selected for further analyzed by FISH.

In addition to simple integration events, a number of multiple integration events were observed either in the same chromosome, or in different chromosomes. In two events we observed chromosomal rearrangements. The additional insertion sites of centromeric repeat CentC co-localized with the marker probe PHP23715 suggesting possible dicentric chromosome formation. Analysis of dividing cells at anaphase showed chromosomal bridges consistent with the presence of dicentric chromosomes with two functional centromeres due to integration of exogenous centromeric CentC DNA sequences. Centromeric function is indicated by the formation of dicentric chromosomes, appearance of chromosomal bridges at anaphases, and the induction of chromosomal breaks. These results indicate that the chromosomal elements can self-assemble within the plant cell into multicopy blocks, associate with chromatin proteins, and in some cases can acquire centromeric function.

One event showed a rearranged chromosome 6 having two insertion sites of the centromeric DNA construct close to the nucleolar organizing region (NOR), as well as one additional minichromosome-like structure with one large centromeric region and one additional small insertion site of the centromeric DNA construct. The cytology of this event may be an indication of chromosomal breakage due to formation of a dicentric chromosome.

### C. Transformation with linearized retrofitted pooled BAC clones

Several BAC clones were retrofitted with Tn5-3 custom made transposon using the transposase system (EPICENTRE EZ::TN™ pMOD™-2 MCS Transposon Construction Vector system (EpiCentre, Madison, WI, USA)) essentially as described in Example 5E. They were linearized and used for biolistic transformation of maize Hi-II immature embryos:
1. Seven different variants of retrofitted bacm.pk128.j21 clone with inverted blocks of centromeric CentC repeats representing different transposase-generated insertions into the same BAC clone were pooled;
2. 84 retrofitted centromeric core set BAC clones were combined to generate 4 pools with 21 individual variants each (Table 8). Each of the four pools was used individually for biolistic transformation;
3. Retrofitted centromeric BAC clones from chromosome 4 were divided into 2 pools containing three BAC clones from B73 and three BAC clones from Mo17 (Table 9); and,
4. Pool 1 from Table 8, was divided into 4 subpools of 5 or 6 retrofitted centromeric core set BAC clones (Table 10). Each of the subpools was used individually for biolistic transformation.

**TABLE 10**

| **Subpool 1.1** | **Subpool 1.2** | **Subpool 1.3** | **Subpool 1.4** |
|---|---|---|---|
| bacm.pk007.a2 | bacm.pk133.b10 | bacm.pk119.a23 | bacm.pk075.l6 |
| bacm.pk036.e13 | bacm.pk077.k5 | bacm2.pk174.e4 | bacm.pk0066.j14 |
| bacm.pk178.c10 | bacm2.pk179.b18 | bacm2.pk116.g16 | bacm2.pk099.m24 |
| bacm2.pk179.e1 | bacm.pk0133.b11 | bacm2.pk023.e24 | bacm2.pk093.h11 |
| bacm2.pk064.e15 | bacm2.pk066.m12 | bacm.pk135.16 | bacm2.pk083.a2 |
| | | | bacm.pk076.m3 |

For each example above, the Hi-II immature embryos were co-transformed with ODP2, WUS, and/or ODP2+WUS expression vectors and the retrofitted BAC pools.

Several different classes of integration events were found when linearized retrofitted constructs from BAC clones were used for transformation. For example, when the constructs from the BAC containing inverted blocks of centromeric CentC repeats (bacm.pk128.j21), or retrofitted pools or subpools of the core set of BAC clones were used for transformation:
1. Single integrations into euchromatic regions of host chromosomes;
2. Multiple integrations into euchromatic regions of host chromosomes;
3. Single integrations into centromeric region of host chromosomes;
4. Multiple integrations into centromeric regions of host chromosomes;
5. Integrations which resulted in chromosome breaks, such as new unusual variants of corn chromosomes with reduced chromosomal arms, or duplication of certain chromosomal regions, for example a chromosome 6 with two NORs, or dicentric chromosome formation;
6. Local amplification of marker and centromeric constructs upon integration;
7. Amplification of marker and centromeric constructs into extrachromosomal chromatin segments in some cells;
8. Creation of new minichromosomes having a functional centromere similar to native chromosomes, for example autonomous segregation in mitosis.

These observations indicate that retrofitted centromeric BAC clone bacm.pk128.j21 and the retrofitted core set of pooled BAC clones are capable of inducing a variety of cytogenetic effects such as dicentric chromosome formation, chromosomal breaks, local amplification of transgenic constructs and formation of extra chromosomal elements, *i.e*. minichromosomes.

Successful minichromosome events that resulted from retrofitting of a single BAC or pool of centromere-specific BACs with the Tn5-3 construct and its subsequent linearization into a linear transformation construct are described below:
1) Pool 1 core set of centromeric-specific BACs (Table 8), or subpools of Pool 1 (Table 10);
2) Pool 3 core set of centromeric-specific BACs (Table 8);
3) a single BAC clone, bacm.pk128.j21, with inverted CentC repeats; and,
4) three B73 chromosome 4 centromere-specific BAC clones (Table 9).

The first maize minichromosome event (CMC3 pool 1 event #14) was found among events generated by biolistic transformation with linearized Tn5-3 retrofitted core set BAC pool 1 (Table 8). On selective media actively growing embryogenic callus expressed the DsRed2 visual marker. FISH analyses at metaphase stage showed 0, 1, 2, or 3 additional minichromosomes having various forms and sizes (Figures 1-4). In this event, 60 of 80 nuclei surveyed had 1, 2, or 3 minichromosomes along with the normal complement of 20 native chromosomes. These artificial chromosomes ranged in size from about 20% to about 50% of the average native corn chromosome as measured at metaphase. Preliminary measurements at prometaphase show the minichromosomes relatively unchanged in size, while the native chromosomes are about 4-5 times longer, therefore the minichrosomes measured at this stage are about 5% to about 15% of the length of an average native corn prometaphase chromosome. As determined by FISH the minichromosomes are predominantly composed of centromeric repeats and Tn5-3 components. Several examples of ring chromosome formation which have more complex organization were also observed. These newly formed minichromosomes are apparently capable of replication and segregation during mitosis (Figure 4), however segregation is not perfect and some non-disjunction was observed, resulting in cells with a change in minichromosome number. Callus of CMC3 pool 1 event #14 was kept actively growing under selection for at least 10 months, sampled at various timepoints, and analyzed by FISH to demonstrate stable maintenance of the minichromosome through many rounds of mitotic cell division. This event, CMC3 pool 1 event #14, was also analyzed by FISH for the presence of telomeres using the Telo-31 overgo probes (SEQ ID NOS: 192 & 193) using callus metaphase nuclei. Two to four telo-31 positive foci were observed on each minichromosome, wherein the two foci observed may represent 4 separate foci which cannot be distinguished at this resolution. The intensity of the telo-31 signal was generally weaker on the minichromosome as compared to the signal observed for the native chromosomes in each sample. Plants were regenerated from this event and their root tips were analyzed with FISH to determine if the minichromosome(s) were heritable through successive mitotic divisions in a greenhouse environment. Five of 19 plants regenerated from this transformation event showed the presence of a minichromosome(s). Four plants had a high incidence of nuclei with a single minichromosome plus the normal complement of 20 native chromosomes. The fifth plant had a majority of its nuclei with 1, 2, or 3 minichromosomes plus the normal complement of 20 native chromosomes. All the minichromosomes described above were comprised predominantly of centromeric repeats and Tn5-3 components.

Subsequently, retrofitted core set BAC pool 1 was further divided into four subpools having 5-6 of the retrofitted core set BAC clones (Table 10). FISH analyses demonstrated the presence of minichromosome(s) in embryonic callus generated by subpools 1.1 and 1.3. Two minichromosome events were produced from subpool 1.1: the first event had the normal complement of 20 chromosomes, plus 1 minichromosome that did not hybridize to PHP23715 marker or CentC at a detectable level; the second event showed 24-28 chromosomes, 3 copies of chromosome 6, and 1 minichromosome. Based on FISH observations, this minichromosome was positive for CentC, but was not consistently positive for the PHP23715 probe. This event may have been produced by integration and breakage of a native chromosome, and/or conditions produced by or resulting from aneuploidy. Subpool 1.3 produced 5 events. Three of the five events appeared to be de *novo* minichromosome formation and had the normal complement of 20 chromosomes plus 1 minichromosome, and the minichromosomes were positive for PHP23715 marker and CentC by FISH analysis of primary callus events at metaphase. One of these events, CMC3 subpool 1.3 event #27, was further analyzed by FISH for the presence of telomeres using the Telo-31 overgo probes (SEQ ID NOS: 192 & 193) using callus metaphase nuclei. This event has a very small minichromosome with two strong CentC foci and two telo-31 foci on each minichromosome. The two telo-31 foci observed may represent 4 separate foci which cannot be distinguished at this resolution. This event has a smaller minichromosome than observed in previous events. When measured at metaphase, the minichromosome is approximately 0.5 to 1 micron in length, which is about one-third to about one-half of the size of minichromosomes in other independent events. The FISH signal for telo-31 was generally weaker on the minichromosome than on the native chromosomes. Callus of CMC3 subpool 1.3 event #27 has been actively growing under selection for approximately 10 months, sampled at various timepoints, and analyzed by FISH to demonstrate stable maintenance of the minichromosome through many rounds of mitotic cell division. The two other subpool 1.3 events had 19 normal chromosomes plus one minichromosome, possibly as a result of integration and chromosome breakage. Using FISH analysis one of the two minichromosomes was positive for CentC only, and the second one was positive for both PHP23715 marker and CentC. Using FISH on metaphase nuclei of callus, all of the subpool events look essentially similar comparable samples from other minichromosome events generated, as shown in Figures 1, 2, 5, 6, and 9.

Another minichromosome event was observed (Figures 5-8) from a biolistic transformation event of a Hi-II immature embryo with linearized retrofitted core set BAC clones pool 3 (Table 8). The resultant embryogenic callus event was positive on Bialophos selection media and expressed the DsRed fluorescent marker protein. FISH analysis showed that this event was tetra-aneuploid, with only 39 chromosomes were observed because one chromosome 6 was absent. Each nucleus had 0, 1, or 2 minichromosomes in this event. As described with the first minichromosome event from pool 1, the minichromosomes in this event were comprised predominantly of centromeric repeats and Tn5-3 components. At anaphase, the sister chromatids of the minichromosome(s) were able to segregate (Figure 7) indicating the presence of functional centromeres. The above indicates that the minichromosomes are autonomously replicating and show stability through successive mitotic divisions.

Another minichromosome event was observed (Figures 9-10) from a biolistic transformation event of a Hi-II immature embryo with linearized, Tn5-3 retrofitted BAC clone bacm.pk128.j21. Again, the embryogenic callus of this third event was positive on Bialophos selection media and expressed the DsRed fluorescent protein. A plant was regenerated from this event and the root tips screened via FISH. Each nucleus had 0 or 1 minichromosome. In those nuclei with a minichromosome, only 19 of the 20 native chromosomes were observed. FISH analysis on metaphase spreads showed that the single minichromosome was composed primarily of centromeric repeats and Tn5-3 components.

Another minichromosome event, bCMC4 event #73, was observed from a biolistic transformation event of a Hi-II immature embryo with linearized, Tn5-3 retrofitted three B73 chromosome 4 centromere-specific BAC clones (Table 9). The resultant embryogenic callus event was positive on Bialophos selection media and expressed the DsRed fluorescent marker protein. FISH analysis showed that this event was aneuploid, with only 19 chromosomes and 1 or 2 minichromosomes. Similar to the minichromosomes described above, the minichromosomes in this event were comprised predominantly of centromeric repeats and Tn5-3 components.

Observations on all minichromosome events indicate that newly formed minichromosomes predominantly resulted from concatenation or/and amplification of the primary linear DNA constructs delivered to the plant cells to produce a de novo minichromosome.

Three of the minichromosome events were further analyzed utilizing immunofluorescence with a fluorescently labeled antibody raised against the centromere/kinetochore-specific protein, Centromeric Protein C (CENPC). Immunostaining of nuclear spreads revealed that CENPC binds specifically to the centromeric region of native chromosomes. In addition, the CENPC localized to distinct positions on all the minichromosomes in all three minichromosome events studied (Figures 3, 4, 8, and 10). Coupling FISH with immunolocalization showed that the CentC repeat and the DsRed2 marker probe localization overlapped with CENPC on minichromosomes. As seen for the native chromosomes, at metaphase the minichromosomes have two distinct foci of CENPC (Figures 3, 8, and 10), and at anaphase the sister chromatids of the minichromosomes separate and each sister chromatid has a single foci of CENPC (Figure 4). The above results indicate that the minichromosomes can recruit the necessary proteins, such as CENPC, for kinetochore formation, and therefore act autonomously of the native chromosomes during replication and segregation into daughter cells during mitosis and meiosis.

Several thousand bialophos-resistant, DsRed positive maize transgenic events have been generated and at least several hundred were cytologically characterized. The events show a high incidence of integration into the host chromosomes, with about 60% of events showing detectable integration by FISH. Both visual and selectable markers are present in almost 39% of the events, but not detectable by FISH analysis. To date most combinations of recombinant constructs produced minichromosomes containing both markers and CentC repeats detectable by FISH in only about 1% of the events (4 events, Figures 1-10). The exception is subpool 1.3 which generated minichromosomes containing both markers and CentC repeats in about 12% of the events analyzed (4 out of 34).

### D. Artificial Minichromosome Size Measurements

Three of the events with autonomous maize minichromosomes were further characterized by measuring the size of the assembled minichromosome and chromosome 6, which is easily identified by the 18-26S rDNA FISH probe. All measurements were taken on metaphase nuclei, which gave most consistent measurements. Other stages are less defined and highly variable in chromosome size, for example, preliminary measurements at prometaphase show the minichromosomes relatively unchanged in size relative to metaphase measurements, while the native chromosomes are about 4-5 times longer, therefore the minichrosomes measured at this stage are about 5% to about 15% of the length of an average native corn prometaphase chromosome. Therefore, minichromosomes measured at metaphase probably appear larger relative to native chromosomes than if measured at a different stage. Chromosomes were measured using a Leica DMRXA fluorescent microscope, images captured with a Photometrics CoolSnap CCD camera and mesurements taken with Metamorph^{®} image analysis software (Molecular Devices, Sunnyvale, CA, USA). All measurements are in microns.
Native Chromosome 6 (n=29):
   Mean = 4.62 (I) and 2.38 (w)
   Range = 3.16 - 5.78 (I) and 2.06 - 2.70 (w)
Minichromosome (n=337):
   Mean = 1.29 (I) and 1.67 (w)
   Range = 0.75 - 3.07 (I) and 1.12 - 3.17 (w)
The maize minichromosomes are on average about 28% of chromosome 6 in length, but can range from about 13-97% of the total length of chromosome 6 at metaphase.

The size of the maize minichromosomes observed can also estimated in Mb. For example, the corn genome comprises about 2500 Mb total DNA, with chromosomes ranging in size from about 150-350 Mb, chromosome 6 is approximately 200 Mb (Seneca 60).

### EXAMPLE 8: Methods

DNA isolation from immature ears or green leaves of maize plants was performed essentially as described in Ananiev et al. (1997) Proc Natl Acad Sci USA 94:3524-3529. BAC clone DNA was isolated using the Nucleobond plasmid kit (BD Biosciences Clontech, California) according to the manufacturer's recommendations. High molecular weight DNA preparation in agarose blocks was performed essentially as described in Liu & Whittier Nucleic Acids Res (1994) 22:2168-2169. DNA restriction digestions, gel electrophoresis, Southern blotting, and filter hybridization were carried out using standard techniques as described in Sambrook et al. (1989) Molecular Cloning: A Laboratory Manual, 2nd Ed., Cold Spring Harbor Laboratory Vols. 1-3.

### A. Overgo probe labeling for colony and Southern hybridization

Pooled overgos for each probe (5 pmol of each oligo) were combined with 2 µl of 10x Klenow buffer, 1 µl of Klenow enzyme (5 U/µl), 1 µl of 1 mM dGTP, 1 µl of 1mM dTTP, [α-³²P]dCTP and [α-³²P]dATP - 5 µl each, and sterile water to a final volume of 20 µl. The reaction mixture was incubated at 14°C for 2 hours. Incorporation percentage was calculated and was considered acceptable at 50% or greater.

### B. Membrane preparation and hybridization

Membranes were prepared using 432 384-well plates evenly distributed between the Mo17 *Ec*oRI and *Hind*III BAC libraries. A 4 x 4 gridding pattern that allowed 96 plates with 384 wells to be spotted onto a single Millipore Imobilon N+ nylon membrane (Bedford, MA) was used. The 96 plates gridded comprised 90 BAC clone plates and 6 plasmid clone plates used as gridding markers. After gridding, membranes were carefully placed bacteria side up on Luria-Bertani agar plates with 17 µg/mL chloramphenicol, the plates were covered, inverted, and grown at 37°C overnight. After colony growth the membranes were removed from the plates and denatured in 1.5 M NaCl and 0.5 M NaOH for 5 min each, followed by neutralization in 1.5 M NaCl, 1 M Tris-HCl two times for 5 min each. Membranes were dried and treated with Proteinase K (100 mls at 1 mg/mL; Sigma, St. Louis, MO) for 50 min at 37°C.

Each membrane was soaked in 6x SSC, 0.5% SDS solution in plastic boxes. Filters were prehybridized at 56°C in 6x SSC, 0.5% SDS with constant agitation for at least 20 minutes. Pooled overgo probes were denatured at 100°C for 5 min and added to the hybridization solution which had been used for prehybridization. Hybridization was for 12-16 h at 56°C. Membranes were washed progressively for 1 h each at 56°C in 2x SSC and 0.1% SDS (wash 1), 1.5x SSC and 0.1% SDS (wash 2), and 0.1x SSC and 0.1% SDS (wash 3). Membranes were sealed in plastic wrapped and exposed to X-ray film for 3 h to overnight. Following hybridization, the filters were stripped in 100 ml of 0.1 x SSC and 0.1 % SDS at 90°C for 10 min and stored at -20°C. Membranes were used multiple times.

### C. Cytological Methods

Any suitable cytological methods, and compositions, including many standard cytological methods, preparations, and like are known in the art and can be used to examine plant tissues.

### i. Preparation of nuclei from maize callus tissue

1. Calli used for making nuclear preparations were first gassed with nitrous oxide at 150psi for 3 hours then immediately fixed. Nitrous oxide arrests nuclei at metaphase which allows for improved chromosomal spreads for FISH analysis.
2. Fix the callus tissue sample in 50% acetic acid for at least 1 hour. Tissue can be stored indefinitely in 50% acetic acid at -20°C.
3. Separate somatic embryos from callus and place in 50 µl drop of PIM buffer (50mM CaCl₂, 10mM sodium acetate, pH 5.8) in a small petri plate.
4. Dissect somatic embryos into smaller pieces of 0.5 mm.
5. Wash tissue in PIM buffer 3-5 times over 1 hour to remove fixative. Slowly pipette several times to wash and replace with fresh PIM buffer.
6. Carefully remove PIM buffer. Add 50 µl enzyme digest solution (2% w/v cellulase (Cat# CEL, Worthington Biochemical Corp. (Lakewood, NJ, USA)), 0.2% w/v pectinase (Cat# PASE, Worthington Biochemical Corp. (Lakewood, NJ, USA)); 0.5% w/v bovine serum albumin)
7. Digest tissue at room temperature, in the dark, in a moist chamber for 1-2 hours. As the tissue begins to soften, very gently pipette and/or squash with probe to break up larger pieces and release cells.
8. Carefully remove enzyme digest solution and replace with about 50 µl PIM buffer.
9. Transfer free cells/nuclei to a microfuge tube. Add more PIM buffer to remaining digested tissue and gently pipette to release cells, transfer these cells to the microfuge tube, repeat as needed.
10. Pellet cells in microcentrifuge at 500 rpm for 3 minutes, remove supernatant. Add fresh PIM buffer and gently resuspend cells. Repeat this wash step 3 more times.
11. Remove PIM buffer and replace with 50% acetic acid. Gently resuspend cells, pellet at 500 rpm for 10 min., remove supernatant and add 50% acetic acid. Repeat.
12. Store isolated nuclei in 50% acetic acid at -20°C. The final volume of 50% acetic acid should be 2X the volume of the nuclear pellet.
13. Transfer 5 µl of resuspended nuclei to a glass slide, add an 18 mm² coverslip.
14. Heat slide on a hot plate at 70°C for 15 seconds.
15. Remove slide from heat and gently press down on coverslip to squash the nuclei.
16.Allow the slide to cool briefly, then dip slide in liquid nitrogen for 10-15 seconds.
17. Remove slide from liquid nitrogen and warm coverslip with your breath.
18. Quickly remove coverslip with the edge of a razor blade.
19. Place slide in 2 changes of 100% EtOH for 2 minutes each.
20.Allow slides to air dry. Store slides at -20°C until needed.

### ii. FISH followed by direct immunolocalization of nuclei

### a. Overgo probe preparation for FISH

Overgo probes are described in Table 1.
1. Add 10 µl of 100 µM overgo mix, comprising equal concentrations of each overgo, to 5 µl of deionized water.
2. Heat at 95°C for 1 min, then transfer to ice.
3. Add to the above mixture:
   - 2 µl 10X DNA polymerase buffer (100mM Tris-HCl, pH 7.5, 100 mM MgCl₂, 7.5 mM DTT)
   - 0.5 µl dUTP fluorophore
      a) dUTP-Cy3 (Amersham)
      b) dUTP-FITC (Roche)
      c) dUTP-Texas Red (Molecular Probes)
   - 2 µl dNTPs (200 µM A-, G-, CTP; 40 µM TTP)
   - 0.5 µl Klenow
4. Incubate at 37°C for 20 min.
5. Clean probe using Quigen Nucleotide Extraction kit. Elute in 50 ml of 50% formamide in kit elution buffer.

### b. Fluorescent in situ hybridization (FISH)

FISH of maize nuclei on slides was done essentially as follows:
1. Fix slide 10 min. in 1% v/v paraformaldehyde in phosphate-buffered saline (PBS) pH 7.2
2. Wash 2X 5 min. in PBS
4. Wash 2 min. in distilled/deionized water
5. Air dry slide
6. Hybridize 2 min at 80°C in titrated fluorescent probe in 50% formamide in a final concentration of 50 mM MgCl₂
7. Hybridize 30 min - overnight in moist chamber at 37°C
8. Wash 5 min. in 2X SSC
9. Wash 5 min. in 0.2X SSC
10. Air dry slide
11. Add Vectashield^{®} with DAPI (Cat# H-1200, Vector Laboratories, Burlingame, CA, USA) and coverslip (5 ml mounting media/22mm coverslip)
12. Examine under microscope using appropriate filter sets and/or immersion oil as needed.

### c. Immunolocalization

After examination and characterization of FISH probe localization, these same samples can be processed and used for immunolocalization using an direct-tagged antibody probe. Immunolocalization of fluorescent-tagged polyclonal rabbit anti-CENPC antibody was done essentially as follows:
1. Remove coverslip
2. Wash 5 min. in 70% v/v EtOH to remove mounting medium and immersion oil
3. Wash 3X 5 min. in PBS
4. Block 1 hour at 37°C in a moist chamber in 5% v/v normal rabbit serum (Jackson Immunoresearch, West Grove, PA, USA) in PBS-BT (PBS with 3% w/v BSA, 0.02% w/v Na azide, 0.5% v/v Triton X-100)
5. Rinse in PBS
6. Incubate overnight at 37°C in a moist chamber with 1 ° antibody in 5% v/v normal rabbit serum in PBS-BT. Rabbit anti-CENPC-Cy3 (or -FITC) was used at a 1:200 dilution, final concentration 2.5 µg/mL of labeled antibody.
7. Wash 3X in PBS over 1 hour period
8. Air dry slide
9. Add Vectashield^{®} with DAPI (Cat# H-1200, Vector Laboratories, Burlingame, CA, USA) and coverslip (5 ml mounting media/22mm coverslip)
10. Seal coverslip with nail polish
11. Examine under microscope using appropriate filters and/or immersion oil as needed.

### d. CENPC Antibody production and labeling

A maize homologue of mammalian CENPC was isolated by Dawe et al. (1999 Plant Cell 11:1227-1238) and shown to be a component of the kinetochore in maize. A 20 amino acid conserved peptide from the amino terminal domain was synthesized and used for polyclonal antibody production in rabbits (Openbiosystems, Huntsville, AL, USA). The resulting antibodies were directly labeled with flurophores suing the Fluorolink-AbCy3 labelling kit (GE Healthcare, UK) or Fluorescein Protein labelling kit (Roche Diagnostics Corp., Indianapolis, IN, USA).

### iii. Fiber-FISH

Extended DNA fibers on cytological slides were prepared as described in Jackson et al. (1998) Genome 41:566-572. Probes for fiber-FISH were labeled with biotin-11-dUTP (Roche, Germany) or DIG-dUTP (Roche, Germany) using Nik Translation Labeling Kit (Roche, Germany) according to manufactures recommendations. After precipitation, the probes were re-dissolved in TE buffer and stored at -20°C. For fiber-FISH, the probes were hybridized to DNA fibers in a mixture of 50% (v/v) formamide, 10% (v/v) SDS, and 2x SSC in a final volume of 10 µL. The slides were covered with cover slips, sealed with rubber cement and incubated at 80°C for 2 min to denature both the probes and the target DNA, followed by incubation at 37°C. The post-hybridization washes and signal detection were performed as described by Zhong et al. (1996) Plant Mol Biol Rep 14: 232-242. The biotin-labeled probes were detected with fluorescein-avidin DN (Vector Laboratories, Burlingame, CA, USA), biotinylated anti-avidin D (Vector Laboratories, Burlingame, CA, USA) and again with fluorescein-avidin DN (Vector Laboratories, Burlingame, CA, USA). The DIG-labeled probes were detected by mouse anti-DIG monoclonal antibodies (Jackson Jackson ImmunoResearch, West Grove, PA, USA) and Cy3-conjugated anti-mouse antibodies in sheep (Jackson ImmunoResearch, West Grove, PA, USA). The slides were then mounted in Vectashield mounting medium (Vector Laboratories, Burlingame, CA, USA). Preparations were examined using a Leica DMRXA fluorescent microscope, images captured with a Photometrics CoolSnap CCD camera. Images were captured using Metamorph^{®} image analysis software (Molecular Devices, Sunnyvale, CA, USA). Fiber-FISH was performed on 3 to 5 preparations from each line.

## Claims

1. An artificial plant minichromosome comprising a functional centromere containing: (a) at least two arrays of tandem repeats of CentC in an inverted orientation wherein the first array comprises at least fifty copies of CentC and the second array comprises at least fifty copies of CentC; and, (b) at least one copy of a retrotransposable element, wherein the retrotransposable element is situated between the first and the second array.

2. The artificial plant minichromosomes of claim 1, wherein the retrotransposable element is selected from CentA, CRM1, and CRM2.

3. The artificial plant minichromosome of claim 1 wherein said minichromosome further comprises at least one functional telomere.

4. The artificial plant minichromosome of any one of claims 1-3, wherein the functional centromere specifically binds centromeric protein C (CENPC).

5. A corn plant comprising the artificial minichromosome of any one of claims 1 to 4.

6. An isolated polynucleotide comprising:
(a) At least two arrays of tandem repeats of CentC in an inverted orientation wherein the first array comprises at least ten copies of CentC and the second array comprises at least ten copies of CentC; and,
(b) at least one copy of a retrotransposable element, wherein the retrotransposable element is situated between the first and the second array.

7. The isolated polynucleotide of claim 6, wherein the retrotransposable element is selected from CentA, CRM1, and CRM2.

8. A recombinant construct comprising the isolated polynucleotide of claim 6 or 7.

9. The recombinant construct of claim 8, further comprising a DNA fragment comprising an array of at least 30 copies of telomeric repeats.

10. A transgenic corn plant comprising the recombinant construct of claim 8 or 9.

## Patentansprüche

1. Künstliches Pflanzenminichromosom umfassend ein funktionelles Centromer enthaltend: (a) mindestens zwei Anordnungen von Tandemwiederholungen von CentC in umgekehrter Orientierung, wobei die erste Anordnung mindestens fünfzig Kopien von CentC umfasst und die zweite Anordnung mindestens fünfzig Kopien von CentC umfasst; und (b) mindestens eine Kopie eines zur Retrotransposition fähigen Elements, wobei das zur Retrotransposition fähige Element sich zwischen der ersten und der zweiten Anordnung befindet.

2. Künstliches Pflanzenminichromosom nach Anspruch 1, wobei das zur Retrotransposition fähige Element unter CentA, CM und CRM2 ausgewählt ist.

3. Künstliches Pflanzenminichromosom nach Anspruch 1, wobei das Minichromosom des Weiteren mindestens ein funktionelles Telomer umfasst.

4. Künstliches Pflanzenminichromosom nach einem der Ansprüche 1 - 3, wobei das funktionelle Centromer centromeres Protein C (CENPC) spezifisch bindet.

5. Maispflanze umfassend das künstliche Minichromosom nach einem der Ansprüche 1 bis 4.

6. Isoliertes Polynucleotid, umfassend:
(a) mindestens zwei Anordnungen von Tandemwiederholungen von CentC in umgekehrter Orientierung, wobei die erste Anordnung mindestens zehn Kopien von CentC umfasst und die zweite Anordnung mindestens zehn Kopien von CentC umfasst; und
(b) mindestens eine Kopie eines zur Retrotransposition fähigen Elements, wobei das zur Retrotransposition fähige Element sich zwischen der ersten und der zweiten Anordnung befindet.

7. Isoliertes Polynucleotid nach Anspruch 6, wobei das zur Retrotransposition fähige Element unter CentA, CRM1 und CRM2 ausgewählt ist.

8. Rekombinantes Konstrukt umfassend das isolierte Polynucleotid nach Anspruch 6 oder 7.

9. Rekombinantes Konstrukt nach Anspruch 8, des Weiteren ein DNA-Fragment umfassend, das eine Anordnung von mindestens 30 Kopien telomerer Wiederholungen umfasst.

10. Transgene Maispflanze umfassend das rekombinante Konstrukt nach Anspruch 8 oder 9.

## Revendications

1. Minichromosome de plante artificielle comprenant un centromère fonctionnel contenant: (a) au moins deux rangées de répétitions en tandem de CentC dans une orientation inversée où la première rangée comprend au moins cinquante copies de CentC et la deuxième rangée comprend au moins cinquante copies de CentC; et (b) au moins une copie d'un élément rétrotransposable, où l'élément rétrotransposable est situé entre la première et la deuxième rangées.

2. Minichromosome de plante artificielle selon la revendication 1, dans lequel l'élément rétrotransposable est choisi parmi CentA, CRM1 et CRM2.

3. Minichromosome de plante artificielle selon la revendication 1, où ledit minichromosome comprend en outre au moins un télomère fonctionnel.

4. Minichromosome de plante artificielle selon l'une quelconque des revendications 1-3, dans lequel le centromère fonctionnel se lie spécifiquement à une protéine C centromère (CENPC).

5. Plante de maïs comprenant le minichromosome artificiel selon l'une quelconque des revendications 1 à 4.

6. Polynucléotide isolé comprenant:
(a) au moins deux rangées de répétitions en tandem de CentC dans une orientation inversée où la première rangée comprend au moins dix copies de CentC et la deuxième rangée comprend au moins dix copies de CentC; et
(b) au moins une copie d'un élément rétrotransposable, où l'élément rétrotransposable est situé entre la première et la deuxième rangées.

7. Polynucléotide isolé selon la revendication 6, dans lequel l'élément rétrotransposable est choisi parmi CentA, CRM1 et CRM2.

8. Construction recombinante comprenant le polynucléotide isolé selon la revendication 6 ou 7.

9. Construction recombinante selon la revendication 8, comprenant en outre un fragment d'ADN comprenant une rangée d'au moins 30 copies de répétitions télomères.

10. Plante de maïs transgénique comprenant la construction recombinante selon la revendication 8 ou 9.
